# EUROPEAN PATENT APPLICATION

(11) **EP 3 901 175 A1**
(43) Date of publication of application: **27.10.2021**
(21) Application number: 20738682.2
(22) Date of filing: 09.01.2020
(51) Int. Cl.: C07K 16/28, C12N 15/13, A61K 39/395, A61P 35/00

(54) **ANTI-CD73 MONOCLONAL ANTIBODY AND APPLICATION THEREOF**

(30) Priority: 11.01.2019 CN 201910025923
(71) Applicant: Chengdu Conmed Biosciences Co., Ltd, Chengdu, Sichuan 610219 (CN)
(72) Inventor: XU, Gang, Chengdu, Sichuan 610219 (CN); CHEN, Bo, Chengdu, Sichuan 610219 (CN); LIU, Chengju, Chengdu, Sichuan 610219 (CN)
(74) Representative: Elzaburu S.L.P.
(86) International application number: PCT/CN2020/071179
(87) International publication number: WO 2020/143710

(57) **Abstract**

Provided in the present invention are an anti-CD73 monoclonal antibody and a preparation method and application thereof.

## Description

CD73 is a glycoprotein with 5'nucleotidase activity, also known as extracellular 5'nucleotidase (NT5E), anchored to the cell membrane surface by Glycosylphosphatidylinositol (GPI). As the GPI anchor is cleaved by enzymes, CD73 on the cell membrane falls off the cell surface, and was released into the blood to form soluble CD73 (sCD73). Both CD73 and sCD73 on cell membranes have hydrolase activity, and can catalyze Adenosine monophosphate (AMP) in extracellular matrix, to dephosphorylate to generate adenosine (ADO). The generated ADO can induce a series of biological effects through the adenosine receptors (AIR, A2AR, A2BR and A3R) on the cell membrane, such as expanding blood vessels, improving endothelial barrier function, stimulating angiogenesis, inhibiting platelet aggregation, reducing free radical production, etc.

CD73 is widely expressed in a variety of human tissues, including intestines, kidneys, brain, liver, heart, lungs, respiratory epithelium, spleen, lymph nodes and bone marrow, etc. In the immune system, CD73 is expressed in lymphocytes, regulatory T cells (Tregs), neutrophils, myeloid-derived suppressor cells (MDSC), dendritic cells, natural killer cells, and macrophages. As a rate-limiting enzyme in the production of ADO, CD73 is one of the determinants of ADO halo surrounding immune cells, and ADO halo locally forms an immunosuppressive environment.

The sCD73 concentration was significantly higher in blood of tumor patients than in normal humans (Huang Q et al, 2017; Hay C et al 2015), CD73 was one of the key enzymes determining the level of ADO in the tumor microenvironment (Augusto et al, 2013), and the overexpression of CD73 in tumor tissues was often associated with poor overall survival or tumor progression (Allard D et al, 2016; Wang R et al, 2017). The ADO pathway is considered to be one of the major inhibitory pathways in the tumor microenvironment. As the content of ADO in the tumor microenvironment increases, ADO as an anti-inflammatory mediator leads to a decrease in infiltration of immune cells, resulting in chronic suppression of the immune response against the tumor. The change of tumor microenvironment generated by the tumor cells, further promotes the growth of the tumor cells through cytokines secreted by inflammatory cells, and inhibits the anti-tumor activity of immune cells, promotes the occurrence and progression of cancers. Repressing the nucleolytic enzymes of cell surface CD73 and sCD73 released into the blood, helps to eliminate the inhibitory effect of high concentrations of adenosine on immune killing in the tumor microenvironment. Studies have shown that CD73 knockout mice significantly increased the immunity that against tumors compared to normal mice (Stagg et al, 2011, 2012).

Blocking the biological activity of CD73 by monoclonal antibodies or small molecules has been shown to slow down the growth and proliferation of tumor cells (Stagg J et al 2010, Zhi X et al 2010, Terp MG et al 2013), reduce neovascularization (koszaka P et al 2014, Burghoff S et al 2014), indicate that CD73 has value as a potential therapeutic target for cancer treatment (Zhang b.2010, Beavis, p.a. et al 2012, Allard B et al 2014, Hay CM et al 2016). The applicant develops a novel monoclonal antibody targeting human CD73, which not only can inhibit the hydrolase activity of membrane-bound CD73 and sCD73, but also can effectively reduce the abundance of CD73 on the cell membrane surface through receptor-mediated endocytosis after CD73 binding, thereby inhibiting the generation of ADO. After the CD73 blocking treatment is combined with other immune molecule regulators (such as anti-PD-1 antibodies), the curative effect is remarkably superior to that of a single treatment, it shows that the combination therapy of CD73 enzyme activity blocking antibody and other immune molecular modulators is an extremely attractive option and is expected to become a novel biological therapy for anti-cancer.

### Summary of the Invention

In one aspect, the present disclosure relates to an antibody or antigen-binding portion thereof that specifically binds to CD73.

In one aspect, the present disclosure provides an antibody or antigen-binding portion thereof that binds to human CD73, which binds to human differentiation determinant (CD73), inhibits hydrolase activity of CD73 on the cell membrane, simultaneously inhibits hydrolase activity of soluble CD73(sCD73) in serum and body fluids, and mediates endocytosis of CD73 on cell surface, effectively reducing CD73 abundance of cell membrane surface.

The antibody or antigen-binding portion thereof according to any one of the preceding aspects, wherein the antibody is an antibody fragment.

The antibody or antigen-binding portion thereof according to any one of the preceding aspects, wherein the antibody or antigen-binding portion thereof can block the hydrolase activity of CD73.

The antibody or antigen-binding portion thereof according to any one of the preceding aspects, wherein the antibody or antigen-binding portion thereof is humanized.

A vector comprising the nucleic acid of any of the preceding aspects.

A cell comprising the vector of any of the preceding aspects.

A pharmaceutical composition or kit comprising the antibody or antigen-binding portion thereof, or the nucleic acid encoding same of any of the preceding aspects, and a pharmaceutically acceptable carrier.

A method for treating cancer, comprising the steps of administering to the mammal a therapeutically effective amount of the antibody or antigen-binding portion thereof, or the nucleic acid molecule, or the vector, or the cell or the pharmaceutical composition of any of the preceding aspects, optionally, the method further comprising administering to the mammal a therapeutically effective amount of anti-PD-1 antibodies.

A method for treating diseases associated with abnormal production of CD73 in a mammal, comprising the steps of administering to the mammal a therapeutically effective amount of the antibody or antigen-binding portion thereof, or the nucleic acid molecule, or the vector, or the cell or the pharmaceutical composition of any of the preceding aspects, optionally, the method further comprising administering to the mammal a therapeutically effective amount of anti-PD-1 antibodies.

Use of the antibody or antigen-binding portion thereof, or the nucleic acid molecule, or the vector, or the cell or the pharmaceutical composition of any of the preceding aspects, in the preparation of a medicament for the treatment of diseases associated with abnormal production of CD73 in a mammal.

Use of the antibody or antigen-binding portion thereof, or the nucleic acid molecule, or the vector, or the cell or the pharmaceutical composition of any of the preceding aspects, in the preparation of a medicament for the treatment of cancer in a mammal.

Use of the antibody or functional fragment thereof, or nucleic acid molecule, or vector, or cell, or pharmaceutical composition or kit of any of the preceding aspects in the preparation of a medicament for inhibiting hydrolase activity of CD73.

Use of the antibody or functional fragment thereof, or nucleic acid molecule, or vector, or cell or pharmaceutical composition or kit of any of the preceding aspects in the preparation of a reagent for mediating endocytosis of CD73 on cell surface.

### Brief Description of the Drawings

Figure 1: The expression of extracellular domain proteins of recombinant human, cynomolgus monkey and mouse CD73 molecules in HEK293 cells.
Figure 2: Flow cytometry test results of the human CD73 stable-transformed CHO cells.
Figure 3: Figure 3A. Binding of CD73 positive clones to Calu6 cells; Figure 3B. Binding of CD73 positive clones to hCD73-CHO-1C11 cells.
Figure 4: Figure 4A. The inhibition of CD73 enzymatic activity on Calu6 cell surface by CD73 antibody; Figure 4B. The inhibition of CD73 enzymatic activity in the solution by CD73 antibody.
Figure 5: Figure 5A. Binding of CD73 chimeric antibody to recombinant human CD73 protein; Figure 5B. Binding of CD73 chimeric antibody to recombinant cynomolgus monkey CD73 protein.
Figure 6: Figure 6A. The inhibition of CD73 enzymatic activity on Calu6 cell surface by anti-CD73 antibody after humanization; Figure 6B. The inhibition of soluble CD73 enzymatic activity by anti-CD73 antibody after humanization.
Figure 7: Humanized anti-CD 73 antibody mediates the endocytosis of CD73 on the cell surface.
Figure 8: The effect of humanized anti-CD73 antibody and PD-1 antibody single or in combination on T lymphocyte activation in the peripheral blood PBMC mixed lymphocyte reaction; Figure 8A. The release of INF-γ after T lymphocyte activation; Figure 8B. The release of INF-α after T lymphocyte activation.

### Detailed Description of Embodiments

### I. Definitions

In the present invention, all scientific and technical terms used herein have the meanings commonly understood by a person skilled in the art unless specified otherwise. In addition, terms and laboratory operation steps related to the protein and nucleic acid chemistry, molecular biology, cell and tissue culture, microbiology and immunology as used herein are terms and conventional steps that are widely used in the corresponding art. To better understand the present invention, definitions and explanations of related terms are provided below.

In one aspect, provided herein are antibodies (e.g., monoclonal antibodies) and antigen-binding fragments thereof that specifically bind to CD73 (e.g., human CD73). In particular aspects, provided herein are monoclonal anti-CD73 antibodies that specifically bind to human CD73, wherein the anti-CD73 antibodies include variants of the parent antibody. In particular aspects, provided herein are antibodies that specifically bind to CD73 (e.g., human CD73). In a specific aspect, provided herein is anti-CD73 antibody comprising one or more modifications in amino acid residues (for example, 5-13 amino acid substitutions in the framework region of the heavy chain variable region), and compared with the parent antibody without such modifications, it maintains the affinity to the antigen. In certain aspects, in vivo or in vitro, or both in vivo and in vitro, such anti-CD73 antibodies have hydrolase activity of CD73.

In certain embodiments, the antibody or antigen-binding fragment described herein may comprise sequences that do not naturally exist within the antibody germline repertoire in animals or mammals (e.g., humans).

As used herein and unless otherwise specified, the term "about" or "approximately" means within plus or minus 10% of a given value or range. Where integers are required, the term means within plus or minus 10% of a given value or range, rounded up or down to the nearest integer.

Human CD73, also known as "cluster of differentiation 73" or "CD73" or extracellular-5'-nucleotidase or 5-pro-ribonucleotide phosphohydrolase, EC3.1.3.5, it is encoded by the NT5E gene and exhibits 5' -nucleotidase (especially AMP-, NAD-, and NMN-nucleotidase) activity. CD73 catalyzes the conversion of purine 5-protomononucleotide to nucleotide at neutral pH, and the preferred substrate is AMP. The enzyme consists of a dimer of 2 identical 70-kD subunits linked to the outer surface of the plasma membrane by glycosylphosphatidylinositol linkages. The amino acid sequence of the human CD73 preproprotein (monomer), including a signal sequence of 1-26 amino acids, which is shown in the gene bank (Genbank) under accession number NP _00251, the entire disclosure of which is incorporated herein by reference.

In the present context, "neutralizing the enzymatic activity of CD73" refers to the process of inhibiting the 5'-nucleotidase (5' -extracellular nucleotidase) activity of CD73, including both CD73 on the cell surface and CD73 in the free state. This especially includes inhibiting CD73-mediated adenosine production, that is, inhibiting CD73-mediated catabolism of AMP into adenosine. This can be measured, for example, in a cell-free assay, which measures the ability of the test compound to inhibit the direct or indirect conversion of AMP to adenosine. In one embodiment, the antibody preparation causes a reduction in the conversion of AMP to adenosine by at least 50%, a reduction in the conversion of AMP to adenosine by at least 70%, or a reduction in the conversion of AMP to adenosine by at least 80%, refer to, for example, the assays described herein.

The phrase "substantially identical" with respect to an antibody chain polypeptide sequence may be construed as an antibody chain exhibiting at least 60%, 65%, 70%, 75%, 80%, 85%, 90%, 95%, 96%, 97%, 98%, 99% or more sequence identity to the reference polypeptide sequence. The term with respect to a nucleic acid sequence may be construed as a sequence of nucleotides exhibiting at least about 60%, 65%, 70%, 75%, 80%, 85%, 90%, 95%, 96%, 97%, 98%, 99% or more sequence identity to the reference nucleic acid sequence.

"identical" or "identity" with respect to a sequence has well-recognized meaning in the art, and the percentage of sequence identity between two nucleic acid or polypeptide molecules or regions can be calculated using the disclosed techniques. Sequence identity can be measured along the entire length of a polynucleotide or polypeptide or along a region of the molecule (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "identity" is well known to a person skilled (Carrillo, H. & Lipman, D., SIAM J Applied Math 48:1073(1988)).

The phrases and terms "functional fragment, variant, derivative or analog" and the like, as well as various forms thereof, of an antibody or antigen is a compound or molecule having qualitative biological activity in common with a full-length antibody or antigen of interest. For example, a functional fragment or analog of an anti-CD73 antibody is one which can bind to an CD73 molecule or one which can prevent or substantially reduce CD73 activity.

"Substitutional" variants are those that have at least one amino acid residue in a native sequence removed and replaced with a different amino acid inserted in its place at the same position. The substitutions may be single, where only one amino acid in the molecule is substituted, or may be multiple, where two or more amino acids are substituted in the same molecule. The multiple substitutions may be at consecutive sites. Also, one amino acid can be substituted with multiple residues, in which case such a variant comprises both a substitution and an insertion. "Insertional" variants are those with one or more amino acids inserted immediately adjacent to an amino acid at a particular position in a native sequence. Immediately adjacent to an amino acid means linked to either the α-carboxyl or α-amino functional group of the amino acid. "Deletional" variants are those with one or more amino acids in the native amino acid sequence removed. Ordinarily, deletional variants will have one or two amino acids deleted in a particular region of the molecule.

As used herein, the term "antibodies" is used in the broadest sense, and specifically covers monoclonal antibodies (including full length monoclonal antibodies), polyclonal antibodies, multispecific antibodies (e.g., bispecific antibodies), antibody fragments or synthetic polypeptides carrying one or more CDR or CDR-derived sequences so long as the polypeptides exhibit the desired biological activity. Antibodies (Abs) and immunoglobulins (Igs) are glycoproteins having the same structural characteristics. "Antibodies" can also refer to immunoglobulins and immunoglobulin fragments, whether produced naturally or partially or fully synthetically (e.g., recombinantly), including any fragment thereof which comprises at least a partial variable region of an immunoglobulin molecule and retains the binding specificity of the full length of the immunoglobulin molecule. Thus, antibodies include any protein having a binding domain which is homologous or substantially homologous to an immunoglobulin antigen binding domain (an antibody binding site). Antibodies include antibody fragments, such as anti-tumor stem cell antibody fragments. As used herein, the term antibody thus includes synthetic antibodies, recombinantly-produced antibodies, multispecific antibodies (e.g., bispecific antibodies), human antibodies, non-human antibodies, humanized antibodies, chimeric antibodies, intrabodies, and antibody fragments, for example without limitation, Fab fragments, Fab' fragments, F(ab')2 fragments, Fv fragments, disulfide-linked Fv (dsFv), Fd fragments, Fd' fragments, single-chain Fv (scFv), single-chain Fab (scFab), diabodies, anti-idiotypic (anti-Id) antibodies, or antigen-binding fragments of any of the above-mentioned antibodies. The antibodies provided herein include any immunoglobulin class (e.g., IgG, IgM, IgD, IgE, IgA, and IgY), and members of any type (e.g., IgG1, IgG2, IgG3, IgG4, IgA1, and IgA2) or subtype (e.g., IgG2a and IgG2b) ("type" and "class", as well as "subtype" and "subclass" are used interchangeably herein). Native or wildtype (that is, obtained from a non-artificially manipulated member of a population) antibodies and immunoglobulins are usually heterotetrameric glycoproteins of about 150,000 daltons, which is composed of two identical light (L) chains and two identical heavy (H) chains. Each heavy chain has at one end a variable domain (V_{H}) followed by a number of constant domains. Each light chain has a variable domain at one end (V_{L}) and a constant domain at the other end. By "non-artificially manipulated" is meant not treated to contain or express a foreign antigen binding molecule. Wild type can refer to the most prevalent allele or species found in a population or to the antibody obtained from a non-manipulated animal, as compared to an allele or polymorphism, or a variant or derivative obtained by a form of manipulation, such as mutagenesis, use of recombinant methods and so on to change an amino acid of the antigen-binding molecule.

As used herein, "anti-CD73 antibody" means an antibody or polypeptide derived therefrom (a derivative) which binds specifically to CD73 as defined herein, including, but not limited to, molecules which inhibit or substantially reduce the binding of CD73 to its ligands or inhibit CD73 activity.

The term "variable" in the context of a variable domain of antibodies, refers to certain portions of the pertinent molecules which differ extensively in sequence between and among antibodies and are used in the specific recognition and binding of a particular antibody for its particular target. However, the variability is not evenly distributed through the variable domains of antibodies. The variability is concentrated in three segments called complementarity determining regions (CDRs; i.e., CDR1, CDR2, and CDR3) or hypervariable regions, both in the light chain and the heavy chain variable domains. The more highly conserved portions of variable domains are called the framework (FR) regions or sequences. The variable domains of native heavy and light chains each comprise four FR regions, largely adopting a β-sheet configuration, linked by three CDRs, wherein the CDRs form loops connecting, and in some cases form part of, the β-sheet structure. The CDRs in each chain are held together often in proximity by the FR regions and, with the CDRs from the other chain, contribute to the formation of the target (epitope or determinant) binding site of antibodies (see Kabat et al. Sequences of Proteins of Immunological Interest, National Institute of Health, Bethesda, MD (1987)). As used herein, numbering of immunoglobulin amino acid residues is done using the immunoglobulin amino acid residue numbering system of Kabat et al., unless otherwise indicated. One CDR can carry the ability to bind specifically to the cognate epitope.

The term "hinge" or "hinge region" as used herein refers to a flexible polypeptide comprising amino acids between the first and second constant domains of an antibody.

As used herein, "antibody fragments" or "antigen-binding fragments" of antibodies refer to any portion of a full length antibody that is less than the full length, but comprises at least a portion of the variable region of the antibody that binds to the antigen (e.g., one or more CDRs and/or one or more antibody binding sites), and thus retain binding specificity as well as at least partial specific binding ability of the full length antibody. Thus, antigen-binding fragments refer to antibody fragments comprising an antigen-binding portion that binds to the same antigen as the antibody from which the antibody fragments are derived. Antibody fragments include antibody derivatives produced by enzymatic treatment of full length antibodies, and derivatives produced by the synthesis, such as recombinantly-produced derivatives. Antibodies include antibody fragments. Examples of antibody fragments include, but not limited to, Fab, Fab', F(ab')2, single chain Fv (scFv), Fv, dsFv, diabodies, Fd and Fd' fragments, and other fragments, including modified fragments (see, for example, Methods in Molecular Biology, Vol 207: Recombinant Antibodies for Cancer Therapy Methods and Protocols (2003); Chapter 1; p 3-25, Kipriyanov). The fragments may comprise a plurality of chains joined together, for example via a disulfide bond and/or via a peptide linker. Antibody fragments usually comprise at least or about 50 amino acids, and typically at least or about 200 amino acids. Antigen-binding fragments include any antibody fragments, upon the antibody fragments are inserted into an antibody framework (e.g., by substitution of a corresponding region), an antibody that immunospecifically binds (i.e., exhibiting at least or at least about 10⁷-10⁸ M⁻¹ of Ka) antigens is obtained. "Functional fragments" or "analogs of anti-CD73 antibodies" are fragments or analogs which prevents or substantially reduces the ability of the receptors to bind to ligands or initiate signal transduction. As used herein, functional fragments generally have the same meaning as "antibody fragments" and, in the case of antibodies, may refer to fragments which prevent or substantially reduce the ability of the receptors to bind to ligands or initiate signal transduction, such as Fᵥ, F_{ab} and F_{(ab')2} et al. "Fᵥ" fragments consist of dimers (V_{H}-V_{L} dimers) formed by a variable domain of a heavy chain and a variable domain of a light chain by non-covalent binding. It is in this configuration that the three CDRs of each variable domain interact to define a target binding site on the surface of the V_{H}-V_{L} dimer, as is the case with an intact antibody. Collectively, the six CDRs confer antigen-binding specificity to the intact antibody. However, even a single variable domain (or half of an Fᵥ comprising only 3 CDRs specific for a target) has the ability to recognize and bind targets.

"Single-chain Fv", "sFᵥ" or "scab" antibody fragments comprise V_{H} and V_{L} domains of antibodies, wherein these domains are present in a single polypeptide chain. Generally, the Fᵥ polypeptide further comprises a polypeptide linker between the V_{H} and V_{L}, which is typically a flexible molecule that enables the sFv to form the desired structure for target binding.

The term "diabody" refers to antibody fragments having two antigen-binding sites, the antibody fragments can comprise a heavy chain variable domain (V_{H}) connected to a light chain variable domain (V_{L}) in the same polypeptide chain. By using a linker that is too short to allow pairing between the two variable domains on the same chain, the diabody domains are forced to pair with the binding domains of another chain and create two antigen-binding sites.

Fab fragments comprise the variable and constant domains of the light chain as well as the variable and first constant domain (CHI) of the heavy chain. Fab' fragments differ from Fab fragments by the addition of a few residues at the carboxyl terminus of the CH1 domain to include one or more cysteines from the antibody hinge region. Fab' fragments can be produced by cleavage of the disulfide bond at the hinge cysteines of the pepsin digestion product of F(ab')2. Additional enzymatic and chemical treatments of antibodies can yield other functional fragments of interest.

The term "linear Fab" refers to a tetravalent antibody as described by Miller et al. (Miller et al. (2003), J Immunol. 170: 4854-4861). The "linear Fab" is composed of a tandem of the same CH1-VH domain, paired with the identical light chain at each CH1-VH position. These molecules have been developed in order to increase the valency of an antibody to enhance its functional affinity through the avidity effect, but they are monospecific.

As used herein, the "monoclonal antibody" refers to a population of identical antibodies, i.e., each individual antibody molecule in the population of the monoclonal antibody is identical to other antibody molecules. This property is in contrast to the property of a polyclonal population of antibodies, which comprises antibodies having a plurality of different sequences. Monoclonal antibodies can be prepared by many well-known methods (Smith et al. (2004) J. Clin. Pathol. 57,912-917; and Nelson et al., J Clin Pathol (2000), 53,111-117). For example, monoclonal antibodies can be prepared by immortalizing B cells, e.g., by fusion with myeloma cells to produce a hybridoma cell line or by infecting B cells with a virus such as EBV. Recombinant techniques can also be used to prepare antibodies from a clonal population of host cells by in vitro transforming the host cells with a plasmid carrying an artificial sequence of a nucleotide encoding the antibodies.

As used herein, the term "hybridoma" or "hybridoma cell" refers to a cell or cell line (typically a myeloma or lymphoma cell) produced by the fusion of antibody-producing lymphocytes and antibody-producing cancer cells. As is known to one of ordinary skill in the art, hybridomas can proliferate and continue to supply to produce specific monoclonal antibodies. Methods for producing hybridomas are known in the art (see, for example, Harlow & Lane, 1988). The term "hybridoma" or "hybridoma cell" when referred herein also includes subclones and progeny cells of the hybridoma.

As used herein, a full length antibody is an antibody comprising two full length heavy chains (e.g., VH-CH1-CH2-CH3 or VH-CH1-CH2-CH3-CH4) and two full length light chains (VL-CL) and a hinge region, for example, an antibody naturally produced by secretion of the antibody by B cells, and a synthetically-produced antibody having the same domain.

As used herein, dsFv refers to an Fv having an engineered intermolecular disulfide bond which stabilizes the VH-VL pair.

As used herein, a scFv fragment refers to an antibody fragment comprising a variable light chain (VL) and a variable heavy chain (VH) covalently linked by a polypeptide linker in any order. The linker is of a length such that the two variable domains are bridged without substantial interference. Exemplary linkers are (Gly-Ser)n residues with some Glu or Lys residues dispersed throughout to increase solubility.

The term "chimeric antibody" refers to an antibody in which the variable region sequence is derived from one species and the constant region sequence is derived from another species, for example, in which the variable region sequence is derived from a mouse antibody and the constant region sequence is derived from a human antibody.

"Humanized" antibody refers to a non-human (e.g., mouse) antibody form that is a chimeric immunoglobulin, immunoglobulin chain, or a fragment thereof (e.g., Fv, Fab, Fab', F(ab')2 or other antigen-binding subsequences of antibodies), and contains minimal sequences derived from non-human immunoglobulins. Preferably, the humanized antibody is a human immunoglobulin (recipient antibody) in which residues of the complementarity determining region (CDR) of the recipient antibody are replaced by residues of the CDR of a non-human species (donor antibody) having the desired specificity, affinity, and capacity, such as mouse, rat, rabbit.

Furthermore, it is also possible in humanization process to mutate amino acid residues within the CDR1, CDR2 and/or CDR3 regions of VH and/or VL, thereby improving one or more binding properties (e.g., affinity) of the antibody. Mutations can be introduced, for example, by PCR-mediation, the effect of the mutations on antibody binding or other functional properties can be assessed using *in vitro* or *in vivo* assays as described herein. Typically, conservative mutations are introduced. Such mutations may be amino acid substitutions, additions or deletions. In addition, the number of mutations within the CDRs is usually one or at most two. Thus, the humanized antibodies of the present invention also encompass antibodies comprising one or two amino acid mutations within the CDRs.

As used herein, the term "epitope" refers to any antigenic determinant on an antigen to which the paratope of an antibody binds. Epitopic determinants usually comprise chemically active surface groupings of molecules such as amino acids or sugar side chains, and typically have specific three dimensional structural characteristics, as well as specific charge characteristics.

As used herein, a variable domain or variable region is a specific Ig domain of the heavy or light chain of an antibody, comprising an amino acid sequence that varies between different antibodies. Each light chain and each heavy chain have a variable region domain VL and VH, respectively. The variable domain provides antigen specificity and is therefore responsible for antigen recognition. Each variable region comprises a CDR and a framework region (FR), wherein the CDR is a part of an antigen-binding site domain.

As used herein, "antigen-binding domain" and "antigen-binding site" are used synonymously to refer to a domain within an antibody that recognizes and physically interacts with a cognate antigen. Native conventional full length antibody molecules have two conventional antigen-binding sites, each comprising a heavy chain variable region portion and a light chain variable region portion. Conventional antigen-binding sites comprise a loop connecting the antiparallel beta strands within the variable region domain. The antigen-binding site may comprise other portions of the variable region domain. Each conventional antigen-binding site comprises 3 hypervariable regions from the heavy chain and 3 hypervariable regions from the light chain. The hypervariable regions are also referred to as complementarity determining regions (CDRs).

As used herein, a functional region of a VH domain is at least a portion of an intact VH domain that retains at least part of the binding specificity of the entire VH domain (e.g., by retaining one or more CDRs of the entire VH domain), such that the functional region of the VH domain binds to the antigen either alone or in combination with another antibody domain (e.g., a VL domain) or a region thereof. The functional region of an exemplary VH domain is a region comprising CDR1, CDR2 and/or CDR3 of the VH domain.

As used herein, a functional region of a VL domain is at least a portion of an intact VL domain that retains at least part of the binding specificity of the entire VL domain (e.g., by retaining one or more CDRs of the entire VL domain), such that the functional region of the VL domain binds to the antigen either alone or in combination with another antibody domain (e.g., a VH domain) or a region thereof. The functional region of an exemplary VL domain is a region comprising CDR1, CDR2 and/or CDR3 of the VL domain.

As used herein, "specifically bind" or "immunospecifically binding" with respect to an antibody or antigen-binding fragment thereof is used interchangeably herein and refers to the ability of an antibody or antigen-binding fragment to form one or more non-covalent bonds with an alloantigen via non-covalent interactions between the antibody and the antibody-binding site of the antigen. The antigen can be an isolated antigen or present in a tumor cell. Typically, an antibody that immunospecifically binds (or specifically binds) to an antigen binds to the antigen with an affinity constant Ka of about 1 × 10⁷M⁻¹ or 1 × 10⁸M⁻¹ or more (or with a dissociation constant (Kd) of 1 × 10⁻⁷M or 1 × 10⁻⁸M or less). Affinity constants can be determined by standard kinetic methods of antibody responses, for example, immunoassays, surface plasmon resonance (SPR) (Rich and Myszka (2000) Curr. Opin. Biotechnol 11:54; Englebienne (1998) Analyst. 123:1599), isothermal titration calorimetry (ITC) or other kinetic interaction assays known in the art (see, for example, Paul, ed., Fundamental Immunology, 2nd ed., Raven Press, New York, pages 332-336(1989); see also US Patent No. 7,229,619, which describes exemplary SPR and ITC methods for calculating the binding affinity of antibodies). Instruments and methods for detecting and monitoring the rate of binding in real time are known and commercially available (see, BiaCore 2000, Biacore AB, Upsala, Sweden and GE Healthcare Life Sciences; Malmqvist (2000) Biochem.Soc.Trans.27:335).

As used herein, the term "competing" with respect to an antibody means that a first antibody or antigen-binding fragment thereof binds to an epitope in a manner sufficiently similar to a second antibody or antigen-binding fragment thereof, and thus the binding result of the first antibody to the associated epitope thereof is detectably reduced in the presence of the second antibody compared to that in the absence of the second antibody; alternatively, the binding of the second antibody to the associated epitope thereof may be, but not necessarily, detectably reduced in the presence of the first antibody compared to that in the absence of the first antibody. That is to say, the first antibody can inhibit the binding of the second antibody to the epitope thereof, however, the second antibody is not necessarily to inhibit the binding of the first antibody to the corresponding epitope thereof. However, in the case where each antibody can detectably inhibit the binding of another antibody to the associated epitope or ligand thereof, whether in an identical, higher or lower degree, the antibodies are referred to as "cross-competitively" binding to the corresponding epitope thereof. Both competing and cross-competing antibodies are encompassed by the present invention. Regardless of the mechanism of such competing or cross-competing (e.g., steric hindrance, conformational change, or binding to a common epitope or a fragment thereof), a person skilled in the art will recognize that, based on the teachings provided in the present invention, that such competing and/or cross-competing antibodies are encompassed by the present invention and can be used in the methods disclosed herein.

As used herein, "polypeptide" refers to two or more amino acids which are linked covalently. The terms "polypeptide" and "protein" are used interchangeably herein.

"Isolated protein", "isolated polypeptide" or "isolated antibody" refers to a protein, polypeptide or antibody: (1) is not associated with naturally associated components that accompany it in its native state; (2) is free of other proteins from the same species; (3) is expressed by a cell from a different species; or (4) does not occur in nature. Thus, a polypeptide that is chemically synthesized or synthesized in a cellular system different from the cell from which it naturally originates will be "isolated" from its naturally associated components. A protein may also be rendered substantially free of naturally associated components by isolation, i.e., using the protein purification technique well known in the art.

Suitable conservative amino acid substitutions in peptides or proteins are known to a person skilled in the art and can generally be carried out without altering the biological activity of the resulting molecule. Typically, a person skilled in the art will recognize that a single amino acid substitution in a non-essential region of a polypeptide does not substantially alter the biological activity (see, for example, Watson et al., Molecular Biology of the Gene, 4th Edition, 1987, The Benjamin/Cummings Pub.co., p.224).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), which are usually linked by a phosphodiester bond.

As used herein, an isolated nucleic acid molecule is a nucleic acid molecule which is isolated from other nucleic acid molecules present in the natural source of the nucleic acid molecule. An "isolated" nucleic acid molecule of, for example, a cDNA molecule, can be substantially free of other cellular material or culture medium when prepared by recombinant techniques, or substantially free of chemical precursors or other chemical components during the chemical synthesis. Exemplary isolated nucleic acid molecules provided herein include isolated nucleic acid molecules encoding the provided antibodies or antigen-binding fragments.

As used herein, "operably linked" with respect to a nucleic acid sequence, region, element or domain means that the nucleic acid regions are functionally related to each other. For example, a promoter can be operably linked to a nucleic acid encoding a polypeptide which enables the promoter to regulate or mediate the transcription of the nucleic acid.

As used herein, the term "nucleic acid molecule" is intended to include DNA molecule and RNA molecule. The nucleic acid molecule can be single-stranded or double-stranded, and can be cDNA.

The "conservative sequence modification" of the sequence described in the sequence listing described herein is also provided, that is, nucleotide and amino acid sequence modifications that do not eliminate the binding of an antibody encoded by a nucleotide sequence or containing an amino acid sequence to an antigen. These conservative sequence modifications include conservative nucleotide and amino acid substitutions, as well as, nucleotide and amino acid additions and deletions. For example, modifications can be introduced into the sequence listing described herein by standard techniques known in the art (such as site-directed mutagenesis and PCR-mediated mutagenesis). Conservative sequence modifications include conservative amino acid substitutions in which amino acid residues are replaced with amino acid residues with similar side chains. The family of amino acid residues with similar side chains is already defined in the art. These families include amino acids with basic side chains (for example, lysine, arginine, histidine), amino acids with acidic side chains (for example, aspartic acid, glutamic acid), and amino acids with uncharged polar side chains (for example, glycine, asparagine, glutamine, serine, threonine, tyrosine, cysteine, tryptophan), amino acids with non-polar side chains (for example, alanine, valine, leucine, isoleucine, proline, phenylalanine, methionine), amino acids with β-branched side chains (for example, threonine, valine, isoleucine) and amino acids with aromatic side chains (for example, tyrosine, phenylalanine, tryptophan, histidine). Therefore, the predicted non-essential amino acid residue in the anti-CD73 antibody is preferably replaced by another amino acid residue from the same side chain family. The methods for identifying conservative substitutions of nucleotides and amino acids that do not eliminate antigen binding are well known in the art (for example, see Brummell et al., Biochem. 32:1180-1187 (1993); Kobayashi et al., Protein Eng. 12 (10)) : 879-884 (1999); and Burks et al., Proc. Natl. Acad. Sci. USA 94:412-417 (1997)).

Alternatively, in another embodiment, mutations can be randomly introduced along all or part of the coding sequence of the anti-CD73 antibody by, for example, saturation mutagenesis, and the resulting modified anti-CD73 antibody can be screened for the improved binding activity.

For nucleic acids, the term "substantial homology" means that two nucleic acids or their designated sequences in the optimally alignment and comparison (wherein appropriate insertion or deletion of nucleotides) are identical at least about 80% of the nucleotides, usually at least about 90% to 95%, and more preferably at least about 98% to 99.5% of the nucleotides. Alternatively, it exists substantial homology when the segment hybridizes to the complement of the strand under selective hybridization conditions.

As used herein, "expression" refers to the process by which a polypeptide is produced by transcription and translation of a polynucleotide. The expression level of a polypeptide can be assessed using any method known in the art, including, for example, methods for determining the amount of polypeptides produced from a host cell. Such methods can include, but not limited to, quantification of polypeptides in cell lysates by ELISA, Coomassie blue staining after gel electrophoresis, Lowry protein assays, and Bradford protein assays.

As used herein, a "host cell" refers to a cell used to receive, maintain, replicate, and amplify a vector. Host cells can also be used to express the polypeptide encoded by the vector. The nucleic acid contained in the vector replicates when the host cell divides, thereby amplifying the nucleic acid. Host cells can be eukaryotic cells or prokaryotic cells. Suitable host cells include, but not limited to, CHO cells, various COS cells, HeLa cells, HEK cells such as HEK 293 cells.

"Codon optimization" refers to a method for modifying a nucleic acid sequence for enhanced expression in a host cell of interest by replacing at least one codon (e.g., about or more than about 1, 2, 3, 4, 5, 10, 15, 20, 25, 50 or more codons) of the native sequence with codons that are more frequently or most frequently used in the genes of the host cell while maintaining the native amino acid sequence. Various species exhibit particular bias for certain codons of a particular amino acid. Codon preference (a difference in codon usage between organisms) usually correlates with the efficiency of translation of messenger RNA (mRNA), which is in turn believed to be dependent on the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the Codon Usage Database available at www.kazusa.orjp/codon/, and these tables can be adapted in different ways. See, Nakamura Y. et al., "Codon usage tabulated from the international DNA sequence databases: status for the year 2000. Nucl. Acids Res., 28:292(2000).

As used herein, a "vector" is a replicable nucleic acid and when a vector is transformed into a suitable host cell, one or more heterologous proteins can be expressed from the vector. The vector as used herein includes the vector into which a nucleic acid encoding a polypeptide or a fragment thereof can be introduced, typically, by restriction digestion and ligation. The vector as used herein also includes the vector comprising a nucleic acid encoding a polypeptide. The vector is used to introduce a nucleic acid encoding a polypeptide into a host cell, to amplify the nucleic acid, or to express/display a polypeptide encoded by a nucleic acid. The vector typically remains free, but can be designed to integrate the gene or a part thereof into the chromosome of the genome. The vectors of artificial chromosomes, such as yeast artificial vectors and mammalian artificial chromosomes, are also taken into consideration. The selection and use of such vehicles are well known to a person skilled in the art.

As used herein, vectors also include "viral vectors" or "vectors of viruses". The vector of virus is an engineered virus, which can be operably linked to an exogenous gene to transfer (as a vehicle or shuttle) the exogenous gene into a cell.

As used herein, an "expression vector" includes a vector capable of expressing DNA, which can be operably linked to a regulatory sequence, such as a promoter region, that is capable of affecting expression of such DNA fragments. Such additional fragments may include promoter and terminator sequences, and may optionally include one or more origins of replication, one or more selectable markers, an enhancer, a polyadenylation signal, etc. Expression vectors are generally derived from plasmid or viral DNA, or may contain elements of both. Thus, an expression vector refers to a recombinant DNA or RNA construct, such as a plasmid, phage, recombinant virus, or other vector which, when introduced into a suitable host cell, results in expression of the cloned DNA. Suitable expression vectors are well known to a person skilled in the art and include expression vectors which are replicable in eukaryotic cells and/or prokaryotic cells, and expression vectors which retain free or expression vectors which are integrated into the genome of a host cell.

As used herein, "treating" an individual having a disease or condition means that the symptoms of the individual are partially or totally relieved, or unchanged after treatment. Thus, treating includes preventing, treating, and/or curing. Preventing refers to the prevention of underlying diseases and/or prevention of worsening symptoms or disease progression. Treating also includes any pharmaceutical use of any of the provided antibodies or antigen-binding fragments thereof and the compositions provided herein.

As used herein, "therapeutic effect" refers to an effect caused by the treatment in an individual that alters, generally ameliorates or improves the symptoms of diseases or conditions, or cures diseases or conditions.

As used herein, "therapeutically effective amount" or "therapeutically effective dose" refers to an amount of a substance, compound, material, or composition comprising the compound which is at least sufficient to produce a therapeutic effect when administered to a subject. Thus, it is an amount essential for preventing, curing, ameliorating, blocking or partially blocking the symptoms of a disease or condition.

As used herein, "prophylactically effective amount" or "prophylactically effective dose" refers to an amount of a substance, compound, material or composition comprising the compound which exerts the desired prophylactic effect when administered to a subject, e.g., to prevent or delay the onset or recurrence of a disease or symptom, reduce the likelihood of the occurring or recurring of a disease or symptom. The complete prophylactic effective dose does not necessarily occur by administration of one dose and can occur upon administration of a series of doses. Thus, a prophylactically effective amount can be administered in one or more administrations.

As used herein, the term "patient" refers to a mammal, such as human.

### II. Detailed Description of Embodiments

In one embodiment, the present disclosure provides an antibody or antigen-binding portion thereof that binds to human CD73, comprising heavy chain CDR selected from the amino acid sequences SEQ ID NO: 6-8, 16-18, 26-28, 36-38, 46-48, 56-58, 66-68, 76-78, 86-88,96-98,106-108,116-118,126-128,131-133,136-138, 141-143, 146-148 or any variant thereof, and/or light chain CDR selected from the amino acid sequences SEQ ID NO: 11-13, 21-23, 31-33, 41-43, 51-53, 61-63, 71-73, 81-83, 91-93, 101-103, 113-123, 153, 156-168, 161-163, 166-168, 171-173 or any variant thereof.

The antibody or antigen-binding portion thereof according to the previous aspect, comprising a heavy chain CDR1 selected from the amino acid sequences SEQ ID NO: 6, 16, 26, 36, 46, 56, 66, 76, 86, 96, 106, 116, 126, 131, 136, 141, 146 or any variant thereof, a heavy chain CDR2 selected from the amino acid sequences SEQ ID NO: 7, 17, 27, 37, 47, 57, 67, 77, 87, 97, 107, 117, 127, 132, 137, 142, 147 or any variant thereof, a heavy chain CDR3 selected from the amino acid sequences SEQ ID NO: 8, 18, 28, 38, 48, 58, 68, 78, 88, 98, 108, 118, 128, 133, 143, 148 or any variant thereof; and/or a light chain CDR1 selected from the amino acid sequences SEQ ID NO: 11, 21, 31, 41, 51, 61, 71, 81, 91, 101, 111, 121, 151, 156, 161, 166, 171 or any variant thereof, a light chain CDR2 selected from the amino acid sequences SEQ ID NO: 12, 22, 32, 42, 52, 62, 72, 82, 92, 102, 112, 122, 152, 157, 162, 167, 172 or any variant thereof, a light chain CDR3 selected from the amino acid sequences SEQ ID NO: 13, 23, 33, 43, 53, 63, 73, 83, 93, 103, 113, 123, 153, 158, 163, 168, 173 or any variant thereof.

The antibody or antigen-binding portion thereof according to any one of the preceding aspects, comprising a heavy chain variable region selected from the amino acid sequences SEQ ID NO: 5, 15, 25, 35, 45, 55, 65, 75, 85, 95, 105, 115, 125, 130, 135, 140, 145 or any variant thereof, and/or a light chain variable region selected from amino acid sequences SEQ ID NO: 10, 20, 30, 40, 50, 60, 70, 80, 90, 100, 110, 120, 150, 155, 160, 165, 170 or any variant thereof.

A nucleic acid molecule encoding the antibody or antigen-binding portion thereof according to any one of the preceding aspects, comprising a nucleic acid sequence selected from SEQ ID NO: 9, 19, 29, 39, 49, 59, 69, 79, 89, 99, 109, 119, 129, 134, 139, 144, 149 or any variant thereof, and/or a nucleic acid sequence selected from SEQ ID NO:14, 24, 34, 44, 54, 64, 74, 84, 94, 104, 114, 124, 154, 159, 164, 174 or any variant thereof, preferably, the nucleic acid molecule comprising a nucleic acid sequence SEQ ID NO: 149 and a nucleic acid sequence SEQ ID NO: 174.

In one aspect, the present disclosure relates to an antibody or antigen-binding portion thereof that binds to human CD73, comprising a heavy chain variable region selected from the amino acid sequence SEQ ID NO: 125 or any variant thereof, and/or a light chain variable region selected from the amino acid sequence SEQ ID NO: 150 or any variant thereof.

In one aspect, the present disclosure relates to an antibody or antigen-binding portion thereof that binds to human CD73, comprising a heavy chain variable region selected from the amino acid sequence SEQ ID NO: 125 or any variant thereof, and/or a light chain variable region selected from the amino acid sequence SEQ ID NO: 155 or any variant thereof.

In one aspect, the present disclosure relates to an antibody or antigen-binding portion thereof that binds to human CD73, comprising a heavy chain variable region selected from the amino acid sequence SEQ ID NO: 125 or any variant thereof, and/or a light chain variable region selected from the amino acid sequence SEQ ID NO: 160 or any variant thereof.

In one aspect, the present disclosure relates to an antibody or antigen-binding portion thereof that binds to human CD73, comprising a heavy chain variable region selected from the amino acid sequence SEQ ID NO: 125 or any variant thereof, and/or a light chain variable region selected from the amino acid sequence SEQ ID NO: 165 or any variant thereof.

In one aspect, the present disclosure relates to an antibody or antigen-binding portion thereof that binds to human CD73, comprising a heavy chain variable region selected from the amino acid sequence SEQ ID NO: 125 or any variant thereof, and/or a light chain variable region selected from the amino acid sequence SEQ ID NO: 170 or any variant thereof.

In one aspect, the present disclosure relates to an antibody or antigen-binding portion thereof that binds to human CD73, comprising a heavy chain variable region selected from the amino acid sequence SEQ ID NO: 130 or any variant thereof, and/or a light chain variable region selected from the amino acid sequence SEQ ID NO: 150 or any variant thereof.

In one aspect, the present disclosure relates to an antibody or antigen-binding portion thereof that binds to human CD73, comprising a heavy chain variable region selected from the amino acid sequence SEQ ID NO: 130 or any variant thereof, and/or a light chain variable region selected from the amino acid sequence SEQ ID NO: 155 or any variant thereof.

In one aspect, the present disclosure relates to an antibody or antigen-binding portion thereof that binds to human CD73, comprising a heavy chain variable region selected from the amino acid sequence SEQ ID NO: 130 or any variant thereof, and/or a light chain variable region selected from the amino acid sequence SEQ ID NO: 160 or any variant thereof.

In one aspect, the present disclosure relates to an antibody or antigen-binding portion thereof that binds to human CD73, comprising a heavy chain variable region selected from the amino acid sequence SEQ ID NO: 130 or any variant thereof, and/or a light chain variable region selected from the amino acid sequence SEQ ID NO: 165 or any variant thereof.

In one aspect, the present disclosure relates to an antibody or antigen-binding portion thereof that binds to human CD73, comprising a heavy chain variable region selected from the amino acid sequence SEQ ID NO: 130 or any variant thereof, and/or a light chain variable region selected from the amino acid sequence SEQ ID NO: 170 or any variant thereof.

In one aspect, the present disclosure relates to an antibody or antigen-binding portion thereof that binds to human CD73, comprising a heavy chain variable region selected from the amino acid sequence SEQ ID NO: 135 or any variant thereof, and/or a light chain variable region selected from the amino acid sequence SEQ ID NO: 150 or any variant thereof.

In one aspect, the present disclosure relates to an antibody or antigen-binding portion thereof that binds to human CD73, comprising a heavy chain variable region selected from the amino acid sequence SEQ ID NO: 135 or any variant thereof, and/or a light chain variable region selected from the amino acid sequence SEQ ID NO: 155 or any variant thereof.

In one aspect, the present disclosure relates to an antibody or antigen-binding portion thereof that binds to human CD73, comprising a heavy chain variable region selected from the amino acid sequence SEQ ID NO: 135 or any variant thereof, and/or a light chain variable region selected from the amino acid sequence SEQ ID NO: 160 or any variant thereof.

In one aspect, the present disclosure relates to an antibody or antigen-binding portion thereof that binds to human CD73, comprising a heavy chain variable region selected from the amino acid sequence SEQ ID NO: 135 or any variant thereof, and/or a light chain variable region selected from the amino acid sequence SEQ ID NO: 165 or any variant thereof.

In one aspect, the present disclosure relates to an antibody or antigen-binding portion thereof that binds to human CD73, comprising a heavy chain variable region selected from the amino acid sequence SEQ ID NO: 135 or any variant thereof, and/or a light chain variable region selected from the amino acid sequence SEQ ID NO: 170 or any variant thereof.

In one aspect, the present disclosure relates to an antibody or antigen-binding portion thereof that binds to human CD73, comprising a heavy chain variable region selected from the amino acid sequence SEQ ID NO: 140 or any variant thereof, and/or a light chain variable region selected from the amino acid sequence SEQ ID NO: 150 or any variant thereof.

In one aspect, the present disclosure relates to an antibody or antigen-binding portion thereof that binds to human CD73, comprising a heavy chain variable region selected from the amino acid sequence SEQ ID NO: 140 or any variant thereof, and/or a light chain variable region selected from the amino acid sequence SEQ ID NO: 155 or any variant thereof.

In one aspect, the present disclosure relates to an antibody or antigen-binding portion thereof that binds to human CD73, comprising a heavy chain variable region selected from the amino acid sequence SEQ ID NO: 140 or any variant thereof, and/or a light chain variable region selected from the amino acid sequence SEQ ID NO: 160 or any variant thereof.

In one aspect, the present disclosure relates to an antibody or antigen-binding portion thereof that binds to human CD73, comprising a heavy chain variable region selected from the amino acid sequence SEQ ID NO: 140 or any variant thereof, and/or a light chain variable region selected from the amino acid sequence SEQ ID NO: 165 or any variant thereof.

In one aspect, the present disclosure relates to an antibody or antigen-binding portion thereof that binds to human CD73, comprising a heavy chain variable region selected from the amino acid sequence SEQ ID NO: 140 or any variant thereof, and/or a light chain variable region selected from the amino acid sequence SEQ ID NO: 170 or any variant thereof.

In one aspect, the present disclosure relates to an antibody or antigen-binding portion thereof that binds to human CD73, comprising a heavy chain variable region selected from the amino acid sequence SEQ ID NO: 145 or any variant thereof, and/or a light chain variable region selected from the amino acid sequence SEQ ID NO: 150 or any variant thereof.

In one aspect, the present disclosure relates to an antibody or antigen-binding portion thereof that binds to human CD73, comprising a heavy chain variable region selected from the amino acid sequence SEQ ID NO: 145 or any variant thereof, and/or a light chain variable region selected from the amino acid sequence SEQ ID NO: 155 or any variant thereof.

In one aspect, the present disclosure relates to an antibody or antigen-binding portion thereof that binds to human CD73, comprising a heavy chain variable region selected from the amino acid sequence SEQ ID NO: 145 or any variant thereof, and/or a light chain variable region selected from the amino acid sequence SEQ ID NO: 160 or any variant thereof.

In one aspect, the present disclosure relates to an antibody or antigen-binding portion thereof that binds to human CD73, comprising a heavy chain variable region selected from the amino acid sequence SEQ ID NO: 145 or any variant thereof, and/or a light chain variable region selected from the amino acid sequence SEQ ID NO: 165 or any variant thereof.

In one aspect, the present disclosure relates to an antibody or antigen-binding portion thereof that binds to human CD73, comprising a heavy chain variable region selected from the amino acid sequence SEQ ID NO: 145 or any variant thereof, and/or a light chain variable region selected from the amino acid sequence SEQ ID NO: 170 or any variant thereof.

An antibody or antigen binding portion thereof that binds to human CD73, comprising three heavy chain variable regions CDRs and three light chain variable regions CDRs that are respectively in a pair of heavy chain and light chain variable region selected from the group: (a) SEQ ID NO: 125 and 150; (b) SEQ ID NO: 125 and 155; (c) SEQ ID NO: 130 and 150; (d) SEQ ID NO: 130 and 155; (e) SEQ ID NO: 135 and 160; (f) SEQ ID NO: 135 and 165; (g) SEQ ID NO: 140 and 160; (h) SEQ ID NO:140 and 165; (i) SEQ ID NO:145 and 160; (j) SEQ ID NO: 145 and 170.

An antibody or antigen-binding portion thereof that binds to human CD73, comprising: (a) heavy chain CDR1, CDR2 and CDR3 sequences respectively comprising SEQ ID NO: 126-128, and/or light chain CDR1, CDR2 and CDR3 sequences respectively comprising SEQ ID NO:151-153; (b) heavy chain CDR1, CDR2 and CDR3 sequences respectively comprising SEQ ID NO: 126-128, and/or light chain CDR1, CDR2 and CDR3 sequences respectively comprising SEQ ID NO: 156-158; (c) heavy chain CDR1, CDR2 and CDR3 sequences respectively comprising SEQ ID NO: 131-133, and/or light chain CDR1, CDR2 and CDR3 sequences respectively comprising SEQ ID NO: 151-153; (d) heavy chain CDR1, CDR2 and CDR3 sequences respectively comprising SEQ ID NO: 131-133, and/or light chain CDR1, CDR2 and CDR3 sequences respectively comprising SEQ ID NO: 156-158; (e) heavy chain CDR1, CDR2 and CDR3 sequences respectively comprising SEQ ID NO: 136-138, and/or light chain CDR1, CDR2 and CDR3 sequences respectively comprising SEQ ID NO: 161-163; (f) heavy chain CDR1, CDR2 and CDR3 sequences respectively comprising SEQ ID NO: 136-138, and/or light chain CDR1, CDR2 and CDR3 sequences respectively comprising SEQ ID NO: 166-168; (g) heavy chain CDR1, CDR2 and CDR3 sequences respectively comprising SEQ ID NO: 141-143, and/or light chain CDR1, CDR2 and CDR3 sequences respectively comprising SEQ ID NO: 161-163; (h) heavy chain CDR1, CDR2 and CDR3 sequences respectively comprising SEQ ID NO: 141-143, and/or light chain CDR1, CDR2 and CDR3 sequences respectively comprising SEQ ID NO: 166-168; (i) heavy chain CDR1, CDR2 and CDR3 sequences respectively comprising SEQ ID NO: 146-148, and/or light chain CDR1, CDR2 and CDR3 sequences respectively comprising SEQ ID NO: 161-163; (j) heavy chain CDR1, CDR2 and CDR3 sequences respectively comprising SEQ ID NO: 146-148, and/or light chain CDR1, CDR2 and CDR3 sequences respectively comprising SEQ ID NO: 171-173.

An antibody or antigen-binding portion thereof that binds to human CD73, having at least greater than 60%, 65%, 70%, 75%, 80%, 85%, 90%, 95%, 96%, 97%, 98%, 99% or higher sequence identity to the antibody or antigen-binding portion of any of the foregoing aspects.

A nucleic acid molecule encoding an antibody or antigen-binding portion thereof according to any one of the foregoing aspects, or a nucleic acid molecule having at least greater than 60%, 65%, 70%, 75%, 80%, 85%, 90%, 95%, 96%, 97%, 98%, 99% or higher sequence identity thereto.

In one aspect, the disclosure further includes kits, e.g., the kits comprise the antibodies and fragments, homologs, and derivatives thereof, etc. of the disclosure, such as labeled or cytotoxic conjugates, and instructions for use of the antibody, conjugates that kills certain types of cells, etc. The instructions may comprise guidance for *in vitro, in vivo,* or *ex vivo* use of the antibodies and the conjugates, etc. The antibody may be in a liquid form or in a solid form, typically in a lyophilized form. The kit may contain other suitable agents such as buffers, reconstitution solutions, and other essential ingredients for the intended use. The packaged combination with predetermined amounts of agents and instructions for use are contemplated, wherein the use is, for example, for therapeutic use or for performing diagnostic assays. Where the antibody is labeled, for example, labeled with an enzyme, the kit may comprise a substrate and a cofactor required for the enzyme (e.g., a substrate precursor which provides a detectable chromophore or fluorophore). In addition, other additives such as stabilizers and buffers (e.g., blocking buffers or lysis buffers) may also be comprised. The relative amounts of the various agents can be varied to provide a concentrate of the agent solution, which provides user flexibility, space savings, agent savings, etc. These agents may also be provided in a dry powder form, typically in a lyophilized form, including excipients which, when dissolved, provide an agent solution with an appropriate concentration.

A method for treating cancer, comprising the steps of administering to the mammal a therapeutically effective amount of the antibody or antigen-binding portion thereof, or the nucleic acid molecule, or the vector, or the cell or the pharmaceutical composition of any of the preceding aspects, optionally, the method further comprising administering to the mammal a therapeutically effective amount of anti-PD-1 antibodies.

A method for treating diseases associated with abnormal production of CD73 in a mammal, comprising the steps of administering to the mammal a therapeutically effective amount of the antibody or antigen-binding portion thereof, or the nucleic acid molecule, or the vector, or the cell or the pharmaceutical composition of any of the preceding aspects, optionally, the method further comprising administering to the mammal a therapeutically effective amount of anti-PD-1 antibodies.

Use of the antibody or antigen-binding portion thereof, or the nucleic acid molecule, or the vector, or the cell or the pharmaceutical composition of any of the preceding aspects, in the preparation of a medicament for the treatment of diseases associated with abnormal production of CD73 in a mammal.

Use of the antibody or antigen-binding portion thereof, or the nucleic acid molecule, or the vector, or the cell or the pharmaceutical composition of any of the preceding aspects, in the preparation of a medicament for the treatment of cancer in a mammal.

Use of the antibody or functional fragment thereof or nucleic acid molecule or vector or cell or pharmaceutical composition or kit of any of the preceding aspects in the preparation of a medicament for inhibiting hydrolase activity of CD73.

Use of the antibody or functional fragment thereof or nucleic acid molecule or vector or cell or pharmaceutical composition or kit of any of the preceding aspects in the preparation of a reagent for mediating endocytosis of CD73 on cell surface. According to any one of the preceding aspects, inhibiting the hydrolase activity of CD73 refers to inhibiting the hydrolase activity of membrane-bound CD73, or inhibiting the hydrolase activity of free CD73, or both inhibiting the hydrolase activity of membrane-bound CD73 and inhibiting the hydrolase activity of free CD73.

The antibodies of the present invention are useful in the treatment of mammals. In one embodiment, for example, an antibody of interest or an equivalent is administered to a non-human mammal for the purpose of obtaining preclinical data. Exemplary non-human mammals to be treated include non-human primates, dogs, cats, rodents, and other mammals, on which preclinical studies are performed. Such mammals may be used to establish animal models for a disease to be treated with the antibody or may be used to study toxicity of the antibody of interest. In each of these embodiments, dose escalation studies may be performed on the mammal.

The antibody, whether or not with a second component (such as a therapeutic agent component conjugated thereto), can be used as a therapeutic agent, either alone or in combination with a cytotoxic factor. The present invention relates to antibody-based therapies which comprise administering the antibodies of the present invention to an animal, mammal or human to treat an CD73 mediated disease, disorder or condition.

The term "treating/treatment/treat" as used in the present invention refers to therapeutic treatment and prophylactic or preventive measures. It refers to preventing, curing, reversing, attenuating, ameliorating, minimizing, inhibiting or stopping the harmful effects of the disease state, disease progression, disease-causing factors (e.g., bacteria or viruses) or other abnormal conditions.

Accordingly, the present invention also encompasses multivalent antibodies, including bispecific anti-CD73 antibodies having attached effector molecules, atoms or other substances with diagnostic or therapeutic functions. For example, an antibody may have a radiodiagnostic tag or a radioactive cytotoxic atom or a metal or cytotoxic substance such as a ricin chain, which are attached to the antibody for *in vivo* diagnosis or treatment of cancer.

Furthermore, the antibodies of the present invention may also be used in immunoassays, purification methods, and other methods in which immunoglobulins or fragments thereof are used. Such uses are well known in the art.

Accordingly, the present invention also provides compositions comprising the anti-CD73 antibody or a fragment thereof of the present invention, wherein the antibody is conveniently combined with pharmaceutically acceptable carriers, diluents or excipients, which is a common means in the art.

The term "pharmaceutical composition" as used herein refers to formulations of various preparations. Formulations containing therapeutically effective amounts of multivalent antibodies are sterile liquid solutions, liquid suspensions or lyophilized forms, and optionally contain stabilizers or excipients.

As used herein, the term "disorder" refers to any condition that would benefit from treatment with the antibody of the present invention. This includes chronic and acute disorders or diseases including those pathological conditions that predispose a mammal, especially a human to the disorder. Examples of non-limiting disorders to be treated herein include cancer, inflammation, autoimmune diseases, infections, cardiovascular diseases, respiratory diseases, neurological diseases, and metabolic diseases.

As used herein, the term "cancer" refers to or describes the physiological condition of a mammal, particularly a human, and is typically characterized by examples of unregulated growth of cancer cells including, but not limited to, carcinoma, lymphoma, blastoma, sarcoma, and leukemia.

The antibodies of the present invention may be used as compositions for administration alone or may be used in combination with other active agents.

A nucleic acid encoding the antibody or antigen-binding portion thereof of any of the preceding aspects.

A vector comprising the nucleic acid of any of the preceding aspects.

A cell comprising the vector of any of the preceding aspects.

A pharmaceutical composition comprising the antibody or antigen-binding portion thereof, or the nucleic acid encoding same of any of the preceding aspects, and a pharmaceutically acceptable carrier.

A method for treating cancers, comprising the step of administering to the mammal a therapeutically effective amount of the antibody or antigen-binding portion thereof, or the nucleic acid encoding same of any of the preceding aspects.

A method for treating diseases associated with abnormal production of CD73 in a mammal, comprising the step of administering to the mammal a therapeutically effective amount of the antibody or antigen-binding portion thereof, or the nucleic acid encoding same of any of the preceding aspects.

It will be appreciated that therapeutics in accordance with the described embodiments will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (15th ed, Mack Publishing Company, Easton, Pa. (1975)), particularly Chapter 87 by Blaug, Seymour, therein. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water- in-oil emulsions, emulsions carbowax (polyethylene glycols of various molecular weights), semi-solid gels, and semi-solid mixtures containing carbowax. Any of the foregoing mixtures may be appropriate for use in treatments and therapies in accordance with the present disclosure, provided that the active ingredient in the formulation is not inactivated by the formulation and the formulation is physiologically compatible and tolerable with the route of administration.

Abnormal production of CD73 or aberrant CD73 expression are used interchangeably herein. In one embodiment, the described antibodies may be used as therapeutic agents. Such agents will generally be employed to treat, alleviate, and/or prevent a disease or pathology associated with aberrant CD73 expression, activity and/or signaling in a subject. A therapeutic regimen is carried out by identifying a subject, e.g., a human patient suffering from (or at risk of developing) a disease or disorder associated with aberrant CD73 expression, activity and/or signaling, e.g., a cancer or other neoplastic disorder, using standard methods. An antibody preparation, preferably one having high specificity and high affinity for its target antigen, is administered to the subject and will generally have an effect due to its binding with the target. Administration of the antibody may abrogate or inhibit or interfere with the expression, activity and/or signaling function of the target (e.g., CD73). Administration of the antibody may abrogate or inhibit or interfere with the binding of the target (e.g., CD73) with an endogenous ligand (e.g., SIRP alpha) to which it naturally binds. For example, the antibody binds to the target and modulates, blocks, inhibits, reduces, antagonizes, neutralizes, or otherwise interferes with CD73 expression, activity and/or signaling. In some embodiments an antibody having heavy and light chain CDRs with the amino acid sequences described in Table 2 may be administered to a subject in order to treat a disease or disorder associated with aberrant CD73 expression. In one embodiment the disease or disorder associated with aberrant CD73 expression may be cancer.

As a non-limiting example, diseases or disorders associated with abnormal CD73 expression, activity, and/or signaling include hematological cancers and/or solid tumors. Hematological cancers include, for example, leukemia, lymphoma and myeloma. As a non-limiting example, certain types of leukemia include acute lymphocytic leukemia (ALL); acute myeloid leukemia (AML); chronic lymphocytic leukemia (CLL); chronic myelogenous leukemia (CML); myeloproliferative Disorder/neoplasm (MPDS); and myelodysplastic syndrome. As non-limiting examples, certain types of lymphoma include Hodgkin's lymphoma, low-grade and aggressive non-Hodgkin's lymphoma, Burkitt's lymphoma, and follicular lymphoma (small cell and Large cells). As a non-limiting example, certain types of myeloma include multiple myeloma (MM), giant cell myeloma, heavy chain myeloma, and light chain or Bence-Jones myeloma. Solid tumors include, for example, breast tumors, ovarian tumors, lung tumors, pancreatic tumors, prostate tumors, melanomas, colorectal tumors, lung tumors, head and neck tumors, bladder tumors, esophageal tumors, liver tumors, and kidney tumors.

Symptoms associated with cancer and other neoplastic disorders include, for example, inflammation, fever, general malaise, fever, pain, often localized to the inflamed area, loss of appetite, weight loss, edema, headache, fatigue, rash, anemia, muscle weakness, muscle fatigue, and abdomen Symptoms such as abdominal pain, diarrhea or constipation.

Therapeutically effective amounts of the antibodies described herein generally relate to amounts necessary to achieve therapeutic purposes. As indicated above, that may be binding interaction between the antibody and its target antigen, hindering the function of the target under certain situations. Additionally, the amount administered depends on the binding affinity of the antibody for its specific antigen, and also depends on the rate of clearance of the administered antibodies from the body. As a non-limiting example, a common range of therapeutically effective doses of the antibodies or antibody fragments described herein, may be from about 0.1 mg/kg body weight to about 100 mg/kg body weight. In one embodiment, a therapeutically effective dosage of the antibodies described herein is from about 0.1 mg/kg body weight to about 0.3 mg/kg body weight. In one embodiment, a therapeutically effective dosage of the antibodies described herein is from about 0.4 mg/kg body weight to about 0.6 mg/kg body weight. In one embodiment, a therapeutically effective dosage of the antibodies described herein is from about 0.7 mg/kg body weight to about 0.9 mg/kg body weight. In one embodiment, a therapeutically effective dosage of the antibodies described herein is from about 1.0 mg/kg body weight to about 2.0 mg/kg body weight. In one embodiment, a therapeutically effective dosage of the antibodies described herein is from about 2.0 mg/kg body weight to about 3.0 mg/kg body weight. In one embodiment, a therapeutically effective dosage of the antibodies described herein is from about 3.0 mg/kg body weight to about 4.0 mg/kg body weight. In one embodiment, a therapeutically effective dosage of the antibodies described herein is from about 4.0 mg/kg body weight to about 5.0 mg/kg body weight. In one embodiment, a therapeutically effective dosage of the antibodies described herein is from about 5.0 mg/kg body weight to about 6.0 mg/kg body weight. In one embodiment, a therapeutically effective dosage of the antibodies described herein is from about 6.0 mg/kg body weight to about 7.0 mg/kg body weight. In one embodiment, a therapeutically effective dosage of the antibodies described herein is from about 7.0 mg/kg body weight to about 8.0 mg/kg body weight. In one embodiment, a therapeutically effective dosage of the antibodies described herein is from about 8.0 mg/kg body weight to about 9.0 mg/kg body weight. In one embodiment, a therapeutically effective dosage of the antibodies described herein is from about 9.0 mg/kg body weight to about 10.0 mg/kg body weight. In one embodiment, a therapeutically effective dosage of the antibodies described herein is from about 10.0 mg/kg body weight to about 15.0 mg/kg body weight. In one embodiment, a therapeutically effective dosage of the antibodies described herein is from about 15.0 mg/kg body weight to about 20.0 mg/kg body weight. Common dosing frequencies may range, for example, from once a day to twice daily to once every other day to once a week.

The effectiveness of the treatment can be determined in conjunction with any known method for diagnosing or treating a specific inflammatory-related disorder. The reduction of one or more symptoms of an inflammatory-related disorder can indicate that the antibody confers clinical benefit.

In another embodiment, antibodies directed against CD73 may be used in methods known within the art relating to the localization and/or quantitation of CD73 (e.g., for use in measuring levels of CD73 and/or both CD73 and SIRP alpha within appropriate physiological samples, for use in diagnostic methods, for use in imaging the protein, and the like). In a given embodiment, antibodies specific to CD73, or derivative, fragment, analog or homolog thereof, that contain the antibody derived antigen binding domain, are utilized as pharmacologically active compounds (referred to hereinafter as "therapeutics").

In another embodiment, an antibody specific for CD73 can be used to isolate a CD73 polypeptide, by standard techniques, such as immunoaffinity, chromatography or immunoprecipitation. Antibodies directed against the CD73 protein (or a fragment thereof) can be used to detect the protein in a biological sample. In some embodiments CD73 may be detected in a biological sample as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive materials include ¹²⁵I, ¹³¹I, ³⁵S, or ³H.

In yet another embodiment, an antibody according to this disclosure can be used as an agent for detecting the presence of CD73 (or a protein fragment thereof) in a sample. In some embodiments, the antibody contains a detectable label. Antibodies are polyclonal, or more preferably, monoclonal. An intact antibody, or a fragment thereof (e.g., Fab, scFv, or F(ab')2) is used. The term "labeled", with regard to an antibody, is intended to encompass direct labeling of the antibody by coupling (i.e., physically linking) a detectable substance to the antibody, as well as indirect labeling of the antibody by reactivity with another reagent that is directly labeled. Examples of indirect labeling include detection of a primary antibody using a fluorescently-labeled secondary antibody and end-labeling of an antibody with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of a described embodiment can be used to detect an analyte mRNA, protein, or genomic DNA in a biological sample in vitro as well as in vivo. For example, in vitro techniques for detection of an analyte mRNA include Northern hybridizations and in situ hybridizations. In vitro techniques for detection of an analyte protein include enzyme linked immunosorbent assays (ELISAs), Western blots, immunoprecipitations, and immunofluorescence. In vitro techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, N.J., 1995; "Immunoassay", E. Diamandis and T. Christopoulus, Academic Press, Inc., San Diego, Calif, 1996; and "Practice and Theory of Enzyme Immunoassays", P. Tijssen, Elsevier Science Publishers, Amsterdam, 1985. Furthermore, in vivo techniques for detection of an analyte protein include introducing into a subject a labeled anti-analyte protein antibody. For example, the antibody can be labeled with a radioactive marker whose presence and location in a subject can be detected by standard imaging techniques.

The antibodies described herein and derivatives, fragments, analogs and homologs thereof, can be incorporated into pharmaceutical compositions suitable for administration. Principles and considerations involved in preparing such compositions, as well as guidance in the choice of components are well known in the art, for example, see Remington's Pharmaceutical Sciences: The Science And Practice Of Pharmacy 19th ed. (Alfonso R. Gennaro, et al, editors) Mack Pub. Co., Easton, Pa.: 1995; Drug Absorption Enhancement: Concepts, Possibilities, Limitations, And Trends, Harwood Academic Publishers, Langhorne, Pa., 1994; and Peptide And Protein Drug Delivery (Advances In Parenteral Sciences, Vol. 4), 1991, M. Dekker, New York.

Such compositions typically comprise the antibody and a pharmaceutically acceptable carrier. Where antibody fragments are used, the smallest inhibitory fragment that specifically binds to the binding domain of the target protein may be preferred. For example, based upon the variable-region sequences of an antibody, peptide molecules can be designed that retain the ability to bind the target protein sequence. Such peptides can be synthesized chemically and/or produced by recombinant DNA technology. (See, e.g., Marasco et al, Proc. Natl. Acad. Sci. USA, 90: 7889-7893 (1993)).

As used herein, the term "pharmaceutically acceptable carrier" is intended to include any and all solvents, dispersion media, coatings, antibacterial and antifungal agents, isotonic and absorption delaying agents, etc., which are compatible with pharmaceutical administration. Suitable carriers are described in the most recent edition of Remington's Pharmaceutical Sciences, a standard reference text in the field, which is incorporated herein by reference. Preferred examples of such carriers or diluents include, but not limited to, water, saline, Ringer's solutions, dextrose solution, and 5% human serum albumin. Liposomes and non-aqueous carriers such as fixed oils may also be used. The use of such media and agents with pharmaceutically active substances is well known in the art. Except insofar as any conventional media or agent is incompatible with the antibody, use thereof in the compositions is contemplated.

The pharmaceutical compositions of the embodiments are formulated to be compatible with their intended route of administration. Examples of routes of administration include parenteral, such as intravenous, intradermal, subcutaneous, oral (e.g., inhalation), transdermal (i.e., topical), transmucosal, and rectal administration. Solutions or suspensions used for parenteral, intradermal or subcutaneous administration may include the following components: sterile diluents for injection such as water, saline solution, fixed oil, polyethylene glycols, glycerol, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates and phosphates; and agents for the adjustment of osmotic pressure such as sodium chloride or dextrose. The pH can be adjusted with an acid or a base such as hydrochloric acid or sodium hydroxide. The parenteral formulation can be enclosed in ampoules, disposable syringes or multiple dose vials made of glass or plastic.

Pharmaceutical compositions suitable for injectable use include sterile aqueous solutions (water soluble herein) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It should be stable under the conditions of manufacture and storage and must be preserved against the contaminating action of microorganisms such as bacteria and fungi. The carrier may be a solvent or dispersion medium containing, for example, water, ethanol, polyol (e.g., glycerol, propylene glycol, liquid polyethylene glycol, etc.), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, etc. In many cases, it may be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the antibody or antibodies in the required amount in an appropriate solvent with one or a combination of ingredients enumerated above, as required, followed by filtered sterilization. Generally, dispersions are prepared by incorporating the antibody or antibodies into a sterile carrier which contains a basic dispersion medium and the required other ingredients from those enumerated above. In the case of sterile powders for the preparation of sterile injectable solutions, the methods of preparation are vacuum drying and freeze-drying which yields a powder of the active ingredient plus any additional desired ingredient from a sterile-filtered solution of the above ingredients.

For administration by inhalation, the compound is delivered in the form of an aerosol spray from a pressurized container or dispenser or nebulizer, which contains a suitable propellant such as carbon dioxide.

Systemic administration can also be performed by transmucosal or transdermal means. For transmucosal or transdermal administration, penetrants appropriate for barrier permeation are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, one or more of the antibodies can be formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the antibodies can be prepared with carriers that will protect the antibodies against rapid elimination from the body, such as a sustained release/controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to a person skilled in the art.

For example, these active ingredients can be encapsulated in microcapsules prepared, for example, by coacervation techniques or by interfacial polymerization methods, for example hydroxymethylcellulose or gelatin microcapsules and poly(methylmethacrylate) microcapsules, respectively in colloidal drug delivery systems (e.g., liposomes, albumin microspheres, microemulsions, nanoparticles, and nanocapsules) or in macroemulsions.

A sustained release formulation can be prepared. Examples of suitable sustained release formulations include semipermeable matrices of solid hydrophobic polymers containing the antibodies, which matrices are in form of shaped articles, e.g., films, or microcapsules. Examples of sustained release matrices include polyesters, hydrogels (e.g., poly(2-hydroxyethyl-methylpropionate), or poly(vinyl alcohol)), polylactide (U.S. Patent No. 3,773,919), copolymers of L-glutamic acid and γ ethyl-L-glutamate, non-degradable ethylene-vinyl acetate, degradable lactic acid-glycollic acid copolymers such as LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

Liposomal suspensions (including liposomes targeted to infected cells with monoclonal antibodies against viral antigens) can also be used as pharmaceutically acceptable carriers. These can be prepared according to methods known to a person skilled in the art, for example, methods described in U.S. Pat. No. 4,522,811.

It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of one or more of the antibodies calculated to produce the desired therapeutic effect in association with the required pharmaceutical carriers. The specifications for the dosage unit forms of the embodiments are dictated by and directly dependent on: the unique characteristics of the antibodies and the particular therapeutic effect to be achieved; and the limitations inherent in the art of formulating such antibodies for the treating individuals.

The pharmaceutical composition can be placed in a container, package, or dispenser together with instructions for administration.

The formulation herein may also contain more than one antibody as necessary for the particular indication being treated, preferably those with complementary activities that do not adversely affect each other. Alternatively, or in addition, the composition may comprise an agent that enhances its function, such as a cytotoxic agent, cytokine, chemotherapeutic agent, or growth-inhibitory agent. Such molecules are suitably present in combination in amounts that are effective for the purpose intended.

In one embodiment, one or more of the antibodies can be administered in a combination therapy, i.e., in combination with other agents, such as therapeutic agents (which can be used to treat pathological conditions or disorders, such as various forms of cancer, autoimmune disorders, and inflammatory disorders). The term "in combination with" as used herein refers to administrating agents substantially simultaneously, simultaneously or sequentially. If administered sequentially, the first compound of two compounds is still preferably detected at an effective concentration at the treatment site upon initiation of administration of the second compound.

For example, a combination therapy may comprise one or more antibodies described herein that are co-formulated and/or co-administered with one or more additional therapeutic agents (e.g., one or more cytokine and growth factor inhibitors, immunosuppressants, anti-inflammatory agents, metabolic inhibitors, enzyme inhibitors, and/or cytotoxin or cytostatic agents, as described in more detail below). Such combination therapies may advantageously utilize lower dosages of the administered therapeutic agents, thus avoiding possible toxicities or complications associated with the various monotherapies.

Preferred therapeutic agents for use in combination with the antibodies described herein are those that interfere with different stages of the inflammatory response. In one embodiment, one or more antibodies described herein can be co-formulated and/or co-administered with one or more additional agents such as other cytokines or growth factor antagonists (e.g., soluble receptors, peptide inhibitors, small molecules, ligand fusions); or antibodies or antigen-binding fragments which bind to other targets (e.g., antibodies which bind to other cytokines or growth factors, receptors thereof, or other cell surface molecules); and anti-inflammatory cytokines or agonists thereof. In some embodiments, the therapeutic agent may be a PD-1 antibody, including but not limited to nivolumab or pembrolizumab.

In other embodiments, the antibodies described herein are used as vaccine adjuvants for autoimmune disorders, inflammatory diseases, etc. Combinations of adjuvants for treating these types of disorders are suitable for use in combination with various antigens, wherein the antigens are derived from the targeted autoantigens, i.e., autoantigens involved in autoimmunity, such as myelin basic protein; inflammatory autoantigens, such as amyloid peptide protein; or transplantation antigens, such as alloantigens. Antigens may include peptides or polypeptides derived from proteins and fragments of any of the following: sugars, proteins, polynucleotides or oligonucleotides, autoantigens, amyloid peptide proteins, transplantation antigens, allergens or other macromolecular components. In some examples, more than one antigen is comprised in an antigenic composition.

For the purpose of clarity and a concise description, features are described herein as part of the same or separate embodiments, however, it will be understood that the scope of the present invention may include some embodiments having combinations of all or some of the features described.

### Embodiments

### 1. Expression of CD73 recombinant protein in eukaryotic cells

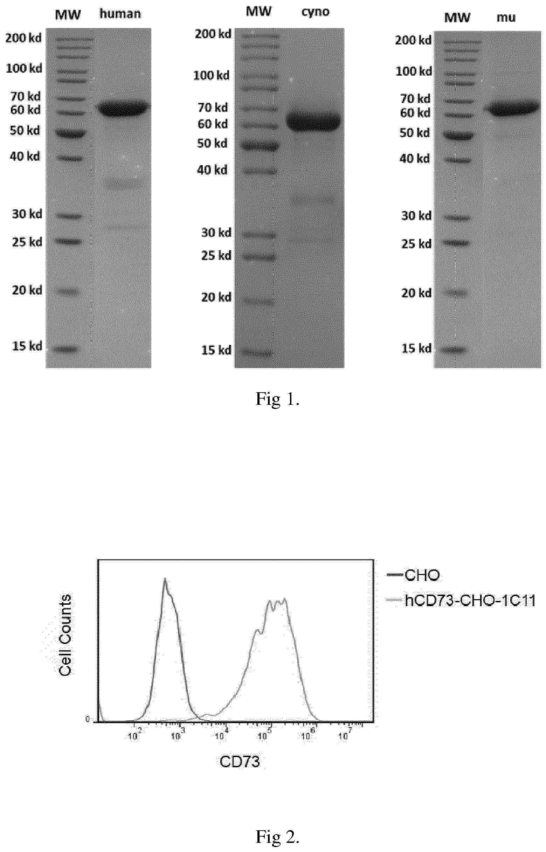

The CD73 cDNA (Sino Biological, HG10904) was amplified by PCR with the upstream primer 5'-ctgagaggtgccagatgttgggagcttacgattttg-3' and the downstream primer 5'-tccgcctccgccgctagccttgatccgaccttcaac-3', to obtain the gene fragment encoding the human CD73 extracellular domain (Trp27-Lys546). The gene fragment was cloned into the downstream of promoter of the eukaryotic expression vector pCMV3, and a tag containing 6 histidines was fused and expressed at the C terminal (see SEQ ID NO: 1 in the sequence list). Because of the high homology between the cynomolgus monkey CD73 extracellular domain and human CD73 extracellular domain, the eukaryotic expression vector expressing the extracellular domain of cynomolgus monkey CD73 (Trp27-Lys546) was obtained by using mutational primers and overlapping PCR (see SEQ ID No: 2 in the sequence list). Similarly, using the purchased mouse CD73 cDNA plasmid gene pMD18-CD73 (Sino Biological, MG50231) as a template, designed the upstream primer 5'-ctgagaggtgccagatgttgggagctcacgatcctg-3', the downstream primer 5'-tccgcctccgccgctagccttgatccgcccttcaacg-3', the gene encoding mouse CD73 (Trp29-Lys549) was cloned into the eukaryotic expression plasmid system (see SEQ ID NO: 3 in the sequence list). After transfecting HEK293.6E cells with these plasmids for 5-7 days, the culture supernatant was collected, and purified by a nickel-chelate column to obtain recombinant human CD73 extracellular domain (hCD73), recombinant monkey CD73 extracellular domain (cynoCD73) or recombinant mouse CD73 extracellular domain protein (muCD73). The SDS-PAGE results were shown in Figure 1.

### 2. Construction of human CD73 stably transformed CHO cell lines

Using the Lenti-Pac HIV Lentiviral Packaging Kit (Guangzhou Funeng Gene Co., Ltd., Cat No. HPK-LvTR-20), the lentiviral vector containing the full-length sequence of human CD73 cDNA (see SEQ ID NO: 4), and the packaging plasmid were co-transfected into 293T cells for lentiviral packaging. The supernatant was collected after transfection for 48 hours, centrifuged at 10,000*g for 10 minutes to remove cell debris, and the culture supernatant containing the lentivirus was obtained, after filtering with a 0.45 µm PES filter membrane, 10 µL was taken out to transduce 1x10⁶ CHO cells, 10 µg/mL puromycin was then added into the medium for selection. Two weeks later, the CD73 expression of CHO-transformed cells was tested by collecting 1x10⁵ cells, adding in 0.5 µg/mL anti-hCD73 antibody (R&D system, Cat No. NBP2-48480), incubating on ice for 45 minutes, and then washing twice with 0.5% BSA/PBS before adding 50µL of 1:300 diluted secondary antibody solution (anti-mouse IgGFc-AF647, Jackson ImmunoResearch), after incubating on ice for 45 minutes and washing off the excess secondary antibody, resuspending the cells with 100µL PBS solution and detecting the FL-4 channel fluorescence signal on flow cytometer. The obtained CHO-CD73 positive clones were subjected to limiting dilution, and finally the hCD73-CHO-1C11 monoclonal cell line stably expressing human full-length CD73 molecules was obtained. The results were shown in Figure 2.

### 3. Mouse immunization and hybridoma clone screening

The female Balb/c mice of 6-8 weeks were taken, the purified recombinant human CD73 extracellular domain protein was sufficiently mixed with the Freund's complete adjuvant (Sigma, Cat No. F5881), then the immunization was performed by subcutaneous multi-point injection, with a dose of 50µg each. Two weeks later, the recombinant human CD73 protein mixed with TiterMax adjuvant (Sigma, Cat No. T2684) were used for immunization, and the immunization was performed alternately with house-constructed expression plasmid containing human CD73 full-length cDNA gene. After detecting that the titer of CD73 antibody produced in the mouse serum reached 1:50,000, the challenging immunization via tail vein was performed with recombinant human CD73 protein or CD73 stably transfected cell line. Three days later, the mice were sacrificed, and the mouse spleen, popliteal lymph nodes, inguinal lymph nodes and iliac lymph nodes were collected, and ground in DMEM medium to obtain a suspension rich in B cells. An appropriate amount of suspension of lymph node and spleen cell was taken and mixed with SP2/0, the cell fusion was performed on electric fusion instrument. The fused cells were seeded in 96-well plates, and cultured at 37°C under 5% CO₂ in DMEM complete medium containing HAT. The growth of hybridoma cells was started to observe in about a week, and when the hybridoma cells grew up to more than 60%, the supernatant was taken out for antibody detection.

### 4. Screening of hybridoma supernatant

1µg/mL recombinant human CD73 extracellular domain protein was prepared with 0.05M pH9.0 bicarbonate buffer, and was added into 96-well microplate (Costar, Cat No. 9018) according to volume of 100µL per well. It was incubated overnight at 4°C, and washed 3 times with PBS the next day, blocked for 2 hours with 200 µL of 2% skimmed milk powder/PBS at room temperature, washed 3 times with PBS, 50 µL hybridoma supernatant was added, after incubating at room temperature for 1 hour, washed 3 times with PBST and PBS repeatedly, then a secondary antibody (anti-mouse IgG-Fc-HRP, Jackson ImmunoResearch, Cat No. 115-035-071) was add and then incubated for 1 hour. After 3 times repeated washing, 50 µL color development substrate (TMB solution, Sigma Cat No.T2885) was added. After standing at 37°C for 5 minutes, 50µL of 2M concentrated sulfuric acid solution was added to stop the reaction, and immediately placed in a microplate reader to read the OD450 absorption.

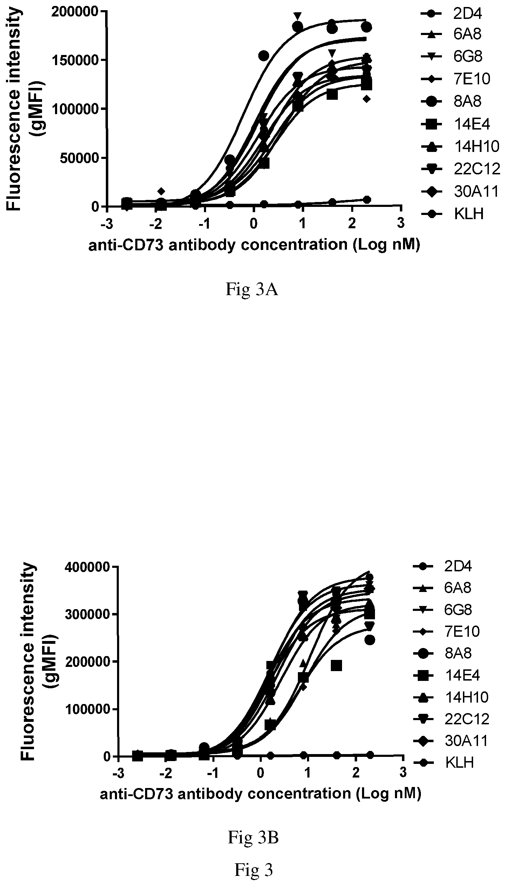

The supernatant of about 1200 CD73 positive clones detected by ELISA, was further verified for the binding with hCD73-CHO-1C11 stably transfected cell lines with high expressing CD73 and human lung degenerative cancer cells (Calu6, ATCC HTB-56) by FACS. The CD73 positive cells were added into a 96-well U-shaped plate at 5×10⁴/well, 50 µL of hybridoma supernatant was further added to each well, incubated at 4°C for 60 minutes, centrifuged and the supernatant was pipetted, washed with 0.5% BSA/PBS, and then 50 µL secondary antibody solution (anti-mouse IgG-Fc-AF647, Jackson ImmunoResearch Cat No. 115-606-071) was added, incubated at 4°C for 45 minutes, then the excess secondary antibody was wash off with 0.5% BSA/PBS, and finally 60µL PBS solution containing 1µg /ml fluorescent dye (propidium iodide, PI, Sigma Cat No. P4170) was used to resuspend the cells and detected using flow cytometry. The results were shown in Figure 3A and Figure 3B.

### 5. Inhibition of CD73 antibody on CD73 hydrolase activity

Bryan C et al. (AACR 2016, US9605080) discovered a CD73 monoclonal antibody, that binds to the domain of the distal membrane end of CD73 through full-length IgG or F(ab')2, which can inhibit the membrane-bound CD73 enzyme activity or the CD73 enzyme activity bound to the magnetic beads, but the Fab with monovalent binding ability only cannot inhibit the CD73 enzyme activity. According to the published sequence (sequence 135, GenBank: ATL10689.1; sequence 8, GenBank: ATL10563.1), the variable region gene of antibody 11F11 was synthesized, and cloned into a plasmid containing human IgG1 constant region, and transiently expressed in HEK293, the antibody 11F11-huG1 was purified by Protein A.

The anti-CD73 hybridoma supernatant obtained by the mouse immunization and the control antibody 11F11-huG1, were verified respectively by the following two experiments for inhibiting the hydrolase activity of CD73 on the cell membrane surface and sCD73 in the solution.

### Assay of inhibiting hydrolase activity of CD73 on cell membrane surface

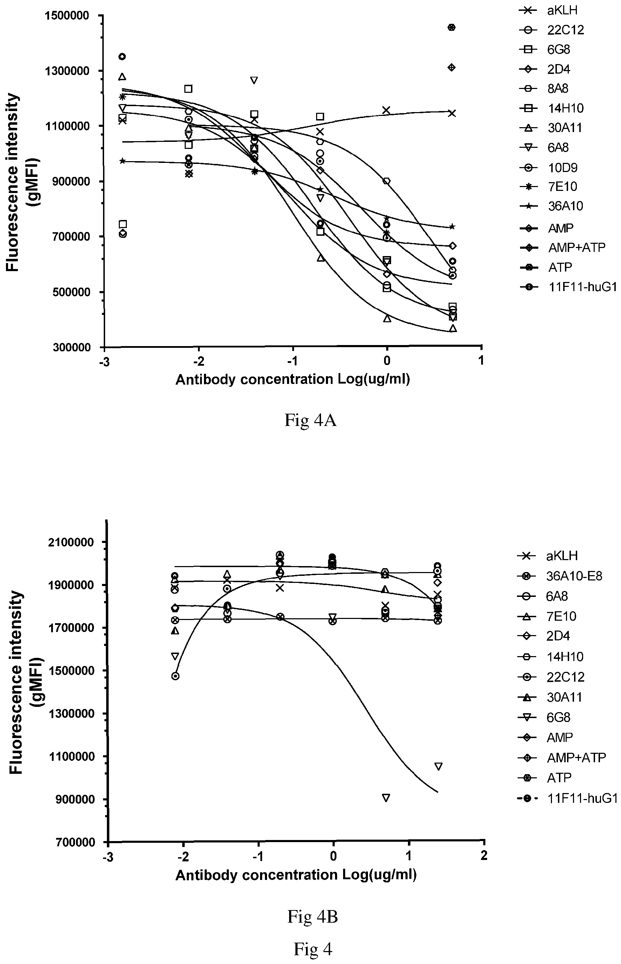

The human lung degenerative cancer cells Calu6 were inoculated in a 96-well U-shaped plate at 3x10⁴ cells/well, and the purified CD73 positive clone antibody with 25µg/mL as the initial concentration, was diluted to 5 concentrations in equal ratio with TM buffer (25mM Tris, pH7.5, 5mM MgC12) in a ratio of 1:5, mixed with cells and then incubated in incubator at 37°C for 20 minutes. After the incubation, the supernatant was discarded, washed twice with PBS, centrifuged at 1500 rpm for 5 minutes, 100µL 180µM AMP (Sigma, Cat No. 01930-5G) solution was added to each well of the experimental group, and an equal amount of TM buffer was added to the control group, incubated in incubator at 37°C for 60 minutes. After the incubation, centrifugation at 1500 rpm for 5 minutes, 50 µL supernatant was mixed with an equal volume of 60 µM ATP (brand: Sigma, Cat NO. A6419-1G) solution in a 96-well black microtiter plate. After reacting at 37°C for 15 minutes, 100µL Celltiter Glo solution (Promega, Cat. No. 7570) was added, placed in a well-plate shaker and shaken at 300g for 2 minutes, kept in the dark for 10 minutes, and detected the fluorescence signals on a microplate reader (BioTek Synergy HT). The results were shown in Figure 4A.

### Assay of inhibiting hydrolase activity of soluble CD73

The anti-CD73 antibody with 60µg/mL as the initial concentration, was diluted to 10 concentration gradients with TM buffer in a ratio of 1:3 in equal, 75µL hCD73-cHis antigen and 25µL antibodies of different concentrations were incubated in a 96-well plate at 37°C for 1 hour, then 100µL AMP/TM buffer was added to each well and incubated at 37°C for 1 hour, then 50µL supernatant was pipetted and mixed with an equal volume of 60µM ATP/TM buffer in a 96-well black microtiter plate. After reacting at 37°C for 15 minutes, 100 µL Celltiter Glo solution was added, placed in a well-plate shaker and shaken at 300g for 2 minutes, kept in the dark for 10 minutes, and finally detected the fluorescence signals on a microplate reader. The results were shown in Figure 4B.

### 6. Species specificity of CD73 antibody

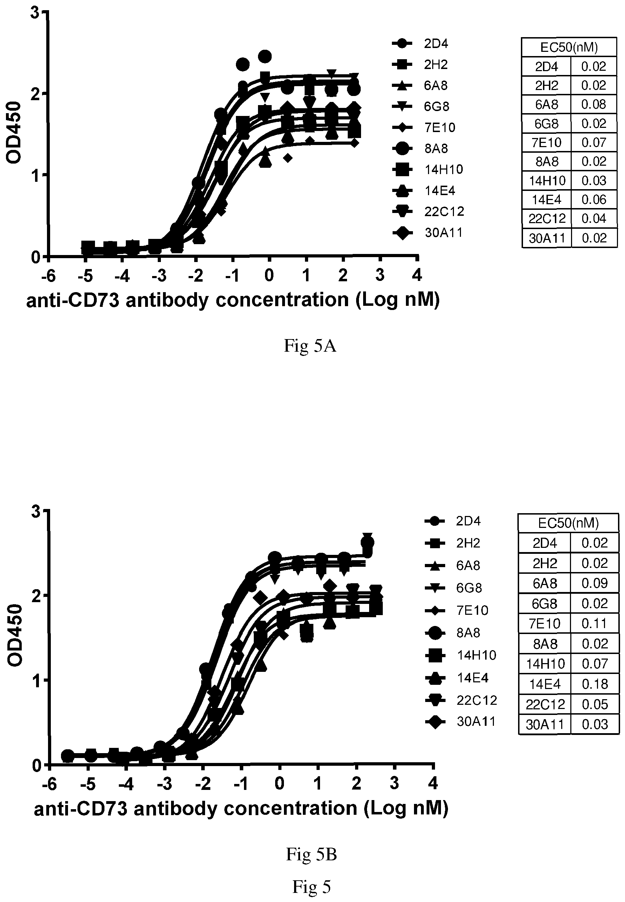

The recombinant human, cynomolgus monkey, and mouse CD73 extracellular domain proteins were diluted respectively with the coating solution to a concentration of 1 µg/mL, added to the immunosorb plate at 100 µL/well, at 4°C overnight. The next day, washed 3 times with PBS, and then blocked with 200 µL 2% skimmed milk powder/PBS for 2 hours at room temperature, and washed 3 times with PBS. 100µL anti-hCD73 antibody with different concentrations was added, and washed 3 times repeatedly with PBST and PBS after incubating for 1 hour at room temperature. 100 µL secondary antibody Anti-hIgG Fc HRP (brand Jackson Cat No. 109-035-098) was added and incubated at room temperature for 1 hour. After 3 times repeated washing, 50µL color development substrate (substrate buffer 9.5 mL, TMB solution 0.5 mL, 3% H₂O₂ 10 µL, mixed well, prepared just before use) was add, kept at 37°C for 5 minutes and then 50µL 2M concentrated sulfuric acid solution was added immediately to stop the reaction, and placed immediately in a microplate reader (BioTek Synergy HT) to read the OD450 absorption. The results were shown in Figure 5A ,Figure 5B.

### 7. Cloning of the DNA sequence of CD73 antibody

10 positive clones of CD73 were selected (Table 1), the RNA of candidate hybridoma monoclonal cells was extracted using TRNzol lysis method, and then single-stranded cDNA was synthesized by reverse transcription kit (Invitrogen, 18080051), and was used as a template to amplify the variable region sequence of the antibody in hybridoma monoclonal cells. The amplified products were sequenced, the obtained heavy chain and light chain variable region sequences of candidate hybridoma are as follows:

### Clone 2D4:

Amino acid sequence of heavy chain variable region

<-------------FR1-----------> CDR1 <----FR2-----> CDR2 <-
---
EVKLVESGGGLVQPGGSLKLSCATSGFTFSDYYMYWVRQTPEKRLEWVAYISNVGGSTYYPDTVK
GRFTIS
----------FR3-------------> CDR3 <---FR4--->
RDNAKNTLYLQMSRLKSEDTAIYYCARGDGYYGYYFDVWGAGTTVTVSS

Nucleic acid sequence of heavy chain variable region 
GAGGTGAAGCTGGTGGAGTCTGGGGGAGGCTTAGTGCAGCCTGGAGGGTCCCTGAAACTCTCCTG
TGCAACCTCTGGATTCACTTTCAGTGACTATTACATGTATTGGGTTCGCCAGACTCCAGAGAAGAGGC
TGGAGTGGGTCGCATACATTAGTAATGTTGGTGGTAGCACCTATTATCCAGACACTGTAAAGGGCCGA
TTCACCATCTCCAGAGACAATGCCAAGAACACCCTGTACCTGCAAATGAGCCGTCTGAAGTCTGAGGA
CACAGCCATATATTACTGTGCAAGAGGGGATGGTTACTACGGATACTACTTCGATGTCTGGGGCGCAG
GGACCACGGTCACCGTCTCCTCA
 Amino acid sequence of light chain variable region

<-----------FR1---------> CDR1 <-----FR2-----> CDR2 <-----------
---
QIVLTQSPAIMSASPGEKVTMTCSASSSVSYMHWYQQKSGTSPKRWIYDTSKLASGVPARFSGSG
SGTSYS
FR3------------> CDR3 <---FR4-->
LTISSMEAEDAATYYCQQWSSNPYTFGGGTKLEIK

Nucleic acid sequence of light chain variable region

CAAATTGTTCTCACCCAGTCTCCAGCAATCATGTCTGCATCTCCAGGGGAGAAGGTCACCATGAC
CTGCAGTGCCAGCTCAAGTGTAAGTTACATGCACTGGTACCAGCAGAAGTCAGGCACCTCCCCCAAAA
GATGGATTTATGACACATCCAAACTGGCTTCTGGAGTCCCTGCTCGCTTCAGTGGCAGTGGGTCTGGG
ACCTCTTACTCTCTCACAATCAGCAGCATGGAGGCTGAAGATGCTGCCACTTATTACTGCCAGCAGTG
GAGTAGTAACCCGTACACGTTCGGAGGGGGGACCAAGCTGGAAATCAAA

### Clone 2H2:

Amino acid sequence of heavy chain variable region

<-----------FR1----------> CDR1 <----FR2-----> CDR2 <-
---
EVKLVESGGGLVQPGGSLKLSCATSGFTFSDYYMYWVRQTPEKRLEWVAYISNVGGSTYYPDTVK
GRFTIS
----------FR3--------------> CDR3 <---FR4--->
RDNAKNTLYLQMSRLKSEDTAMYYCARGDGYYGYYFDVWGAGTTVTVSS

Nucleic acid sequence of heavy chain variable region

GAAGTGAAGCTGGTGGAATCTGGGGGAGGCTTAGTGCAGCCTGGAGGGTCCCTGAAACTCTCCTG
TGCAACCTCTGGATTCACTTTCAGTGACTATTACATGTATTGGGTTCGCCAGACTCCAGAGAAGAGGC

TGGAGTGGGTCGCATATATTAGTAATGTTGGTGGTAGCACCTATTATCCAGACACTGTAAAGGGCCGA
TTCACCATCTCCAGAGACAATGCCAAGAACACCCTGTACCTGCAAATGAGCCGTCTGAAGTCTGAGGA
CACAGCCATGTATTACTGTGCAAGAGGGGATGGTTACTACGGATACTACTTCGATGTCTGGGGCGCAG
GGACCACGGTCACCGTCTCCTCA

Amino acid sequence of light chain variable region

<-----------FR1--------->   CDR1   <-----FR2-----> CDR2   <-----------
---
ENVLTQSPAIMSASPGEKVTMTCSASSSVSYMHWYQQKSGTSPKRWIYDTSKLASGVPTRFSGSG
SGTSFS
FR3------------->   CDR3  <---FR4-->
LTISSMEAEDAATYYCQQWSSNPYTFGGGTKLEIK

Nucleic acid sequence of light chain variable region

GAAAATGTGCTCACCCAGTCTCCAGCAATCATGTCTGCATCTCCAGGGGAGAAGGTCACCATGAC
CTGCAGTGCCAGCTCAAGTGTAAGTTACATGCACTGGTACCAGCAGAAGTCTGGCACCTCCCCCAAAA
GATGGATTTATGACACATCCAAACTGGCTTCTGGAGTCCCTACTCGCTTCAGTGGCAGTGGGTCTGGG
ACCTCTTTCTCTCTCACAATCAGCAGCATGGAGGCTGAAGATGCTGCCACTTATTACTGCCAGCAGTG
GAGTAGTAACCCGTACACGTTCGGAGGGGGGACCAAGCTGGAAATAAAA

### Clone 6A8:

Amino acid sequence of heavy chain variable region

<-------------FR1-----------> CDR1 <----FR2-----> CDR2 <----
QVQFQQSGAELARPGTSVKLSCKASGYIFTSYWIQWIKQRPGQGLEWIGAIYPGDGDTRYTQKFKGKATLT
----------FR3-------------->CDR3<---FR4--->
AEKSSSTAYMQLRSLASEDSAVYYCAGFASWGQGTLLTVSA

Nucleic acid sequence of heavy chain variable region 
CAGGTTCAGTTCCAGCAGTCAGGGGCTGAGCTGGCAAGACCTGGGACTTCAGTGAAGTTGTCCTG
CAAGGCTTCTGGCTACATTTTACTAGTTACTGGATACAGTGGATAAAACAGAGACCTGGACAGGGTC
TGGAATGGATTGGGGCTATTTATCCTGGAGATGGTGATACTAGATATACTCAGAAGTTTAAGGGCAAG
GCCACATTGACTGCAGAAAAATCCTCCAGTACAGCCTACATGCAACTCAGAAGCTTGGCGTCTGAGGA
CTCTGCGGTCTATTACTGTGCAGGTTTTGCTTCCTGGGGCCAAGGGACTCTGcTCACTGTCTCTGCA
 Amino acid sequence of light chain variable region

    <----------FR1-------->   CDR1    <-----FR2-----> CDR2  <----------
---
    DIQMTQSPSSLSASLGERVNLTCRASQDIGDSLIWLQQEPDGTIKRLIYATSSLDSGVPKRFSGS
RSGSDY
    FR3------------->   CDR3  <---FR4-->
    SLTISSLESEDLVDYYCLQYASSPFTFGGGTKLEIK

Nucleic acid sequence of light chain variable region

GACATCCAGATGACCCAGTCTCCATCCTCCTTATCTGCCTCTCTGGGAGAAAGAGTCAATCTCAC
TTGTCGGGCAAGTCAGGACATTGGTGATAGTTTAATCTGGCTTCAGCAGGAACCAGATGGAACTATTA
AACGCCTGATCTACGCCACATCCAGTTTAGATTCTGGTGTCCCCAAAAGGTTCAGTGGCAGTAGGTCT
GGGTCAGATTATTCTCTCACCATCAGCAGCCTTGAGTCTGAAGATCTTGTAGACTATTACTGTCTACA
ATATGCTAGTTCTCCGTTCACGTTCGGAGGGGGGACCAAGCTGGAAATCAAA

### Clone 6G8:

Amino acid sequence of heavy chain variable region

<-----------FR1----------->    CDR1    <-----FR2------>    CDR2    <---
--
QVTLKESGPGILKPSQTLSLTCSFSGFSLNTSGMGVGWIRQPSGKGLEWLAHIWWDDDKYYNPSL
KSQLTI
--------FR3----------------->  CDR3   <---FR4--->
SKDTSRNQVFLRIASVDTADTATYYCARRERWAWFAYWGQGTLVTVSA

Nucleic acid sequence of heavy chain variable region

CAAGTTACTCTAAAAGAGTCTGGCCCTGGGATATTGAAGCCCTCACAGACCCTCAGTCTGACTTG
TTCTTTCTCTGGGTTTTCACTGAACACTTCTGGTATGGGTGTAGGCTGGATTCGTCAGCCTTCAGGGA
AGGGTCTGGAGTGGCTGGCGCACATTTGGTGGGATGATGATAAATACTATAATCCATCCCTGAAGAGC
CAGCTCACAATCTCCAAGGATACCTCCAGAAACCAGGTATTCCTCAGGATCGCCAGTGTGGACACTGC

AGATACTGCCACTTACTACTGTGCTCGAAGAGAACGGTGGGCCTGGTTTGCTTACTGGGGCCAAGGGA
CTCTGGTCACTGTCTCTGCA

Amino acid sequence of light chain variable region

    <----------FR1--------->   CDR1    <-----FR2-----> CDR2  <----------
----
    DIVMTQSQKFMSASVGDRVSVTCKASQNVFTNVAWYQQKPGQSPKVLIYSASYRYSGVPDRFTGS
GSGTDF
    FR3-------------->  CDR3  <---FR4-->
    TLTINNVQSEDLAEYFCQQYNSYPTFGSGTKLEIK

Nucleic acid sequence of light chain variable region

GACATTGTGATGACCCAGTCTCAAAAATTCATGTCCGCATCTGTAGGAGACAGGGTCAGCGTCAC
CTGCAAGGCCAGTCAGAATGTGTTTACTAATGTAGCCTGGTATCAGCAGAAACCAGGGCAATCTCCTA
AAGTACTGATTTACTCGGCATCCTACCGGTACAGTGGAGTCCCTGATCGCTTCACAGGCTcTGGCTCT
GGGACAGATTTCACTCTCACCATCAACAATGTGCAGTCTGAAGACTTGGCAGAGTATTTCTGTCAGCA
ATATAACAGCTATCCCACGTTCGGCTCGGGGACAAAGTTGGAAATAAAA

### Clone 7E10:

Amino acid sequence of heavy chain variable region

    <----------FR1---------->   CDR1   <-----FR2---->          CDR2        <--
--
    QVQFQQSGAELVRPGASVKLSCKASGYIFSSYWIQWVKQRPGQGLEWIGAIYPGDGDTRYTQKFK
DKATLT
    --------FR3-------------> CDR3<---FR4--->
    ADKSSSTAYMQLSSLASEDSAVYYCAGFASWGQGTLVTVSA

Nucleic acid sequence of heavy chain variable region

CAGGTTCAGTTCCAGCAGTCTGGGGCTGAGCTGGTAAGACCTGGGGCTTCAGTGAAGTTGTCCTG
CAAGGCTTCTGGCTACATCTTTAGTAGCTACTGGATACAGTGGGTAAAACAGAGGCCTGGACAGGGTC
TGGAATGGATTGGGGCTATTTATCCTGGAGATGGTGATACTAGATACACTCAGAAGTTCAAGGACAAG
GCCACATTGACTGCAGATAAATCCTCCAGCACAGCCTACATGCAACTCAGCAGCTTGGCATCTGAGGA
CTCTGCGGTCTATTACTGTGCAGGTTTTGCTTCCTGGGGCCAAGGGACTCTGGTCACTGTCTCTGCA

Amino acid sequence of light chain variable region

    <----------FR1-------->   CDR1    <-----FR2----->  CDR2  <----------
---
    DIQMTQSPSSLSASLGERVSLTCRASQDIGSSLNWLQQEPNGTIKRLIYATSSLDSGVPKRFSGS
RSGSDY
    FR3------------->   CDR3  <---FR4-->
    SLTISSLESEDFADYYCLQYASSPYTFGGGTKLEIK

Nucleic acid sequence of light chain variable region

GACATCCAGATGACCCAGTCTCCATCCTCCTTATCTGCCTCTCTGGGAGAAAGAGTCAGTCTCAC
TTGTCGGGCAAGTCAGGACATTGGTAGTAGCTTAAACTGGCTTCAGCAGGAACCAAATGGAACTATTA
AACGCCTGATCTACGCCACATCCAGTTTAGATTCTGGTGTCCCCAAAAGGTTCAGTGGCAGTAGGTCT
GGGTCAGATTATTCTCTAACCATCAGCAGCCTTGAGTCTGAAGATTTTGCAGACTATTATTGTCTACA
ATATGCTAGTTCTCCGTACACGTTCGGAGGGGGGACCAAGCTGGAAATCAAA

### Clone 8A8:

Amino acid sequence of heavy chain variable region

<----------FR1---------->   CDR1   <-----FR2---->          CDR2        <--
--
QVQFQQSGAELARPGASVKLSCKASGYIFTSYWIQWIKQRPGQGLEWIGAIYPGDGDTRYTQKFK
GKATLT
--------FR3------------> CDR3<---FR4--->
ADKSSSTAYMQLRSLASEDSAVYYCAGFASWGQGTLLTVSA

Nucleic acid sequence of heavy chain variable region

CAGGTTCAGTTCCAGCAGTCTGGGGCTGAGCTGGCAAGACCTGGGGCTTCAGTGAAGTTGTCCTG
CAAGGCTTCTGGCTACATTTTTACTAGTTACTGGATACAGTGGATAAAACAGAGACCTGGACAGGGTC
TGGAATGGATTGGGGCTATTTATCCTGGAGATGGTGATACTAGATATACTCAGAAGTTTAAGGGCAAG
GCCACATTGACTGCAGATAAATCCTCCAGTACAGCCTACATGCAACTCAGAAGCTTGGCGTCTGAGGA
CTCTGCGGTCTATTACTGTGCAGGTTTTGCTTCCTGGGGCCAAGGGACTCTGcTCACTGTCTCTGCA

Amino acid sequence of light chain variable region

   <----------FR1-------->   CDR1    <-----FR2-----> CDR2  <----------
---
   DIQMTQSPSSLSASLGERVSLTCRASQDIGESLIWLQQEPDGTIKRLIYATSSLDSGVPKRFSGS
RSGSDY
   FR3-------------->   CDR3  <---FR4-->
   SLSISSLESDDFVDYYCLQYSSSPYTFGGGTKLEIK

Nucleic acid sequence of light chain variable region

GACATCCAGATGACCCAGTCTCCATCCTCCTTATCTGCCTCTCTGGGAGAAAGAGTCAGTCTCAC
TTGTCGGGCAAGTCAGGACATTGGTGAGAGCTTAATCTGGCTTCAGCAGGAACCAGATGGAACTATTA
AACGCCTGATCTACGCCACATCCAGTTTAGATTCTGGTGTCCCCAAAAGGTTCAGTGGCAGTAGGTCT
GGGTCAGATTATTCTCTCTCCATCAGCAGCCTTGAGTCTGACGATTTTGTAGACTATTACTGTCTACA
ATATTCTAGTTCTCCGTACACGTTCGGAGGGGGGACCAAGCTGGAAATCAAA

### Clone 10D9:

Amino acid sequence of heavy chain variable region

Nucleic acid sequence of heavy chain variable region

CAGGTCCAACTGCAGCAGCCTGGACTGAACTGGTGAAGCCCGGGGCTTCAGTGAAGTTGTCCTG
CAAGGCTTCTGGCTACACCTTCACCAGCTACTATCTGTACTGGGTGAAGCAGAGGCCTGGACAAGGCC
TTGAGTGGATTGGGGGGATTAATCCTAGCAATGGTGGTTCTAACTTCAATGAGAAGTTCAAGACCAAG
GCCACACTGACTGTAGATAAAACCTCCAACACAGCCTACATGCAACTCAGCAGCCTGACATCTGAGGA
CTCTGCGGTCTATTTTTGTACAAGAAGGGGTAGTAGTTGGAACTTCGATGTCTGGGGCGCAGGGACCA
CGGTCACCGTCTCCTCA

Amino acid sequence of light chain variable region

<----------FR1--------> CDR1 <-----FR2-----> CDR2 <-----------
----
DIKMTQSPSSMYASLGERVTIGCKASQDINSYLSWFQQKPGKSPKTLIYRTNRLVDGVPSRFSGS
GSGQDY
FR3-------------> CDR3 <---FR4-->
SLTISSLEYEDVGIYYCLQYDEFPLTFGAGTKLELK

Nucleic acid sequence of light chain variable region

GACATCAAGATGACCCAGTCTCCATCTTCCATGTATGCATCTCTAGGAGAGAGAGTCACTATCGG
TTGCAAGGCGAGTCAGGACATTAATAGCTATTTAAGCTGGTTCCAACAGAAACCAGGGAAATCTCCTA
AGACCCTGATCTATCGTACAAACAGATTGGTAGATGGGGTCCCATCAAGGTTCAGTGGCAGTGGATCT
GGGCAAGATTATTCTCTCACCATCAGCAGCCTGGAATATGAAGATGTGGGAATTTATTATTGTCTACA
GTATGATGAGTTTCCGCTCACGTTCGGTGCTGGGACCAAGCTGGAGCTGAAA

### Clone 14E4:

Amino acid sequence of heavy chain variable region

<-----------FR1----------->   CDR1   <-----FR2---->          CDR2        <----
QVQLQQSGPELVKPGASVKISCKASGYIFTNYYIHWVKQRPGQGLEWIGWIFPGSGNTKYSEKFKDKATLT
--------FR3-------------> CDR3<---FR4--->
ADTSSSTAYMQLSSLTSEDSAVYFCAGLSDWGQGTTLTVSS

Nucleic acid sequence of heavy chain variable region

CAGGTCCAGCTGCAGCAGTCTGGACCTGAGCTGGTGAAGCCTGGGGCTTCAGTGAAGATATCCTG
CAAGGCTTCTGGCTACATCTTCACAAACTACTATATACACTGGGTGAAGCAGAGGCCTGGACAGGGAC
TTGAGTGGATTGGATGGATTTTTCCTGGAAGTGGTAATACTAAGTACAGTGAGAAGTTCAAGGACAAG
GCCACACTGACGGCAGACACATCCTCCAGCACAGCCTACATGCAGCTCAGCAGCCTGACATCTGAGGA
CTCTGCAGTCTATTTCTGTGCTGGCCTGTCCGACTGGGGCCAAGGCACCACTCTCACAGTCTCCTCA

Amino acid sequence of light chain variable region

<-----------FR1--------> CDR1 <-----FR2-----> CDR2 <-----------
----
DIQMTQSPSSLSASLGERVSLTCRASQDIGDRLIWLQQEPAGTFKRLIYATSSLDSGVPKRFSGS
RSGSDY
FR3-------------> CDR3 <---FR4-->
SLTISSLESEDFVDYYCLQYASSPWTFGGGTKLEIK

Nucleic acid sequence of light chain variable region

GACATCCAGATGACCCAGTCTCCTTCCTCCTTATCTGCCTCTCTGGGAGAAAGAGTCAGTCTCAC
TTGTCGGGCAAGTCAGGACATTGGTGATAGATTAATCTGGCTTCAGCAGGAACCAGCTGGAACTTTTA
AACGCCTGATCTACGCCACATCCAGTTTAGATTCTGGTGTCCCCAAAAGGTTCAGTGGCAGTAGGTCT
GGGTCAGATTATTCTCTCACCATCAGCAGCCTTGAGTCTGAAGACTTTGTAGACTATTACTGTCTACA
GTATGCTAGTTCTCCGTGGACGTTCGGTGGAGGCACCAAGCTGGAAATCAAA

### Clone 14H10:

Amino acid sequence of heavy chain variable region

<----------FR1---------->   CDR1   <-----FR2---->          CDR2        <-
---
QIQLQQSGAELARPGASVNLSCKASGYSFTSYWMQWLKQRPGQGLEWIGAIYPGDGDTRYTQKFK
GKATLT
--------FR3-------------> CDR3<---FR4--->
ADKSSSTAYMQLSSLASEDSAVYYCALFSYWGQGTLVTVSA

Nucleic acid sequence of heavy chain variable region

CAGATTCAGCTCCAGCAGTCTGGGGCTGAACTGGCAAGACCTGGGGCTTCAGTGAATTTGTCCTG
CAAGGCTTCTGGCTACAGCTTTACTAGCTACTGGATGCAGTGGCTAAAACAGAGGCCTGGACAGGGTC
TGGAATGGATTGGGGCTATTTATCCTGGAGATGGTGATACTAGGTACACTCAAAAGTTCAAGGGCAAG
GCCACATTGACTGCAGATAAATCCTCCAGCACAGCCTACATGCAACTCAGCAGCTTGGCATCTGAGGA
CTCTGCGGTCTATTACTGTGCACTCTTCTCTTACTGGGGCCAAGGGACTCTGGTCACTGTCTCTGCA

Amino acid sequence of light chain variable region

<-----------FR1--------> CDR1 <-----FR2-----> CDR2 <-----------
----
DIQMTQSPSSLSASLGERVSLTCRASQDIGSSLNWLQQEPDGTFKRLIYATSTLDSGVPKRFSGS
RSGSDY
FR3-------------> CDR3 <---FR4-->
SLTISSLESEDFVDYYCLQYASFPYTFGGGTKLEIK

Nucleic acid sequence of light chain variable region

GACATCCAGATGACCCAGTCTCCATCCTCCTTATCTGCCTCTCTGGGAGAAAGAGTCAGTCTCAC
TTGTCGGGCAAGTCAGGACATTGGTAGTAGTTTAAACTGGCTTCAGCAGGAACCAGATGGAACTTTTA
AACGCCTGATCTACGCCACATCCACTTTAGATTCTGGTGTCCCCAAAAGGTTCAGTGGCAGTAGGTCT
GGGTCAGATTATTCTCTCACCATCAGCAGCCTTGAGTCTGAAGATTTTGTAGACTATTACTGTCTACA
ATATGCTAGTTTTCCGTACACGTTCGGAGGGGGGACCAAGCTGGAAATCAAA

### Clone 22C12:

Amino acid sequence of heavy chain variable region

<-----------FR1----------->   CDR1   <-----FR2---->          CDR2        <-
---
QVQFQQSGAELARPGASVKLSCKASGYTFTGYWIQWVKQRPGQGLEWIGAIYPGDDDTRYTQKFK
GRATLT
----------FR3---------------->   CDR3   <---FR4--->
ADTSSSTAYMQLSSLASEDSAVYYCARGNWDDWDFDVWGAGTTVTVSS

Nucleic acid sequence of heavy chain variable region

CAGGTTCAGTTCCAGCAGTCTGGGGCTGAGCTGGCAAGACCTGGGGCTTCAGTGAAGTTGTCCTG
CAAGGCTTCTGGCTACACCTTTACTGGCTACTGGATACAGTGGGTAAAACAGAGGCCTGGACAGGGTC
TGGAATGGATTGGGGCTATTTATCCTGGAGATGATGATACTAGGTACACTCAGAAGTTTAAGGGCAGG
GCCACATTGACTGCAGATACATCCTCCAGCACAGCCTACATGCAACTCAGCAGCTTGGCATCTGAGGA
CTCTGCGGTCTATTACTGTGCAAGAGGGAACTGGGACGACTGGGACTTCGATGTCTGGGGCGCAGGGA
CCACGGTCACCGTCTCCTCA

Amino acid sequence of light chain variable region



DIQMTQTTSSLSASLGDRVTISCRASQDISNYLNWYQQKPDGTVKLLIHYTSRLHSGVPSRFSGS
GSGTDY
FR3-------------> CDR3 <---FR4-->
SLTISNLEQEDIATYFCQQGDTLPWTFGGGTKLEIK

Nucleic acid sequence of light chain variable region

GATATCCAGATGACACAGACTACATCCTCCCTGTCTGCCTCTCTGGGAGACAGAGTCACCATCAG
TTGCAGGGCAAGTCAGGACATTAGCAATTATTTAAACTGGTATCAGCAGAAACCAGATGGAACTGTTA
AACTCCTGATCCATTACACATCAAGATTACACTCAGGAGTCCCATCAAGGTTCAGTGGCAGTGGGTCT
GGAACAGACTATTCTCTCACCATTAGCAACCTGGAACAAGAAGATATTGCCACTTACTTTTGCCAACA
GGGTGATACGCTTCCGTGGACGTTCGGTGGAGGCACCAAGCTGGAAATCAAA

### Clone 30A11:

Amino acid sequence of heavy chain variable region 
<-----------FR1----------->   CDR1   <-----FR2---->          CDR2        <----
QVHLQQSGAELARPGASVKLSCKASGYSFISYWMEWVKQRPGQGLEWIGAIYPGDGDTRYTQEFKGKATLT
---------FR3--------------->   CDR3   <---FR4--->
ADESSSTAYMQLSSLASEDSAVYYCARGNWDDWDFDVWGAGTTVTVSS
 Nucleic acid sequence of heavy chain variable region

CAGGTTCACCTCCAGCAGTCTGGGGCTGAGCTGGCAAGACCTGGGGCTTCAGTGAAGTTGTCCTG
CAAGGCTTCTGGCTACTCCTTTATTAGTTACTGGATGGAGTGGGTAAAACAGAGGCCTGGACAGGGTC
TGGAATGGATTGGGGCTATTTATCCTGGAGATGGTGATACTAGGTACACTCAGGAGTTCAAGGGCAAG
GCCACATTGACTGCAGATGAATCCTCCAGCACAGCCTACATGCAACTCAGCAGCTTGGCATCTGAGGA
CTCTGCGGTCTATTACTGTGCAAGAGGGAACTGGGACGACTGGGACTTCGATGTCTGGGGCGCAGGGA
CCACGGTCACCGTCTCCTCA

Amino acid sequence of light chain variable region

<----------FR1--------> CDR1 <-----FR2-----> CDR2 <-----------
----
DIQMTQTTSSLSASLGDRVTISCRASQDISNYLNWYQQKPDGTVKLLIHYTSRLHSGVPSRFSGS
GSGTDY
FR3-------------> CDR3 <---FR4-->
SLTISNLEQEDIATYFCQQGNTLPWTFGGGTKLEIK

Nucleic acid sequence of light chain variable region

GATATCCAGATGACACAGACTACATCCTCCCTGTCTGCCTCTCTGGGAGACAGAGTCACCATCAG
TTGCAGGGCAAGTCAGGACATTAGCAATTATTTAAACTGGTATCAGCAGAAACCAGATGGAACTGTTA
AACTCCTGATCCATTACACATCAAGATTACACTCAGGAGTCCCATCAAGGTTCAGTGGCAGTGGGTCT
GGAACAGACTATTCTCTCACCATTAGCAACCTGGAACAAGAAGATATTGCCACTTACTTTTGCCAACA
GGGTGATACGCTTCCGTGGACGTTCGGTGGAGGCACCAAGCTGGAAATCAAA

### Clone 36A10:

Amino acid sequence of heavy chain variable region

<-----------FR1----------->   CDR1   <-----FR2---->          CDR2        <----
QVQLQQPGAEVVKPGASVRLSCKPSGYFFTSYWMHWVKQRPGQGLEWIGEINPSNGRTNYNENFKSKATLT
---------FR3--------------->   CDR3   <---FR4--->
VDTSSSTAYLQLSSLTSEDSAVYYCARRNYGYPDYWGQGTTLTVSS

Nucleic acid sequence of heavy chain variable region

CAGGTCCAACTGCAGCAGCCTGGGGCTGAAGTGGTGAAGCCTGGGGCTTCAGTGAGACTGTCCTG
TAAGCCTTCTGGCTACTTCTTCACCAGCTACTGGATGCACTGGGTGAAACAGAGGCCTGGACAAGGCC
TTGAGTGGATTGGAGAGATTAATCCTAGTAACGGTCGTACTAACTACAATGAGAACTTCAAGAGCAAG
GCCACACTGACTGTAGACACATCTTCCAGCACAGCCTACCTGCAACTCAGCAGCCTGACATCTGAGGA
CTCTGCGGTCTATTACTGTGCAAGAAGAAACTACGGCTACCCTGACTACTGGGGCCAAGGCACCACTC
TCACAGTCTCCTCA

Amino acid sequence of light chain variable region

    <----------FR1-------->   CDR1    <-----FR2-----> CDR2  <----
----

DIKMTQSPSSMYASLGERVTFTCKASQDINTYLNWFQQKPGKSPKTLIYRANRLLDGVPSRFSGS
GSGQDY
FR3-------------> CDR3 <---FR4-->
SLTISSLEYEDMGIYYCLQYDEIPYTFGGGTKLEIK

Nucleic acid sequence of light chain variable region

GACATCAAGATGACCCAGTCTCCATCTTCCATGTATGCATCTCTAGGAGAGAGAGTCACTTTCAC
TTGCAAGGCGAGTCAGGACATTAATACCTATTTAAACTGGTTCCAGCAGAAACCAGGGAAATCTCCTA
AGACCCTGATATACCGTGCAAACAGATTGTTAGATGGGGTCCCATCAAGGTTCAGTGGCAGTGGATCT
GGGCAAGATTATTCTCTCACCATCAGCAGCCTGGAGTATGAAGACATGGGAATTTATTATTGTCTACA
GTATGATGAGATTCCGTACACGTTCGGAGGGGGGACCAAGCTGGAAATCAAA

The above heavy chain and light chain variable region sequence fragments were amplified by PCR, the heavy chain variable region was cloned into a vector containing human heavy chain constant region and regulatory elements, to express intact IgG heavy chain in mammalian cells. Similarly, the light chain variable region was cloned into a vector containing a human light chain constant region and regulatory elements, to express intact light chain kappa in mammalian cells. After correct sequencing, it was transfected into HEK293-6E mammalian cells, IgG was expressed and secreted into the medium, and the supernatants were combined and collected, purified after being filtered. The IgG was purified by Protein A chromatography, the culture supernatant was loaded on a Protein A column with suitable size, washed with 50mM Tris-HCl pH8.0, 250mM NaCl, and the bound IgG was eluted with 0.1M Glycine-HCl (pH3.0). The protein was ultrafiltered and concentrated by using a concentration tube (Millipore), OD280 was detected, and the concentration of IgG was determined by spectrophotometry.

**Table 1: Affinity of CD73 antibody and inhibition of CD73 hydrolase activity**

| **CD73 antibod y** | **ELISA EC50(nM)** | | **FACS EC50(nM)** | | **Inhibition of CD73 enzyme activity IC50(nM)** | |
|---|---|---|---|---|---|---|
| | **hCD73** | **cynoCD7 3** | **Calu6** | **CHO-CD73-1C11** | **Calu6** | **Soluble CD73** |
| 2D4 | 0.02 | 0.02 | 1.25 | 1.76 | 0.47 | \ |
| 6A8 | 0.08 | 0.09 | 3.51 | 11.0 | 6.63 | \ |
| 6G8 | 0.02 | 0.02 | 1.03 | 1.91 | 1.72 | 1.03 |
| 7E10 | 0.07 | 0.11 | 2.52 | 8.59 | 7.66 | \ |
| 8A8 | 0.02 | 0.02 | 0.61 | 1.47 | 4.89 | \ |
| 14H10 | 0.03 | 0.07 | 2.28 | 2.62 | 1.71 | \ |
| 22C12 | 0.04 | 0.05 | 1.20 | 1.43 | 0.97 | \ |
| 30A11 | 0.02 | 0.03 | 1.50 | 2.27 | 1.18 | \ |
| 2H2 | 0.02 | 0.02 | 0.93 | 1.53 | 4.03 | \ |
| 14E4 | 0.06 | 0.18 | 2.56 | 6.55 | 4.57 | \ |

### 8. CD73 antibody-mediated endocytosis of CD73 cells

Calu6 cells were inoculated in a 24-well plate at 1x10⁵ cells/well, placed at 37°C, under 5% CO₂ in incubator overnight. The next day, the complete medium containing the antibody to be detected was added, incubated for 0,30 minutes, 60 minutes, 120 minutes, 240 minutes, and 480 minutes respectively, then the supernatant was pipetted, washed twice with pre-chilled PBS, then the cells were digested with trypsin and collected. The CD73 abundance on the cell surface was detected by flow cytometry: first, the collected cells were blocked with 3% BSA/PBS for 30 minutes, and a 1:300 diluted secondary antibody solution (anti-human IgGFc-AF647, Jackson ImmunoResearch, Cat No. 109-606-170) was added, incubated on ice for 45 minutes, washed twice to wash off the excess secondary antibody, and finally 100µL PBS solution containing 5 µg/mL PI was used to resuspend the cells and detected on flow cytometry. The results were shown in Table 2.

**Table 2: CD73 antibody mediated endocytosis of CD73 cells**

| | CD73 endocytosis ratio% | | | | |
|---|---|---|---|---|---|
| CD73 antibody | 30 minutes | 60 minutes | 120 minutes | 240 minutes | 480 minutes |
| 2D4 | 19.1 | 28.1 | 29.7 | 39.5 | 36.9 |
| 2H2 | 22.4 | 27.4 | 45.8 | 33.4 | 49.3 |
| 6A8 | - | 16.2 | - | - | - |
| 6G8 | 17.1 | 38.5 | 22.2 | 35.3 | 47.7 |
| 7E10 | 9.9 | 25.1 | - | 6.6 | 6.2 |
| 8A8 | 21.2 | 25.6 | 27.3 | 44.2 | 40.5 |
| 14E4 | - | 19.3 | 2.6 | 12.7 | 2.6 |
| 14H10 | - | 33.1 | - | - | - |
| 22C12 | - | 6.1 | 11.1 | - | 13.2 |
| 30A11 | 3.0 | 11.7 | 13.7 | 12.0 | 25.9 |

### 9. Humanization of anti-CD73 antibody

The selected mouse monoclonal antibody variable region sequence was aligned with the human antibody sequence, to find a suitable human germline gene sequence with high homology for CDR transplantation; for example, the mAb22C12 mouse antibody light chain was mouse kappa IGKV10-96#01, the human Vk1-39 with the highest homology to its framework region was selected for CDR transplantation, and the mouse antibody heavy chain was IGHV1-50#01, the human germline gene IGHV1-2 was selected for CDR transplantation; the mAb6G8 mouse antibody light chain was mouse kappa IGKV6-15#01, the human IGKV_1_33 or IGKV6-15 with high homology to its framework region was selected for CDR transplantation, the mouse antibody heavy chain was IGHV8S9#01, the human germline gene IGHV2-70 was selected for CDR transplantation. Subsequently the homology modeling was performed by computer at the same time, the CDR region and its surrounding framework amino acid sequence were analyzed, to avoid the concentrated distribution of charge or hydrophobic regions on the molecular surface caused by the selected human germline genes; at the same time, by calculating the electrostatic force, van der Waals force, and hydrophilicity and entropy values, the key amino acid individuals that may interact with CD73 and maintain the spatial framework in each gene sequence of positive monoclonal antibody were analyzed, and the reverse mutation sites were designed on this basis.

### hu22C12VHv9:

Amino acid sequence of heavy chain variable region

    <----------FR1---------->  CDR1  <-----FR2---->        CDR2       <-
---
    QVQLVQSGAEVKKPGASVKVSCKASGYTFTGYWIQWVRQAPGQGLEWMGAIYPGDSDTRYTQKFQ
GRVTMT
    ---------FR3--------------->  CDR3  <---FR4--->
    ADTSISTAYMELSRLRSDDTAVYYCARGNWDDWDFDVWGQGTTVTVSS

Nucleic acid sequence of heavy chain variable region

CAGGTTCAGCTGGTTCAGTCTGGCGCCGAAGTGAAGAAACCTGGCGCCTCTGTGAAGGTGTCCTG
CAAGGCTAGCGGCTACACATTCACCGGCTATTGGATCCAGTGGGTCCGACAGGCTCCTGGACAGGGAC
TTGAATGGATGGGCGCTATCTACCCTGGCGACtcCGACACCAGATACACCCagAAATTCCAGGGCAGA
GTGACCATGACCgccGACACCAGCATCAGCACCGCCTACATGGAACTGAGCAGACTGAGAAGCGACGA
CACAGCCGTGTACTACTGCGCCAGAGGCAACTGGGACGACTGGGACTTTGATGTGTGGGGCCAGGGCA
CCACCGTGACAGTTTCTTCT

### hu22C12 VHv10:

Amino acid sequence of heavy chain variable region

<----------FR1----------> CDR1 <-----FR2----> CDR2 <-
---
QVQLVQSGAEVKKPGASVKVSCKASGYTFTGYWIQWVRQAPGQGLEWMGAIYPGDEDTRYTQKFQ
GRVTMT
---------FR3---------------> CDR3 <---FR4--->
ADTSISTAYMELSRLRSDDTAVYYCARGNWDDWDFDVWGQGTTVTVSS

Nucleic acid sequence of heavy chain variable region

CAGGTTCAGCTGGTTCAGTCTGGCGCCGAAGTGAAGAAACCTGGCGCCTCTGTGAAGGTGTCCTG
CAAGGCTAGCGGCTACACATTCACCGGCTATTGGATCCAGTGGGTCCGACAGGCTCCTGGACAGGGAC
TTGAATGGATGGGCGCTATCTACCCTGGCGACGAaGACACCAGATACACCCAGAAATTCCAGGGCAGA
GTGACCATGACCgccGACACCAGCATCAGCACCGCCTACATGGAACTGAGCAGACTGAGAAGCGACGA

CACAGCCGTGTACTACTGCGCCAGAGGCAACTGGGACGACTGGGACTTTGATGTGTGGGGCCAGGGCA
CCACCGTGACAGTTTCTTCT

### hu6G8 VHv1:

Amino acid sequence of heavy chain variable region

<-----------FR1----------->    CDR1      <-----FR2---->          CDR2
<---
QVTLRESGPALVKPTQTLTLTCTFSGFSLSTSGMGVGWIRQPPGKALEWLAHIWWDDDKYYNPSL
KSRLTI
-----------FR3--------------->  CDR3   <---FR4--->
SKDTSKNQVVLTMTNMDPVDTATYYCARRERWAWFAYWGQGTLVTVSS

Nucleic acid sequence of heavy chain variable region

CAAGTGACCCTGAGAGAGTCTGGACCCGCTCTGGTCAAGCCCACACAGACCCTGACACTGACCTG
TACCTTCAGCGGCTTCAGCCTGAGCACATCTGGCATGGGAGTCGGCTGGATCAGACAGCCTCCTGGCA
AAGCTCTGGAATGGCTGGCCCACATTTGGTGGGACGACGACAAGTACTACAACCCCAGCCTGAAGTCC
AGACTGACCATCAGCAAGGACACCAGCAAGAACCAGGTGGTGCTGACCATGACCAACATGGACCCTGT
GGACACCGCCACCTACTACTGCGCTAGAAGAGAAAGATGGGCTTGGTTTGCCTACTGGGGCCAGGGAA
CACTGGTCACCGTTAGCTCT

### hu6G8 VHv2:

Amino acid sequence of heavy chain variable region

<-----------FR1-----------> CDR1 <-----FR2----> CDR2
<---
QVTLRESGPALVKPTQTLTLTCTFSGFSLSTSGMGVGWIRQPPGKALEWLAHIWWDDDKYYNPSL
KTRLTI
-----------FR3---------------> CDR3 <---FR4--->
SKDTSRNQVVLTMTNMDPVDTATYYCARRERWAWFAYWGQGTLVTVSS

Nucleic acid sequence of heavy chain variable region

CAAGTGACCCTGAGAGAGTCTGGACCCGCTCTGGTCAAGCCCACACAGACCCTGACACTGACCTG
TACCTTCAGCGGCTTCAGCCTGAGCACATCTGGCATGGGAGTCGGCTGGATCAGACAGCCTCCTGGCA
AAGCTCTGGAATGGCTGGCCCACATTTGGTGGGACGACGACAAGTACTACAACCCCAGCCTGAAGaCC
AGACTGACCATCAGCAAGGACACCAGCAGAAACCAGGTGGTGCTGACCATGACCAACATGGACCCTGT
GGACACCGCCACCTACTACTGCGCTAGAAGAGAAAGATGGGCTTGGTTTGCCTACTGGGGCCAGGGAA
CACTGGTCACCGTTAGCTCT

### hu 6G8 VHv8:

Amino acid sequence of heavy chain variable region

<-----------FR1-----------> CDR1 <-----FR2----> CDR2
<---
QVTLRESGPALVKPTQTLTLTCTFSGFSLSTSGMGVGWIRQPPGKALEWLAHIWWDEDKYYNPSL
KSRLTI
-----------FR3---------------> CDR3 <---FR4--->
SKDTSKNQVVLTMTNMDPVDTATYYCARRERWAWFAYWGQGTLVTVSS

Nucleic acid sequence of heavy chain variable region

CAAGTGACCCTGAGAGAGTCTGGACCCGCTCTGGTCAAGCCCACACAGACCCTGACACTGACCTG
TACCTTCAGCGGCTTCAGCCTGAGCACATCTGGCATGGGAGTCGGCTGGATCAGACAGCCTCCTGGCA
AAGCTCTGGAATGGCTGGCCCACATTTGGTGGGACGAgGACAAGTACTACAACCCCAGCCTGAAGTCC
AGACTGACCATCAGCAAGGACACCAGCAAGAACCAGGTGGTGCTGACCATGACCAACATGGACCCTGT
GGACACCGCCACCTACTACTGCGCTAGAAGAGAAAGATGGGCTTGGTTTGCCTACTGGGGCCAGGGAA
CACTGGTCACCGTTAGCTCT

### hu22C12 Vkv2:

Amino acid sequence of light chain variable region

<----------FR1--------> CDR1 <-----FR2-----> CDR2 <----------
----
DIQMTQSPSSLSASVGDRVTITCRASQDISNYLNWYQQKPGKAPKLLIHYTSRLHSGVPSRFSGS
GSGTDY

FR3-------------->   CDR3  <---FR4-->
TLTISSLQPEDFATYYCQQGDTLPWTFGQGTKVEIK

Nucleic acid sequence of light chain variable region

GACATCCAGATGACACAGAGCCCTAGCAGCCTGTCTGCCAGCGTGGGAGACAGAGTGACCATCAC
CTGTAGAGCCAGCCAGGACATCAGCAACTACCTGAACTGGTATCAGCAGAAGCCCGGCAAGGCCCCTA
AGCTGCTGATCCACTACACCAGCAGACTGCACAGCGGCGTGCCAAGCAGATTTTCTGGCAGCGGCTCT
GGCACCGACTaCACCCTGACAATCTCTAGCCTGCAGCCTGAGGACTTCGCCACCTACTATTGTCAGCA
GGGCGACACCCTGCCTTGGACATTTGGCCAGGGCACCAAGGTGGAAATCAAG

### hu22C12 Vkv3:

Amino acid sequence of light chain variable region

<-----------FR1--------> CDR1 <-----FR2-----> CDR2 <-----------
-----
DIQMTQSPSSLSASVGDRVTITCRASQDISNYLNWYQQKPGKAPKLLIHYTSRLHSGVPSRFSGS
GSGTDF
FR3-------------> CDR3 <---FR4-->
TLTISSLQPEDFATYYCQQGDTLPWTFGQGTKVEIK

Nucleic acid sequence of light chain variable region

GACATCCAGATGACACAGAGCCCTAGCAGCCTGTCTGCCAGCGTGGGAGACAGAGTGACCATCAC
CTGTAGAGCCAGCCAGGACATCAGCAACTACCTGAACTGGTATCAGCAGAAGCCCGGCAAGGCCCCTA
AGCTGCTGATCCACTACACCAGCAGACTGCACAGCGGCGTGCCAAGCAGATTTTCTGGCAGCGGCTCT
GGCACCGACTTCACCCTGACAATCTCTAGCCTGCAGCCTGAGGACTTCGCCACCTACTATTGTCAGCA
GGGCGACACCCTGCCTTGGACATTTGGCCAGGGCACCAAGGTGGAAATCAAG

### hu 6G8 Vkv1:

Amino acid sequence of light chain variable region

<-----------FR1--------> CDR1 <-----FR2-----> CDR2 <--------------
DIQMTQSPSSLSASVGDRVTITCRASQNVFTNVAWYQQKPGKAPKVLIYSASYRYSGVPSRFSGSGSGTDF
FR3-------------> CDR3 <---FR4-->
TLTISSLQPEDFATYFCQQYNSYPTFGQGTKLEIK

Nucleic acid sequence of light chain variable region

GACATCCAGATGACACAGAGCCCTAGCAGCCTGTCTGCCAGCGTGGGAGACAGAGTGACCATCAC
CTGTAGAGCCAGCCAGAACGTGTTCACCAACGTGGCCTGGTATCAGCAGAAGCCTGGCAAGGCTCCCA
AGGTGCTGATCTACAGCGCCAGCTACAGATACAGCGGCGTGCCCTCTAGATTCAGCGGCTCTGGCTCT
GGCACCGACTTCACCCTGACAATCTCTAGCCTGCAGCCTGAGGACTTCGCTACCTACTTCTGCCAGCA
GTACAACAGCTACCCCACCTTCGGCCAGGGCACCAAGCTGGAAATCAAG

### hu 6G8 Vkv2:

Amino acid sequence of light chain variable region

<-----------FR1--------> CDR1 <-----FR2-----> CDR2 <----------
-----
DIQMTQSPSSLSASVGDRVTITCQASQNVFTNVAWYQQKPGKAPKVLIYSASYRYSGVPSRFSGS
GSGTDF
FR3-------------> CDR3 <---FR4-->
TFTISSLQPEDIATYYCQQYNSYPTFGQGTKLEIK

Nucleic acid sequence of light chain variable region

GACATCCAGATGACACAGAGCCCTAGCAGCCTGTCTGCCAGCGTGGGAGACAGAGTGACCATCAC
CTGTcagGCCAGCCAGAACGTGTTCACCAACGTGGCCTGGTATCAGCAGAAGCCTGGCAAGGCTCCCA
AGGTGCTGATCTACAGCGCCAGCTACAGATACAGCGGCGTGCCCTCTAGATTCAGCGGCTCTGGCTCT
GGCACCGACTTCACCttcACAATCTCTAGCCTGCAGCCTGAGGACaTCGCTACCTACTaCTGCCAGCA
GTACAACAGCTACCCCACCTTCGGCCAGGGCACCAAGCTGGAAATCAAG

### hu 6G8 VkV4:

Amino acid sequence of light chain variable region

<-----------FR1--------> CDR1 <-----FR2-----> CDR2 <----------
-----
EIVMTQSPATLSVSPGERATLSCRASQNVFTNVAWYQQKPGQAPRVLIYSASYRYSGIPARFSGS
GSGTEF
FR3-------------> CDR3 <---FR4-->
TLTISSLQSEDFAVYFCQQYNSYPTFGQGTKLEIK

Nucleic acid sequence of light chain variable region

GAGATCGTGATGACACAGAGCCCCGCCACACTGAGTGTGTCTCCAGGCGAAAGAGCTACCCTGAG
CTGTAGAGCCAGCCAGAACGTGTTCACCAACGTGGCCTGGTATCAGCAGAAGCCTGGACAGGCTCCCA
GAGTGCTGATCTACAGCGCCAGCTACAGATACAGCGGCATCCCCGCTAGATTCAGCGGCTCTGGATCT
GGCACCGAGTTCACCCTGACAATCAGCAGCCTGCAGAGCGAGGACTTCGCCGTGTACTTCTGCCAGCA
GTACAACAGCTACCCCACCTTCGGCCAGGGCACCAAGCTGGAAATCAAG

The designed multiple light and heavy chain derivatives were subjected to DNA synthesis respectively (Suzhou Jinweizhi Biotechnology Co., Ltd.), and cloned into the eukaryotic expression vector containing the constant region of the antibody kappa chain or the constant region of human IgG1 CH1-CH3, the light and heavy chains of the plasmid were paired and then transfected into HEK293.6E cells, and expressed for 5-7 days, the culture supernatant was collected, after being purified by Protein A column, the concentration and purity were determined. The purified humanized antibody was assayed by ELISA and FACS for binding to CD73 positive cells, by Biacore for the affinity to CD73, and the inhibition of CD73 enzyme activity, etc., to confirm the affinity and biological activity of the humanized antibody.

### 10. Plasma surface resonance determination of the binding characteristics of humanized anti-CD73 antibody

The affinity of CD73 antibody was determined by plasma surface resonance on Biacore T200. At room temperature, the antibody concentration was diluted to 0.5 µg/mL with 1×HBS-EP buffer (prepared from HBS-EP+Buffer 10×, GE, BR1008-26), the Protein A chip (GE, 29-1275-56) was coated with a flow rate of 10µL per minute, the capture time was about 40 seconds, and the coated RU value was controlled to be about 100 RU. The recombinant human CD73 extracellular domain protein was diluted to 3µg/mL with 1×HBS-EP buffer, diluted to two-fold gradient, a total of 6 concentrations, 200µL of each concentration gradient. The 1×HBS-EP buffer was used as a control, the flow rate was 30 µl/min, the binding time was 120 seconds, and the dissociation time was 600 seconds. The affinity was fitted using Langmuir 1:1 Kinetic theoretical model and analyzed by Biacore T200 Evaluation Software (Table 3).

**Table 3. Affinities of Humanized antibody**

| **CD73 humanized antibody** | **ka (1/Ms)** | **kd (1/s)** | **KD (M)** |
|---|---|---|---|
| 6G8 Chimera | 6.17E+05 | 1.11E-04 | 1.80E-10 |
| 6G8 VH -vl/VK-vl | 3.89E+05 | 1.06E-04 | 2.72E-10 |
| 6G8 VH-v8/VK-v4 | 3.30E+05 | 8.48E-05 | 2.57E-10 |
| 22C12 VH-v9/Vk-v2 | 1.22E+06 | 1.65E-04 | 1.35E-10 |
| 22C12 VH-v10/VL-v2 | 1.68E+06 | 2.25E-04 | 1.34E-10 |
| 22C12 VH-v10/VL-v3 | 1.87E+06 | 1.79E-04 | 9.56E-11 |

### 11. Inhibition of humanized antibody on hydrolase activity of CD73

### Inhibition experiment of humanized antibody on hydrolase activity of CD73 on cell membrane surface

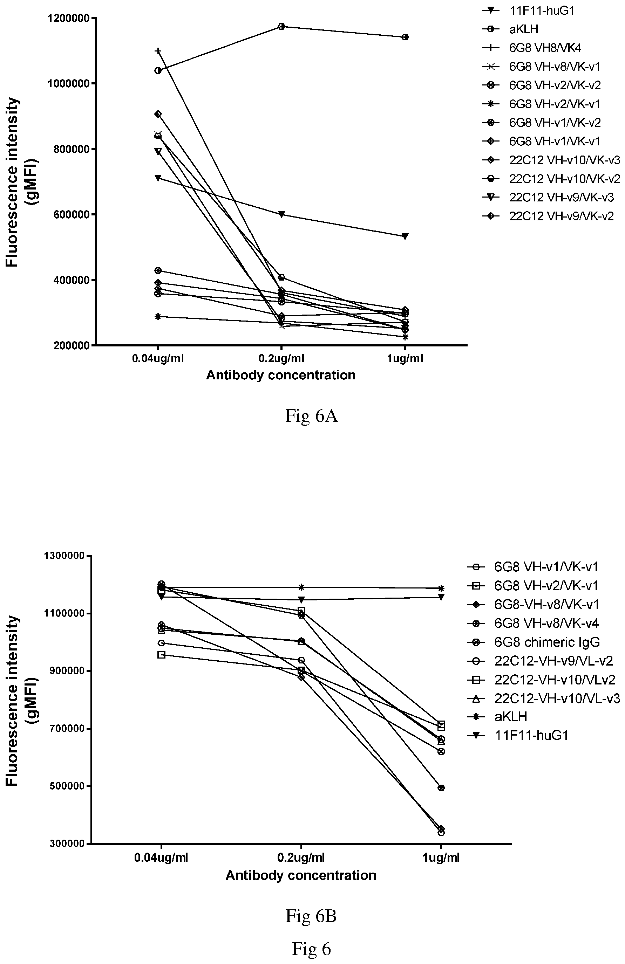

The human lung degenerative cancer cells Calu6 were inoculated in a 96-well U-shaped plate at 3x10⁴ cells/well, the purified humanized antibody with 25µg/mL as the initial concentration, was diluted with TM buffer (25mM Tris, pH7.5, 5mM MgC12) in a ratio of 1:5 to 3 concentrations in equal ratio. It was mixed with the cells and incubated in an incubator at 37°C for 20 minutes. After the incubation, the supernatant was discarded, it was washed twice with PBS, centrifuged at 1500 rpm for 5 minutes, the 100µL 180µM AMP (Sigma, Cat No. 01930-5G) solution was added to each well of experimental group, an equal amount of TM buffer was added to the control group, incubated at 37°C in an incubator for 60 minutes. After the incubation, centrifuged at 1500 rpm for 5 minutes, and 50µL supernatant was taken and mixed with an equal volume of 60 µM ATP (brand: Sigma, Cat NO. A6419-1G) solution in a 96-well black microtiter plate. After reacting at 37°C for 15 minutes, 100 µL of Celltiter Glo solution (Promega, Cat No. 7570) was added, placed on a well-plate shaker and shaken at 300 g for 2 minutes, kept in the dark for 10 minutes, the fluorescence signal was detected on a microplate reader (BioTek Synergy HT), the results showed that all the humanized antibodies of the present invention significantly inhibited CD73 activity on the cell membrane surface compared to the control antibody 11F11-huG1 (Figure. 6A).

### Inhibition experiment of humanized antibody on hydrolase activity of soluble CD73

The purified humanized antibody with 60 µg/mL as the initial concentration, was diluted with TM buffer (25 mM Tris, pH 7.5, 5 mM MgCl2) in a ratio of 1:5 to 3 concentrations in equal ratio, 75µL hCD73-cHis antigen was mixed with 25µL antibody of different concentrations in a 96-well plate, incubated at 37°C for 1 hour, then 100µLAMP/TM buffer was added to each well and incubated at 37°C for 1 hour. 50µL supernatant was pipetted and mixed with an equal volume of 60µM ATP/TM buffer in a 96-well black microtiter plate, reacted at 37°C for 15 minutes, 100µL Celltiter Glo solution was added, placed in a well plate shaker and shaken at 300g for 2 minutes, kept in dark for10 minutes. The fluorescence signal was detected on a microplate reader. The results showed that all humanized antibodies of the present invention could inhibit activity of soluble CD73, while the control antibody 11F11-huG1 could not (Figure 6B).

### 12. Humanized antibody mediated endocytosis of CD73 cells

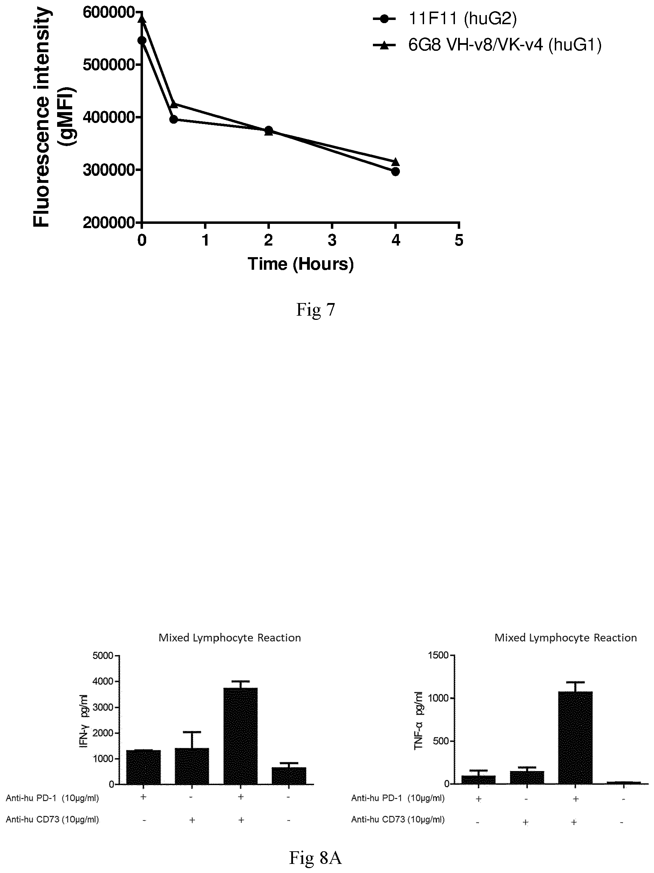

The Calu6 cells were inoculated into a 24-well plate according to 1x10⁵ cells/well, and placed in an incubator at 37°C under overnight. The next day, the complete medium containing 10 µg/mL tested antibodies was added, incubated at 4°C for 60 minutes, the supernatant was discard, washed twice with complete medium, and transferred to incubator under 5% CO₂ at 37°C, incubated for 0, 30 minutes, 120 minutes, and 240 minutes respectively, the supernatant was pipetted, washed twice with pre-chilled PBS, then the cells were digested with trypsin and collected. The CD73 abundance on the cell surface was detected by flow cytometry: first, the collected cells were blocked with 3% BSA/PBS for 30 minutes, and a 1:300 diluted secondary antibody solution (anti-human IgGFc-AF647, Jackson ImmunoResearch, Cat No. 109-606-170) was added, incubated on ice for 45 minutes, washed twice to wash off the excess secondary antibody, and finally 100µL PBS solution containing 5 µg/mL PI was used to resuspend the cells and detected on flow cytometry. The results showed that the anti-CD73 antibody of the present invention can effectively mediate the endocytosis of CD73 on the cell surface (see Table 4, Figure 7).

**Table 4: Humanized antibody-mediated endocytosis of CD73**

| | CD73 endocytosis ratio% | | | |
|---|---|---|---|---|
| CD73 antibody | 0 minute | 30 minutes | 120 minutes | 240 minutes |
| 6G8 VHv8-VKv4 | 0% | 28% | 37% | 46% |
| 11F11-huG1 | 0% | 28% | 31% | 46% |

### 13. PBMC Mixed Lymphocyte Reaction

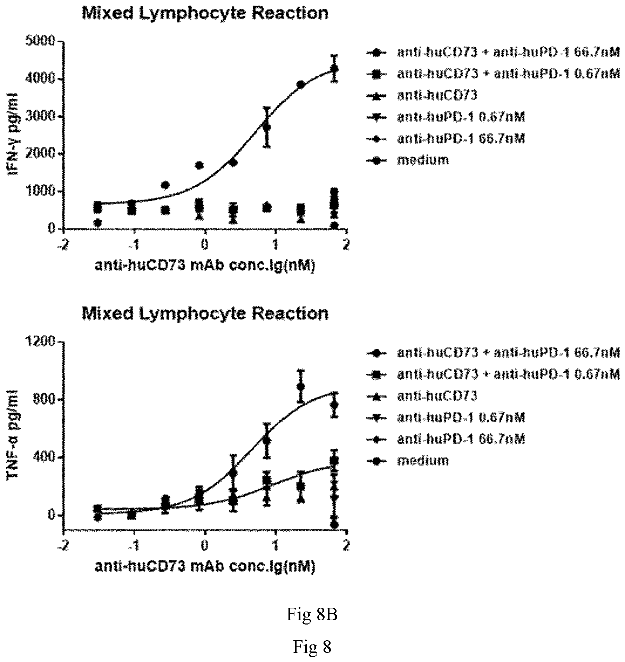

30 mL fresh blood of healthy volunteer was taken and added into 15 mL PBS, mixed well and reserved. 20mL Ficoll Paque Plus (GE Healthcare, Cat No. 17-1440-02) was taken and added into a 50mL centrifuge tube, then 30ml fresh blood diluted was taken and gently placed on the surface of Ficoll Paque Plus. Centrifuged at 2000 rpm for 30 minutes, the uppermost serum from the centrifuge tube was pipetted off, PBMC was pipetted to a new 50 mL centrifuge tube, 40mL PBS was added and mixed well, centrifuged at 1300 rpm for 10 minutes at 4°C. The supernatant was pipetted off, then 15 mL PBS was added to resuspend the cells, which were counted, and centrifuged at 1300 rpm for 10 minutes at 4°C. The density of PBMC was adjusted to 4×10⁶/mL with X-vivo15 medium (Lonza, Cat No. 04-418Q), and added into a flat-bottom 96-well plate at 50 µL/well. The anti-human PD1 antibody Nivolumab and anti-human CD73 antibody (6G8 VHv8-VKv4) were diluted with X-vivo15 medium to the desired concentration, and added to the cells separately at 50µL/well, the experimental wells for combination medication were set up, that is, the anti-human PD1 antibody and anti-human CD73 antibody were added respectively at 50µL/well at the same time, and the medium was added to the control wells only, and the volume of all experimental wells was made up to 200 µL. The well plate was placed in a 5% CO₂ 37°C constant temperature incubator, and incubated for 96 hours. According to the kit instructions, the content of TNF-α (Human TNFa ELISA Kit, BD OptEIA, Cat No. 555212) and IFN-γ (Human IFN-γ ELISA Kit, BD OptEIA, Cat No. 555142) in the cell supernatant were determined. The results show that the combination of anti-CD73 antibody and anti-PD-1 antibody can significantly stimulate lymphocytes to produce TNF-α (see Figure 8A, Figure 8B).

## Claims

1. An antibody or antigen binding portion thereof that binds to human CD73, comprising a heavy chain CDR selected from amino acid sequences SEQ ID NO: 6-8, 16-18, 26-28, 36-38, 46-48, 56-58, 66-68, 76-78, 86-88, 96-98, 106-108, 116-118, 126-128, 131-133, 136-138, 141-143, 146-148 or any variant thereof, and/or a light chain CDR selected from amino acid sequences SEQ ID NO: 11-13, 21-23, 31-33, 41-43, 51-53, 61-63, 71-73, 81-83, 91-93, 101-103, 111-113, 121-123, 151-153, 156-158, 161-163, 166-168, 171-173 or any variant thereof.

2. An antibody or antigen binding portion thereof, comprising a heavy chain CDR1 selected from amino acid sequences SEQ ID NO: 6, 16, 26, 36, 46, 56, 66, 76, 86, 96, 106, 116, 126, 131, 136, 141, 146 or any variant thereof, a heavy chain CDR2 selected from amino acid sequences SEQ ID NO: 7, 17, 27, 37, 47, 57, 67, 77, 87, 97, 107, 117, 127, 132, 137, 142, 147 or any variant thereof, a heavy chain CDR3 selected from amino acid sequences SEQ ID NO: 8, 18, 28, 38, 48, 58, 68, 78, 88, 98, 108, 118, 128, 133, 138, 143, 148 or any variant thereof; and/or a light chain CDR1 selected from amino acid sequences SEQ ID NO: 11, 21, 31, 41, 51, 61, 71, 81, 91, 101, 111, 121, 151, 156, 161, 166, 171 or any variant thereof, a light chain CDR2 selected from amino acid sequences SEQ ID NO: 12, 22, 32, 42, 52, 62, 72, 82, 92, 102, 112, 122, 152, 157, 162, 167, 172 or any variant thereof, a light chain CDR3 selected from amino acid sequences SEQ ID NO: 13, 23, 33, 43, 53, 63, 73, 83, 93, 103, 113, 123, 153, 158, 163, 168, 173 or any variant thereof, optionally, the antibody or antigen binding portion thereof inhibits the CD73 hydrolase activity and mediates the endocytosis of CD73 on the cell surface.

3. An antibody or antigen-binding portion thereof that binds to human CD73, comprising a heavy chain variable region selected from amino acid sequences SEQ ID NO: 5, 15, 25, 35, 45, 55, 65, 75, 85, 95, 105, 115, 125, 130, 135, 140, 145 or any variant thereof, and/or a light chain variable region selected from amino acid sequences SEQ ID NO: 10, 20, 30, 40, 50, 60, 70, 80, 90, 100, 110, 120, 150, 155, 160, 165, 170 or any variant thereof, optionally, the antibody or antigen binding portion thereof inhibits the CD73 hydrolase activity and mediates the endocytosis of CD73 on the cell surface.

4. An antibody or antigen binding portion thereof that binds to human CD73, comprising three heavy chain variable regions CDRs and three light chain variable regions CDRs that are respectively in a pair of heavy chain and light chain variable region selected from the group: (a) SEQ ID NO:125 and 150; (b) SEQ ID NO: 125 and 155; (c) SEQ ID NO:130 and 150; (d) SEQ ID NO:130 and 155; (e) SEQ ID NO:135 and 160; (f) SEQ ID NO: 135 and 165; (g) SEQ ID NO: 140 and 160; (h) SEQ ID NO:140 and 165; (i) SEQ ID NO: 145 and 160; (j) SEQ ID NO:145 and 170, optionally, the antibody or antigen binding portion thereof inhibits the CD73 hydrolase activity and mediates the endocytosis of CD73 on the cell surface.

5. An antibody or antigen-binding portion thereof that binds to human CD73, comprising: (a) heavy chain CDR1, CDR2 and CDR3 sequences respectively comprising SEQ ID NO:126-128, and/or light chain CDR1, CDR2 and CDR3 sequences respectively comprising SEQ ID NO:151-153; (b) heavy chain CDR1, CDR2 and CDR3 sequences respectively comprising SEQ ID NO: 126-128, and/or light chain CDR1, CDR2 and CDR3 sequences respectively comprising SEQ ID NO: 156-158; (c) heavy chain CDR1, CDR2 and CDR3 sequences respectively comprising SEQ ID NO: 131-133, and/or light chain CDR1, CDR2 and CDR3 sequences respectively comprising SEQ ID NO: 151-153; (d) heavy chain CDR1, CDR2 and CDR3 sequences respectively comprising SEQ ID NO: 131-133, and/or light chain CDR1, CDR2 and CDR3 sequences respectively comprising SEQ ID NO: 156-158; (e) heavy chain CDR1, CDR2 and CDR3 sequences respectively comprising SEQ ID NO: 136-138, and/or light chain CDR1, CDR2 and CDR3 sequences respectively comprising SEQ ID NO: 161-163; (f) heavy chain CDR1, CDR2 and CDR3 sequences respectively comprising SEQ ID NO: 136-138, and/or light chain CDR1, CDR2 and CDR3 sequences respectively comprising SEQ ID NO: 166-168; (g) heavy chain CDR1, CDR2 and CDR3 sequences respectively comprising SEQ ID NO: 141-143, and/or light chain CDR1, CDR2 and CDR3 sequences respectively comprising SEQ ID NO: 161-163; (h) heavy chain CDR1, CDR2 and CDR3 sequences respectively comprising SEQ ID NO: 141-143, and/or light chain CDR1, CDR2 and CDR3 sequences respectively comprising SEQ ID NO: 166-168; (i) heavy chain CDR1, CDR2 and CDR3 sequences respectively comprising SEQ ID NO: 146-148, and/or light chain CDR1, CDR2 and CDR3 sequences respectively comprising SEQ ID NO: 161-163; (j) heavy chain CDR1, CDR2 and CDR3 sequences respectively comprising SEQ ID NO: 146-148, and/or light chain CDR1, CDR2 and CDR3 sequences respectively comprising SEQ ID NO: 171-173; optionally, the antibody or antigen binding portion thereof inhibits the CD73 hydrolase activity and mediates the endocytosis of CD73 on the cell surface.

6. The antibody or antigen-binding portion thereof according to any one of the preceding claims, comprising a heavy chain CDR1 selected from amino acid sequence SEQ ID NO:146, a heavy chain CDR2 selected from amino acid sequence SEQ ID NO:147, a heavy chain CDR3 selected from amino acid sequence SEQ ID NO:148; and a light chain CDR1 selected from amino acid sequence SEQ ID NO:171, a light chain CDR2 selected from amino acid sequence SEQ ID NO:172, a light chain CDR3 selected from amino acid sequence SEQ ID NO: 173, preferably the antibody or antigen-binding portion thereof comprising a heavy chain variable region selected from SEQ ID NO:145 and a light chain variable region selected from SEQ ID NO: 170.

7. A nucleic acid molecule encoding the antibody or a functional fragment thereof according to any one of the preceding claims, and/or a vector comprising the nucleic acid according to any one of the preceding aspects, and/or a cell comprising the vector according to any one of the preceding aspects, and/or a pharmaceutical composition comprising the antibody or a functional fragment thereof according to any one of the preceding claims, or the nucleic acid encoding same and a pharmaceutically acceptable carrier.

8. A pharmaceutical composition or kit, comprising an antibody or antigen-binding portion thereof that binds to human CD73 and an anti-PD-1 antibody, optionally, the antibody or antigen-binding portion thereof that binds to human CD73 is an antibody or functional fragment thereof according to any one of the preceding claims, optionally, the anti-PD-1 antibody is selected from nivolumab or pembrolizumab.

9. Use of the antibody or functional fragment thereof, or the nucleic acid molecule or the vector, or the cell, or the pharmaceutical composition, or the kit according to any one of the preceding claims in the preparation of a drug for the treatment of diseases associated with abnormal production of CD73 in a mammal, preferably, wherein the disease associated with abnormal production of CD73 in a mammal is cancer.

10. Use of the antibody or functional fragment thereof, or the nucleic acid molecule or the vector, or the cell, or the pharmaceutical composition, or the kit according to any one of the preceding claims in the preparation of a drug for inhibiting the activity of CD73 hydrolase.

11. Use of the antibody or functional fragment thereof, or the nucleic acid molecule or the vector, or the cell, or the pharmaceutical composition, or the kit according to any one of the preceding claims in the preparation of a reagent for mediating the endocytosis of CD73 on the cell surface.


Sequence listing
<110>  CHENGDU CONMED BIOSCIENCES CO.,LTD
<120>  ANTI-CD73 MONOCLONAL ANTIBODY AND APPLICATION THEREOF
<130>  20180924
<160>  174
<170>  PatentIn version 3.5
<210>  1
<211>  534
<212>  PRT
<213>  Mus musculus
<400>  1
Trp Glu Leu Thr Ile Leu His Thr Asn Asp Val His Ser Arg Leu Glu
1               5                   10                  15
Gln Thr Ser Glu Asp Ser Ser Lys Cys Val Asn Ala Ser Arg Cys Met
            20                  25                  30
Gly Gly Val Ala Arg Leu Phe Thr Lys Val Gln Gln Ile Arg Arg Ala
        35                  40                  45
Glu Pro Asn Val Leu Leu Leu Asp Ala Gly Asp Gln Tyr Gln Gly Thr
    50                  55                  60
Ile Trp Phe Thr Val Tyr Lys Gly Ala Glu Val Ala His Phe Met Asn
65                  70                  75                  80
Ala Leu Arg Tyr Asp Ala Met Ala Leu Gly Asn His Glu Phe Asp Asn
                85                  90                  95
Gly Val Glu Gly Leu Ile Glu Pro Leu Leu Lys Glu Ala Lys Phe Pro
            100                 105                 110
Ile Leu Ser Ala Asn Ile Lys Ala Lys Gly Pro Leu Ala Ser Gln Ile
        115                 120                 125
Ser Gly Leu Tyr Leu Pro Tyr Lys Val Leu Pro Val Gly Asp Glu Val
    130                 135                 140
Val Gly Ile Val Gly Tyr Thr Ser Lys Glu Thr Pro Phe Leu Ser Asn
145                 150                 155                 160
Pro Gly Thr Asn Leu Val Phe Glu Asp Glu Ile Thr Ala Leu Gln Pro
                165                 170                 175
Glu Val Asp Lys Leu Lys Thr Leu Asn Val Asn Lys Ile Ile Ala Leu
            180                 185                 190
Gly His Ser Gly Phe Glu Met Asp Lys Leu Ile Ala Gln Lys Val Arg
        195                 200                 205
Gly Val Asp Val Val Val Gly Gly His Ser Asn Thr Phe Leu Tyr Thr
    210                 215                 220
Gly Asn Pro Pro Ser Lys Glu Val Pro Ala Gly Lys Tyr Pro Phe Ile
225                 230                 235                 240
Val Thr Ser Asp Asp Gly Arg Lys Val Pro Val Val Gln Ala Tyr Ala
                245                 250                 255
Phe Gly Lys Tyr Leu Gly Tyr Leu Lys Ile Glu Phe Asp Glu Arg Gly
            260                 265                 270
Asn Val Ile Ser Ser His Gly Asn Pro Ile Leu Leu Asn Ser Ser Ile
        275                 280                 285
Pro Glu Asp Pro Ser Ile Lys Ala Asp Ile Asn Lys Trp Arg Ile Lys
    290                 295                 300
Leu Asp Asn Tyr Ser Thr Gln Glu Leu Gly Lys Thr Ile Val Tyr Leu
305                 310                 315                 320
Asp Gly Ser Ser Gln Ser Cys Arg Phe Arg Glu Cys Asn Met Gly Asn
                325                 330                 335
Leu Ile Cys Asp Ala Met Ile Asn Asn Asn Leu Arg His Thr Asp Glu
            340                 345                 350
Met Phe Trp Asn His Val Ser Met Cys Ile Leu Asn Gly Gly Gly Ile
        355                 360                 365
Arg Ser Pro Ile Asp Glu Arg Asn Asn Gly Thr Ile Thr Trp Glu Asn
    370                 375                 380
Leu Ala Ala Val Leu Pro Phe Gly Gly Thr Phe Asp Leu Val Gln Leu
385                 390                 395                 400
Lys Gly Ser Thr Leu Lys Lys Ala Phe Glu His Ser Val His Arg Tyr
                405                 410                 415

Gly Gln Ser Thr Gly Glu Phe Leu Gln Val Gly Gly Ile His Val Val
                420                 425                 430
Tyr Asp Leu Ser Arg Lys Pro Gly Asp Arg Val Val Lys Leu Asp Val
        435                 440                 445
Leu Cys Thr Lys Cys Arg Val Pro Ser Tyr Asp Pro Leu Lys Met Asp
    450                 455                 460
Glu Val Tyr Lys Val Ile Leu Pro Asn Phe Leu Ala Asn Gly Gly Asp
465                 470                 475                 480
Gly Phe Gln Met Ile Lys Asp Glu Leu Leu Arg His Asp Ser Gly Asp
                485                 490                 495
Gln Asp Ile Asn Val Val Ser Thr Tyr Ile Ser Lys Met Lys Val Ile
            500                 505                 510
Tyr Pro Ala Val Glu Gly Arg Ile Lys Ala Ser Gly Gly Gly Gly Ser
        515                 520                 525
His His His His His His
    530

<210>  2
<211>  534
<212>  PRT
<213>  Mus musculus

<400>  2
Trp Glu Leu Thr Ile Leu His Thr Asn Asp Val His Ser Arg Leu Glu
1               5                   10                  15
Gln Thr Ser Glu Asp Ser Ser Lys Cys Val Asn Ala Ser Arg Cys Met
            20                  25                  30
Gly Gly Val Ala Arg Leu Phe Thr Lys Val Gln Gln Ile Arg Arg Ala
        35                  40                  45
Glu Pro Asn Val Leu Leu Leu Asp Ala Gly Asp Gln Tyr Gln Gly Thr
    50                  55                  60
Ile Trp Phe Thr Val Tyr Lys Gly Ala Glu Val Ala His Phe Met Asn
65                  70                  75                  80
Ala Leu Arg Tyr Asp Ala Met Ala Leu Gly Asn His Glu Phe Asp Asn
                85                  90                  95
Gly Val Glu Gly Leu Ile Glu Pro Leu Leu Lys Glu Ala Lys Phe Pro
            100                 105                 110
Ile Leu Ser Ala Asn Ile Lys Ala Lys Gly Pro Leu Ala Ser Gln Ile
        115                 120                 125
Ser Gly Leu Tyr Leu Pro Tyr Lys Val Leu Pro Val Gly Asp Glu Val
    130                 135                 140
Val Gly Ile Val Gly Tyr Thr Ser Lys Glu Thr Pro Phe Leu Ser Asn
145                 150                 155                 160
Pro Gly Thr Asn Leu Val Phe Glu Asp Glu Ile Thr Ala Leu Gln Pro
                165                 170                 175
Glu Val Asp Lys Leu Lys Thr Leu Asn Val Asn Lys Ile Ile Ala Leu
            180                 185                 190
Gly His Ser Gly Phe Glu Thr Asp Lys Leu Ile Ala Gln Lys Val Arg
        195                 200                 205
Gly Val Asp Val Val Val Gly Gly His Ser Asn Thr Phe Leu Tyr Thr
    210                 215                 220
Gly Asn Pro Pro Ser Lys Glu Val Pro Ala Gly Lys Tyr Pro Phe Ile
225                 230                 235                 240
Val Thr Ser Asp Asp Gly Arg Lys Val Pro Val Val Gln Ala Tyr Ala
                245                 250                 255
Phe Gly Lys Tyr Leu Gly Tyr Leu Lys Ile Glu Phe Asp Glu Arg Gly
            260                 265                 270
Asn Val Ile Ser Ser His Gly Asn Pro Ile Leu Leu Asn Ser Ser Ile
        275                 280                 285
Pro Glu Asp Pro Ser Ile Lys Ala Asp Ile Asn Lys Trp Arg Ile Lys
    290                 295                 300
Leu Asp Asn Tyr Ser Thr Gln Glu Leu Gly Lys Thr Ile Val Tyr Leu
305                 310                 315                 320

Asp Gly Ser Ser Gln Ser Cys Arg Phe Arg Glu Cys Asn Met Gly Asn
                325                 330                 335
Leu Ile Cys Asp Ala Met Ile Asn Asn Asn Leu Arg His Ala Asp Glu
            340                 345                 350
Met Phe Trp Asn His Val Ser Met Cys Ile Leu Asn Gly Gly Gly Ile
        355                 360                 365
Arg Ser Pro Ile Asp Glu Arg Asn Asn Gly Thr Ile Thr Trp Glu Asn
    370                 375                 380
Leu Ala Ala Val Leu Pro Phe Gly Gly Thr Phe Asp Leu Val Gln Leu
385                 390                 395                 400
Lys Gly Ser Thr Leu Lys Lys Ala Phe Glu His Ser Val His Arg Tyr
                405                 410                 415
Gly Gln Ser Thr Gly Glu Phe Leu Gln Val Gly Gly Ile His Val Val
            420                 425                 430
Tyr Asp Leu Ser Arg Lys Pro Gly Asp Arg Val Val Lys Leu Asp Val
        435                 440                 445
Leu Cys Thr Lys Cys Arg Val Pro Ser Tyr Asp Pro Leu Lys Met Asp
    450                 455                 460
Glu Ile Tyr Lys Val Ile Leu Pro Asn Phe Leu Ala Asn Gly Gly Asp
465                 470                 475                 480
Gly Phe Gln Met Ile Lys Asp Glu Leu Leu Arg His Asp Ser Gly Asp
                485                 490                 495
Gln Asp Ile Asn Val Val Ser Thr Tyr Ile Ser Lys Met Lys Val Ile
            500                 505                 510
Tyr Pro Ala Val Glu Gly Arg Ile Lys Ala Ser Gly Gly Gly Gly Ser
        515                 520                 525
His His His His His His
    530

<210>  3
<211>  534
<212>  PRT
<213>  Mus musculus

<400>  3
Trp Glu Leu Thr Ile Leu His Thr Asn Asp Val His Ser Arg Leu Glu
1               5                   10                  15
Gln Thr Ser Asp Asp Ser Thr Lys Cys Leu Asn Ala Ser Leu Cys Val
            20                  25                  30
Gly Gly Val Ala Arg Leu Phe Thr Lys Val Gln Gln Ile Arg Lys Glu
        35                  40                  45
Glu Pro Asn Val Leu Phe Leu Asp Ala Gly Asp Gln Tyr Gln Gly Thr
    50                  55                  60
Ile Trp Phe Thr Val Tyr Lys Gly Leu Glu Val Ala His Phe Met Asn
65                  70                  75                  80
Ile Leu Gly Tyr Asp Ala Met Ala Leu Gly Asn His Glu Phe Asp Asn
                85                  90                  95
Gly Val Glu Gly Leu Ile Asp Pro Leu Leu Arg Asn Val Lys Phe Pro
            100                 105                 110
Ile Leu Ser Ala Asn Ile Lys Ala Arg Gly Pro Leu Ala His Gln Ile
        115                 120                 125
Ser Gly Leu Phe Leu Pro Ser Lys Val Leu Ser Val Gly Gly Glu Val
    130                 135                 140
Val Gly Ile Val Gly Tyr Thr Ser Lys Glu Thr Pro Phe Leu Ser Asn
145                 150                 155                 160
Pro Gly Thr Asn Leu Val Phe Glu Asp Glu Ile Ser Ala Leu Gln Pro
                165                 170                 175
Glu Val Asp Lys Leu Lys Thr Leu Asn Val Asn Lys Ile Ile Ala Leu
            180                 185                 190
Gly His Ser Gly Phe Glu Met Asp Lys Leu Ile Ala Gln Lys Val Arg
        195                 200                 205
Gly Val Asp Ile Val Val Gly Gly His Ser Asn Thr Phe Leu Tyr Thr
    210                 215                 220

Gly Asn Pro Pro Ser Lys Glu Val Pro Ala Gly Lys Tyr Pro Phe Ile
225                 230                 235                 240
Val Thr Ala Asp Asp Gly Arg Gln Val Pro Val Val Gln Ala Tyr Ala
                245                 250                 255
Phe Gly Lys Tyr Leu Gly Tyr Leu Lys Val Glu Phe Asp Asp Lys Gly
            260                 265                 270
Asn Val Ile Thr Ser Tyr Gly Asn Pro Ile Leu Leu Asn Ser Ser Ile
        275                 280                 285
Pro Glu Asp Ala Thr Ile Lys Ala Asp Ile Asn Gln Trp Arg Ile Lys
    290                 295                 300
Leu Asp Asn Tyr Ser Thr Gln Glu Leu Gly Arg Thr Ile Val Tyr Leu
305                 310                 315                 320
Asp Gly Ser Thr Gln Thr Cys Arg Phe Arg Glu Cys Asn Met Gly Asn
                325                 330                 335
Leu Ile Cys Asp Ala Met Ile Asn Asn Asn Leu Arg His Pro Asp Glu
            340                 345                 350
Met Phe Trp Asn His Val Ser Met Cys Ile Val Asn Gly Gly Gly Ile
        355                 360                 365
Arg Ser Pro Ile Asp Glu Lys Asn Asn Gly Thr Ile Thr Trp Glu Asn
    370                 375                 380
Leu Ala Ala Val Leu Pro Phe Gly Gly Thr Phe Asp Leu Val Gln Leu
385                 390                 395                 400
Lys Gly Ser Thr Leu Lys Lys Ala Phe Glu His Ser Val His Arg Tyr
                405                 410                 415
Gly Gln Ser Thr Gly Glu Phe Leu Gln Val Gly Gly Ile His Val Val
            420                 425                 430
Tyr Asp Ile Asn Arg Lys Pro Trp Asn Arg Val Val Gln Leu Glu Val
        435                 440                 445
Leu Cys Thr Lys Cys Arg Val Pro Ile Tyr Glu Pro Leu Glu Met Asp
    450                 455                 460
Lys Val Tyr Lys Val Thr Leu Pro Ser Tyr Leu Ala Asn Gly Gly Asp
465                 470                 475                 480
Gly Phe Gln Met Ile Lys Asp Glu Leu Leu Lys His Asp Ser Gly Asp
                485                 490                 495
Gln Asp Ile Ser Val Val Ser Glu Tyr Ile Ser Lys Met Lys Val Val
            500                 505                 510
Tyr Pro Ala Val Glu Gly Arg Ile Lys Ala Ser Gly Gly Gly Gly Ser
        515                 520                 525
His His His His His His
    530

<210>  4
<211>  574
<212>  PRT
<213>  Homo sapiens

<400>  4
Met Cys Pro Arg Ala Ala Arg Ala Pro Ala Thr Leu Leu Leu Ala Leu
1               5                   10                  15
Gly Ala Val Leu Trp Pro Ala Ala Gly Ala Trp Glu Leu Thr Ile Leu
            20                  25                  30
His Thr Asn Asp Val His Ser Arg Leu Glu Gln Thr Ser Glu Asp Ser
        35                  40                  45
Ser Lys Cys Val Asn Ala Ser Arg Cys Met Gly Gly Val Ala Arg Leu
    50                  55                  60
Phe Thr Lys Val Gln Gln Ile Arg Arg Ala Glu Pro Asn Val Leu Leu
65                  70                  75                  80
Leu Asp Ala Gly Asp Gln Tyr Gln Gly Thr Ile Trp Phe Thr Val Tyr
                85                  90                  95
Lys Gly Ala Glu Val Ala His Phe Met Asn Ala Leu Arg Tyr Asp Ala
            100                 105                 110
Met Ala Leu Gly Asn His Glu Phe Asp Asn Gly Val Glu Gly Leu Ile
        115                 120                 125

Glu Pro Leu Leu Lys Glu Ala Lys Phe Pro Ile Leu Ser Ala Asn Ile
    130                 135                 140
Lys Ala Lys Gly Pro Leu Ala Ser Gln Ile Ser Gly Leu Tyr Leu Pro
145                 150                 155                 160
Tyr Lys Val Leu Pro Val Gly Asp Glu Val Val Gly Ile Val Gly Tyr
                165                 170                 175
Thr Ser Lys Glu Thr Pro Phe Leu Ser Asn Pro Gly Thr Asn Leu Val
            180                 185                 190
Phe Glu Asp Glu Ile Thr Ala Leu Gln Pro Glu Val Asp Lys Leu Lys
        195                 200                 205
Thr Leu Asn Val Asn Lys Ile Ile Ala Leu Gly His Ser Gly Phe Glu
    210                 215                 220
Met Asp Lys Leu Ile Ala Gln Lys Val Arg Gly Val Asp Val Val Val
225                 230                 235                 240
Gly Gly His Ser Asn Thr Phe Leu Tyr Thr Gly Asn Pro Pro Ser Lys
                245                 250                 255
Glu Val Pro Ala Gly Lys Tyr Pro Phe Ile Val Thr Ser Asp Asp Gly
            260                 265                 270
Arg Lys Val Pro Val Val Gln Ala Tyr Ala Phe Gly Lys Tyr Leu Gly
        275                 280                 285
Tyr Leu Lys Ile Glu Phe Asp Glu Arg Gly Asn Val Ile Ser Ser His
    290                 295                 300
Gly Asn Pro Ile Leu Leu Asn Ser Ser Ile Pro Glu Asp Pro Ser Ile
305                 310                 315                 320
Lys Ala Asp Ile Asn Lys Trp Arg Ile Lys Leu Asp Asn Tyr Ser Thr
                325                 330                 335
Gln Glu Leu Gly Lys Thr Ile Val Tyr Leu Asp Gly Ser Ser Gln Ser
            340                 345                 350
Cys Arg Phe Arg Glu Cys Asn Met Gly Asn Leu Ile Cys Asp Ala Met
        355                 360                 365
Ile Asn Asn Asn Leu Arg His Thr Asp Glu Met Phe Trp Asn His Val
    370                 375                 380
Ser Met Cys Ile Leu Asn Gly Gly Gly Ile Arg Ser Pro Ile Asp Glu
385                 390                 395                 400
Arg Asn Asn Gly Thr Ile Thr Trp Glu Asn Leu Ala Ala Val Leu Pro
                405                 410                 415
Phe Gly Gly Thr Phe Asp Leu Val Gln Leu Lys Gly Ser Thr Leu Lys
            420                 425                 430
Lys Ala Phe Glu His Ser Val His Arg Tyr Gly Gln Ser Thr Gly Glu
        435                 440                 445
Phe Leu Gln Val Gly Gly Ile His Val Val Tyr Asp Leu Ser Arg Lys
    450                 455                 460
Pro Gly Asp Arg Val Val Lys Leu Asp Val Leu Cys Thr Lys Cys Arg
465                 470                 475                 480
Val Pro Ser Tyr Asp Pro Leu Lys Met Asp Glu Val Tyr Lys Val Ile
                485                 490                 495
Leu Pro Asn Phe Leu Ala Asn Gly Gly Asp Gly Phe Gln Met Ile Lys
            500                 505                 510
Asp Glu Leu Leu Arg His Asp Ser Gly Asp Gln Asp Ile Asn Val Val
        515                 520                 525
Ser Thr Tyr Ile Ser Lys Met Lys Val Ile Tyr Pro Ala Val Glu Gly
    530                 535                 540
Arg Ile Lys Phe Ser Thr Gly Ser His Cys His Gly Ser Phe Ser Leu
545                 550                 555                 560
Ile Phe Leu Ser Leu Trp Ala Val Ile Phe Val Leu Tyr Gln
                565                 570

<210>  5
<211>  120
<212>  PRT
<213>  Mus musculus

<400>  5

Glu Val Lys Leu Val Glu Ser Gly Gly Gly Leu Val Gln Pro Gly Gly
1               5                   10                  15
Ser Leu Lys Leu Ser Cys Ala Thr Ser Gly Phe Thr Phe Ser Asp Tyr
            20                  25                  30
Tyr Met Tyr Trp Val Arg Gln Thr Pro Glu Lys Arg Leu Glu Trp Val
        35                  40                  45
Ala Tyr Ile Ser Asn Val Gly Gly Ser Thr Tyr Tyr Pro Asp Thr Val
    50                  55                  60
Lys Gly Arg Phe Thr Ile Ser Arg Asp Asn Ala Lys Asn Thr Leu Tyr
65                  70                  75                  80
Leu Gln Met Ser Arg Leu Lys Ser Glu Asp Thr Ala Ile Tyr Tyr Cys
                85                  90                  95
Ala Arg Gly Asp Gly Tyr Tyr Gly Tyr Tyr Phe Asp Val Trp Gly Ala
            100                 105                 110
Gly Thr Thr Val Thr Val Ser Ser
        115                 120

<210>  6
<211>  6
<212>  PRT
<213>  Mus musculus

<400>  6
Ser Asp Tyr Tyr Met Tyr
1               5

<210>  7
<211>  17
<212>  PRT
<213>  Mus musculus

<400>  7
Tyr Ile Ser Asn Val Gly Gly Ser Thr Tyr Tyr Pro Asp Thr Val Lys
1               5                   10                  15
Gly


<210>  8
<211>  11
<212>  PRT
<213>  Mus musculus

<400>  8
Gly Asp Gly Tyr Tyr Gly Tyr Tyr Phe Asp Val
1               5                   10

<210>  9
<211>  360
<212>  DNA
<213>  Mus musculus

<400>  9
gaggtgaagc tggtggagtc tgggggaggc ttagtgcagc ctggagggtc cctgaaactc      60
tcctgtgcaa cctctggatt cactttcagt gactattaca tgtattgggt tcgccagact     120
ccagagaaga ggctggagtg ggtcgcatac attagtaatg ttggtggtag cacctattat     180
ccagacactg taaagggccg attcaccatc tccagagaca atgccaagaa caccctgtac     240
ctgcaaatga gccgtctgaa gtctgaggac acagccatat attactgtgc aagaggggat     300
ggttactacg gatactactt cgatgtctgg ggcgcaggga ccacggtcac cgtctcctca     360

<210>  10
<211>  106
<212>  PRT
<213>  Mus musculus

<400>  10
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Tyr Thr
                85                  90                  95
Phe Gly Gly Gly Thr Lys Leu Glu Ile Lys
            100                 105

<210>  11
<211>  10
<212>  PRT
<213>  Mus musculus

<400>  11
Ser Ala Ser Ser Ser Val Ser Tyr Met His
1               5                   10

<210>  12
<211>  7
<212>  PRT
<213>  Mus musculus

<400>  12
Asp Thr Ser Lys Leu Ala Ser
1               5

<210>  13
<211>  9
<212>  PRT
<213>  Mus musculus

<400>  13
Gln Gln Trp Ser Ser Asn Pro Tyr Thr
1               5

<210>  14
<211>  318
<212>  DNA
<213>  Mus musculus

<400>  14
caaattgttc tcacccagtc tccagcaatc atgtctgcat ctccagggga gaaggtcacc      60
atgacctgca gtgccagctc aagtgtaagt tacatgcact ggtaccagca gaagtcaggc     120
acctccccca aaagatggat ttatgacaca tccaaactgg cttctggagt ccctgctcgc     180
ttcagtggca gtgggtctgg gacctcttac tctctcacaa tcagcagcat ggaggctgaa     240
gatgctgcca cttattactg ccagcagtgg agtagtaacc cgtacacgtt cggagggggg     300
accaagctgg aaatcaaa                                                   318

<210>  15
<211>  120
<212>  PRT
<213>  Mus musculus

<400>  15

Glu Val Lys Leu Val Glu Ser Gly Gly Gly Leu Val Gln Pro Gly Gly
1               5                   10                  15
Ser Leu Lys Leu Ser Cys Ala Thr Ser Gly Phe Thr Phe Ser Asp Tyr
            20                  25                  30
Tyr Met Tyr Trp Val Arg Gln Thr Pro Glu Lys Arg Leu Glu Trp Val
        35                  40                  45
Ala Tyr Ile Ser Asn Val Gly Gly Ser Thr Tyr Tyr Pro Asp Thr Val
    50                  55                  60
Lys Gly Arg Phe Thr Ile Ser Arg Asp Asn Ala Lys Asn Thr Leu Tyr
65                  70                  75                  80
Leu Gln Met Ser Arg Leu Lys Ser Glu Asp Thr Ala Met Tyr Tyr Cys
                85                  90                  95
Ala Arg Gly Asp Gly Tyr Tyr Gly Tyr Tyr Phe Asp Val Trp Gly Ala
            100                 105                 110
Gly Thr Thr Val Thr Val Ser Ser
        115                 120

<210>  16
<211>  6
<212>  PRT
<213>  Mus musculus

<400>  16
Ser Asp Tyr Tyr Met Tyr
1               5

<210>  17
<211>  17
<212>  PRT
<213>  Mus musculus

<400>  17
Tyr Ile Ser Asn Val Gly Gly Ser Thr Tyr Tyr Pro Asp Thr Val Lys
1               5                   10                  15
Gly


<210>  18
<211>  11
<212>  PRT
<213>  Mus musculus

<400>  18
Gly Asp Gly Tyr Tyr Gly Tyr Tyr Phe Asp Val
1               5                   10

<210>  19
<211>  360
<212>  DNA
<213>  Mus musculus

<400>  19
gaagtgaagc tggtggaatc tgggggaggc ttagtgcagc ctggagggtc cctgaaactc      60
tcctgtgcaa cctctggatt cactttcagt gactattaca tgtattgggt tcgccagact     120
ccagagaaga ggctggagtg ggtcgcatat attagtaatg ttggtggtag cacctattat     180
ccagacactg taaagggccg attcaccatc tccagagaca atgccaagaa caccctgtac     240
ctgcaaatga gccgtctgaa gtctgaggac acagccatgt attactgtgc aagaggggat     300
ggttactacg gatactactt cgatgtctgg ggcgcaggga ccacggtcac cgtctcctca     360

<210>  20
<211>  106
<212>  PRT
<213>  Mus musculus

<400>  20
Glu Asn Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Thr Arg Phe Ser Gly Ser
    50                  55                  60
Gly Ser Gly Thr Ser Phe Ser Leu Thr Ile Ser Ser Met Glu Ala Glu
65                  70                  75                  80
Asp Ala Ala Thr Tyr Tyr Cys Gln Gln Trp Ser Ser Asn Pro Tyr Thr
                85                  90                  95
Phe Gly Gly Gly Thr Lys Leu Glu Ile Lys
            100                 105

<210>  21
<211>  10
<212>  PRT
<213>  Mus musculus

<400>  21
Ser Ala Ser Ser Ser Val Ser Tyr Met His
1               5                   10

<210>  22
<211>  7
<212>  PRT
<213>  Mus musculus

<400>  22
Asp Thr Ser Lys Leu Ala Ser
1               5

<210>  23
<211>  9
<212>  PRT
<213>  Mus musculus

<400>  23
Gln Gln Trp Ser Ser Asn Pro Tyr Thr
1               5

<210>  24
<211>  318
<212>  DNA
<213>  Mus musculus

<400>  24
gaaaatgtgc tcacccagtc tccagcaatc atgtctgcat ctccagggga gaaggtcacc      60
atgacctgca gtgccagctc aagtgtaagt tacatgcact ggtaccagca gaagtctggc     120
acctccccca aaagatggat ttatgacaca tccaaactgg cttctggagt ccctactcgc     180
ttcagtggca gtgggtctgg gacctctttc tctctcacaa tcagcagcat ggaggctgaa     240
gatgctgcca cttattactg ccagcagtgg agtagtaacc cgtacacgtt cggagggggg     300
accaagctgg aaataaaa                                                   318

<210>  25
<211>  112
<212>  PRT
<213>  Mus musculus

<400>  25

Gln Val Gln Phe Gln Gln Ser Gly Ala Glu Leu Ala Arg Pro Gly Thr
1               5                   10                  15
Ser Val Lys Leu Ser Cys Lys Ala Ser Gly Tyr Ile Phe Thr Ser Tyr
            20                  25                  30
Trp Ile Gln Trp Ile Lys Gln Arg Pro Gly Gln Gly Leu Glu Trp Ile
        35                  40                  45
Gly Ala Ile Tyr Pro Gly Asp Gly Asp Thr Arg Tyr Thr Gln Lys Phe
    50                  55                  60
Lys Gly Lys Ala Thr Leu Thr Ala Glu Lys Ser Ser Ser Thr Ala Tyr
65                  70                  75                  80
Met Gln Leu Arg Ser Leu Ala Ser Glu Asp Ser Ala Val Tyr Tyr Cys
                85                  90                  95
Ala Gly Phe Ala Ser Trp Gly Gln Gly Thr Leu Leu Thr Val Ser Ala
            100                 105                 110

<210>  26
<211>  6
<212>  PRT
<213>  Mus musculus

<400>  26
Thr Ser Tyr Trp Ile Gln
1               5

<210>  27
<211>  17
<212>  PRT
<213>  Mus musculus

<400>  27
Ala Ile Tyr Pro Gly Asp Gly Asp Thr Arg Tyr Thr Gln Lys Phe Lys
1               5                   10                  15
Gly

<210>  28
<211>  4
<212>  PRT
<213>  Mus musculus

<400>  28
Phe Ala Ser Trp
1

<210>  29
<211>  336
<212>  DNA
<213>  Mus musculus

<400>  29
caggttcagt tccagcagtc aggggctgag ctggcaagac ctgggacttc agtgaagttg      60
tcctgcaagg cttctggcta catttttact agttactgga tacagtggat aaaacagaga     120
cctggacagg gtctggaatg gattggggct atttatcctg gagatggtga tactagatat     180
actcagaagt ttaagggcaa ggccacattg actgcagaaa aatcctccag tacagcctac     240
atgcaactca gaagcttggc gtctgaggac tctgcggtct attactgtgc aggttttgct     300
tcctggggcc aagggactct gctcactgtc tctgca                               336

<210>  30
<211>  107
<212>  PRT
<213>  Mus musculus

<400>  30

Asp Ile Gln Met Thr Gln Ser Pro Ser Ser Leu Ser Ala Ser Leu Gly
1               5                   10                  15
Glu Arg Val Asn Leu Thr Cys Arg Ala Ser Gln Asp Ile Gly Asp Ser
            20                  25                  30
Leu Ile Trp Leu Gln Gln Glu Pro Asp Gly Thr Ile Lys Arg Leu Ile
        35                  40                  45
Tyr Ala Thr Ser Ser Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Leu Val Asp Tyr Tyr Cys Leu Gln Tyr Ala Ser Ser Pro Phe
                85                  90                  95
Thr Phe Gly Gly Gly Thr Lys Leu Glu Ile Lys
            100                 105

<210>  31
<211>  11
<212>  PRT
<213>  Mus musculus

<400>  31
Arg Ala Ser Gln Asp Ile Gly Asp Ser Leu Ile
1               5                   10

<210>  32
<211>  7
<212>  PRT
<213>  Mus musculus

<400>  32
Ala Thr Ser Ser Leu Asp Ser
1               5

<210>  33
<211>  9
<212>  PRT
<213>  Mus musculus

<400>  33
Leu Gln Tyr Ala Ser Ser Pro Phe Thr
1               5

<210>  34
<211>  321
<212>  DNA
<213>  Mus musculus

<400>  34
gacatccaga tgacccagtc tccatcctcc ttatctgcct ctctgggaga aagagtcaat      60
ctcacttgtc gggcaagtca ggacattggt gatagtttaa tctggcttca gcaggaacca     120
gatggaacta ttaaacgcct gatctacgcc acatccagtt tagattctgg tgtccccaaa     180
aggttcagtg gcagtaggtc tgggtcagat tattctctca ccatcagcag ccttgagtct     240
gaagatcttg tagactatta ctgtctacaa tatgctagtt ctccgttcac gttcggaggg     300
gggaccaagc tggaaatcaa a                                               321

<210>  35
<211>  119
<212>  PRT
<213>  Mus musculus

<400>  35
Gln Val Thr Leu Lys Glu Ser Gly Pro Gly Ile Leu Lys Pro Ser Gln
1               5                   10                  15

Thr Leu Ser Leu Thr Cys Ser Phe Ser Gly Phe Ser Leu Asn Thr Ser
            20                  25                  30
Gly Met Gly Val Gly Trp Ile Arg Gln Pro Ser Gly Lys Gly Leu Glu
        35                  40                  45
Trp Leu Ala His Ile Trp Trp Asp Asp Asp Lys Tyr Tyr Asn Pro Ser
    50                  55                  60
Leu Lys Ser Gln Leu Thr Ile Ser Lys Asp Thr Ser Arg Asn Gln Val
65                  70                  75                  80
Phe Leu Arg Ile Ala Ser Val Asp Thr Ala Asp Thr Ala Thr Tyr Tyr
                85                  90                  95
Cys Ala Arg Arg Glu Arg Trp Ala Trp Phe Ala Tyr Trp Gly Gln Gly
            100                 105                 110
Thr Leu Val Thr Val Ser Ala
        115

<210>  36
<211>  12
<212>  PRT
<213>  Mus musculus

<400>  36
Gly Phe Ser Leu Asn Thr Ser Gly Met Gly Val Gly
1               5                   10

<210>  37
<211>  16
<212>  PRT
<213>  Mus musculus

<400>  37
His Ile Trp Trp Asp Asp Asp Lys Tyr Tyr Asn Pro Ser Leu Lys Ser
1               5                   10                  15

<210>  38
<211>  9
<212>  PRT
<213>  Mus musculus

<400>  38
Arg Glu Arg Trp Ala Trp Phe Ala Tyr
1               5

<210>  39
<211>  357
<212>  DNA
<213>  Mus musculus

<400>  39
caagttactc taaaagagtc tggccctggg atattgaagc cctcacagac cctcagtctg      60
acttgttctt tctctgggtt ttcactgaac acttctggta tgggtgtagg ctggattcgt     120
cagccttcag ggaagggtct ggagtggctg gcgcacattt ggtgggatga tgataaatac     180
tataatccat ccctgaagag ccagctcaca atctccaagg atacctccag aaaccaggta     240
ttcctcagga tcgccagtgt ggacactgca gatactgcca cttactactg tgctcgaaga     300
gaacggtggg cctggtttgc ttactggggc caagggactc tggtcactgt ctctgca        357

<210>  40
<211>  106
<212>  PRT
<213>  Mus musculus

<400>  40
Asp Ile Val Met Thr Gln Ser Gln Lys Phe Met Ser Ala Ser Val Gly
1               5                   10                  15

Asp Arg Val Ser Val Thr Cys Lys Ala Ser Gln Asn Val Phe Thr Asn
            20                  25                  30
Val Ala Trp Tyr Gln Gln Lys Pro Gly Gln Ser Pro Lys Val Leu Ile
        35                  40                  45
Tyr Ser Ala Ser Tyr Arg Tyr Ser Gly Val Pro Asp Arg Phe Thr Gly
    50                  55                  60
Ser Gly Ser Gly Thr Asp Phe Thr Leu Thr Ile Asn Asn Val Gln Ser
65                  70                  75                  80
Glu Asp Leu Ala Glu Tyr Phe Cys Gln Gln Tyr Asn Ser Tyr Pro Thr
                85                  90                  95
Phe Gly Ser Gly Thr Lys Leu Glu Ile Lys
            100                 105

<210> 41
<211> 11
<212> PRT
<213> Mus musculus

<400> 41
Lys Ala Ser Gln Asn Val Phe Thr Asn Val Ala
1               5                   10

<210> 42
<211> 7
<212> PRT
<213> Mus musculus

<400> 42
Ser Ala Ser Tyr Arg Tyr Ser
1               5

<210> 43
<211> 8
<212> PRT
<213> Mus musculus

<400> 43
Gln Gln Tyr Asn Ser Tyr Pro Thr
1               5

<210> 44
<211> 318
<212> DNA
<213> Mus musculus

<400> 44
gacattgtga tgacccagtc tcaaaaattc atgtccgcat ctgtaggaga cagggtcagc 60
gtcacctgca aggccagtca gaatgtgttt actaatgtag cctggtatca gcagaaacca 120
gggcaatctc ctaaagtact gatttactcg gcatcctacc ggtacagtgg agtccctgat 180
cgcttcacag gctctggctc tgggacagat ttcactctca ccatcaacaa tgtgcagtct 240
gaagacttgg cagagtattt ctgtcagcaa tataacagct atcccacgtt cggctcgggg 300
acaaagttgg aaataaaa 318

<210> 45
<211> 112
<212> PRT
<213> Mus musculus

<400> 45
Gln Val Gln Phe Gln Gln Ser Gly Ala Glu Leu Val Arg Pro Gly Ala
1               5                   10                  15
Ser Val Lys Leu Ser Cys Lys Ala Ser Gly Tyr Ile Phe Ser Ser Tyr
            20                  25                  30

Trp Ile Gln Trp Val Lys Gln Arg Pro Gly Gln Gly Leu Glu Trp Ile
        35                  40                  45
Gly Ala Ile Tyr Pro Gly Asp Gly Asp Thr Arg Tyr Thr Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Ala Ser Glu Asp Ser Ala Val Tyr Tyr Cys
                85                  90                  95
Ala Gly Phe Ala Ser Trp Gly Gln Gly Thr Leu Val Thr Val Ser Ala
            100                 105                 110

<210> 46
<211> 10
<212> PRT
<213> Mus musculus

<400> 46
Gly Tyr Ile Phe Ser Ser Tyr Trp Ile Gln
1               5                   10

<210> 47
<211> 17
<212> PRT
<213> Mus musculus

<400> 47
Ala Ile Tyr Pro Gly Asp Gly Asp Thr Arg Tyr Thr Gln Lys Phe Lys
1               5                   10                  15
Asp

<210> 48
<211> 5
<212> PRT
<213> Mus musculus

<400> 48
Ala Gly Phe Ala Ser
1               5

<210> 49
<211> 336
<212> DNA
<213> Mus musculus

<400> 49
caggttcagt tccagcagtc tggggctgag ctggtaagac ctggggcttc agtgaagttg 60
tcctgcaagg cttctggcta catctttagt agctactgga tacagtgggt aaaacagagg 120
cctggacagg gtctggaatg gattggggct atttatcctg gagatggtga tactagatac 180
actcagaagt tcaaggacaa ggccacattg actgcagata aatcctccag cacagcctac 240
atgcaactca gcagcttggc atctgaggac tctgcggtct attactgtgc aggttttgct 300
tcctggggcc aagggactct ggtcactgtc tctgca 336

<210> 50
<211> 107
<212> PRT
<213> Mus musculus

<400> 50
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Ser Ser
            20                  25                  30

Leu Asn Trp Leu Gln Gln Glu Pro Asn Gly Thr Ile Lys Arg Leu Ile
        35                  40                  45
Tyr Ala Thr Ser Ser Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Ala Asp Tyr Tyr Cys Leu Gln Tyr Ala Ser Ser Pro Tyr
                85                  90                  95
Thr Phe Gly Gly Gly Thr Lys Leu Glu Ile Lys
            100                 105

<210>  51
<211>  11
<212>  PRT
<213>  Mus musculus

<400>  51
Arg Ala Ser Gln Asp Ile Gly Ser Ser Leu Asn
1               5                   10

<210>  52
<211>  8
<212>  PRT
<213>  Mus musculus

<400>  52
Ala Thr Ser Ser Leu Asp Ser Gly
1               5

<210>  53
<211>  9
<212>  PRT
<213>  Mus musculus

<400>  53
Leu Gln Tyr Ala Ser Ser Pro Tyr Thr
1               5

<210>  54
<211>  321
<212>  DNA
<213>  Mus musculus

<400>  54
gacatccaga tgacccagtc tccatcctcc ttatctgcct ctctgggaga aagagtcagt      60
ctcacttgtc gggcaagtca ggacattggt agtagcttaa actggcttca gcaggaacca     120
aatggaacta ttaaacgcct gatctacgcc acatccagtt tagattctgg tgtccccaaa     180
aggttcagtg gcagtaggtc tgggtcagat tattctctaa ccatcagcag ccttgagtct     240
gaagattttg cagactatta ttgtctacaa tatgctagtt ctccgtacac gttcggaggg     300
gggaccaagc tggaaatcaa a                                               321

<210>  55
<211>  112
<212>  PRT
<213>  Mus musculus

<400>  55
Gln Val Gln Phe Gln Gln Ser Gly Ala Glu Leu Ala Arg Pro Gly Ala
1               5                   10                  15
Ser Val Lys Leu Ser Cys Lys Ala Ser Gly Tyr Ile Phe Thr Ser Tyr
            20                  25                  30
Trp Ile Gln Trp Ile Lys Gln Arg Pro Gly Gln Gly Leu Glu Trp Ile
        35                  40                  45

Gly Ala Ile Tyr Pro Gly Asp Gly Asp Thr Arg Tyr Thr Gln Lys Phe
    50                  55                  60
Lys Gly Lys Ala Thr Leu Thr Ala Asp Lys Ser Ser Ser Thr Ala Tyr
65                  70                  75                  80
Met Gln Leu Arg Ser Leu Ala Ser Glu Asp Ser Ala Val Tyr Tyr Cys
                85                  90                  95
Ala Gly Phe Ala Ser Trp Gly Gln Gly Thr Leu Leu Thr Val Ser Ala
            100                 105                 110

<210>  56
<211>  10
<212>  PRT
<213>  Mus musculus

<400>  56
Gly Tyr Ile Phe Thr Ser Tyr Trp Ile Gln
1               5                   10

<210>  57
<211>  17
<212>  PRT
<213>  Mus musculus

<400>  57
Ala Ile Tyr Pro Gly Asp Gly Asp Thr Arg Tyr Thr Gln Lys Phe Lys
1               5                   10                  15
Gly

<210>  58
<211>  5
<212>  PRT
<213>  Mus musculus

<400>  58
Ala Gly Phe Ala Ser
1               5

<210>  59
<211>  336
<212>  DNA
<213>  Mus musculus

<400>  59
caggttcagt tccagcagtc tggggctgag ctggcaagac ctggggcttc agtgaagttg      60
tcctgcaagg cttctggcta catttttact agttactgga tacagtggat aaaacagaga     120
cctggacagg gtctggaatg gattggggct atttatcctg gagatggtga tactagatat     180
actcagaagt ttaagggcaa ggccacattg actgcagata aatcctccag tacagcctac     240
atgcaactca gaagcttggc gtctgaggac tctgcggtct attactgtgc aggttttgct     300
tcctggggcc aagggactct gctcactgtc tctgca                               336

<210>  60
<211>  107
<212>  PRT
<213>  Mus musculus

<400>  60
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Glu Ser
            20                  25                  30
Leu Ile Trp Leu Gln Gln Glu Pro Asp Gly Thr Ile Lys Arg Leu Ile
        35                  40                  45

Tyr Ala Thr Ser Ser Leu Asp Ser Gly Val Pro Lys Arg Phe Ser Gly
    50                  55                  60
Ser Arg Ser Gly Ser Asp Tyr Ser Leu Ser Ile Ser Ser Leu Glu Ser
65                  70                  75                  80
Asp Asp Phe Val Asp Tyr Tyr Cys Leu Gln Tyr Ser Ser Ser Pro Tyr
                85                  90                  95
Thr Phe Gly Gly Gly Thr Lys Leu Glu Ile Lys
            100                 105

<210>  61
<211>  11
<212>  PRT
<213>  Mus musculus

<400>  61
Arg Ala Ser Gln Asp Ile Gly Glu Ser Leu Ile
1               5                   10

<210>  62
<211>  7
<212>  PRT
<213>  Mus musculus

<400>  62
Ala Thr Ser Ser Leu Asp Ser
1               5

<210>  63
<211>  9
<212>  PRT
<213>  Mus musculus

<400>  63
Leu Gln Tyr Ser Ser Ser Pro Tyr Thr
1               5

<210>  64
<211>  321
<212>  DNA
<213>  Mus musculus

<400>  64
gacatccaga tgacccagtc tccatcctcc ttatctgcct ctctgggaga aagagtcagt        60
ctcacttgtc gggcaagtca ggacattggt gagagcttaa tctggcttca gcaggaacca       120
gatggaacta ttaaacgcct gatctacgcc acatccagtt tagattctgg tgtccccaaa       180
aggttcagtg gcagtaggtc tgggtcagat tattctctct ccatcagcag ccttgagtct       240
gacgattttg tagactatta ctgtctacaa tattctagtt ctccgtacac gttcggaggg       300
gggaccaagc tggaaatcaa a                                                 321

<210>  65
<211>  118
<212>  PRT
<213>  Mus musculus

<400>  65
Gln Val Gln Leu Gln Gln Pro Gly Thr Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Tyr Leu Tyr Trp Val Lys Gln Arg Pro Gly Gln Gly Leu Glu Trp Ile
        35                  40                  45
Gly Gly Ile Asn Pro Ser Asn Gly Gly Ser Asn Phe Asn Glu Lys Phe
    50                  55                  60

Lys Thr Lys Ala Thr Leu Thr Val Asp Lys Thr Ser Asn Thr Ala Tyr
65                  70                  75                  80
Met Gln Leu Ser Ser Leu Thr Ser Glu Asp Ser Ala Val Tyr Phe Cys
                85                  90                  95
Thr Arg Arg Gly Ser Ser Trp Asn Phe Asp Val Trp Gly Ala Gly Thr
            100                 105                 110
Thr Val Thr Val Ser Ser
        115

<210>  66
<211>  10
<212>  PRT
<213>  Mus musculus

<400>  66
Gly Tyr Thr Phe Thr Ser Tyr Tyr Leu Tyr
1               5                   10

<210>  67
<211>  17
<212>  PRT
<213>  Mus musculus

<400>  67
Gly Ile Asn Pro Ser Asn Gly Gly Ser Asn Phe Asn Glu Lys Phe Lys
1               5                   10                  15
Thr

<210>  68
<211>  11
<212>  PRT
<213>  Mus musculus

<400>  68
Thr Arg Arg Gly Ser Ser Trp Asn Phe Asp Val
1               5                   10

<210>  69
<211>  354
<212>  DNA
<213>  Mus musculus

<400>  69
caggtccaac tgcagcagcc tgggactgaa ctggtgaagc ccggggcttc agtgaagttg      60
tcctgcaagg cttctggcta caccttcacc agctactatc tgtactgggt gaagcagagg     120
cctggacaag gccttgagtg gattgggggg attaatccta gcaatggtgg ttctaacttc     180
aatgagaagt tcaagaccaa ggccacactg actgtagata aaacctccaa cacagcctac     240
atgcaactca gcagcctgac atctgaggac tctgcggtct atttttgtac aagaaggggt     300
agtagttgga acttcgatgt ctggggcgca gggaccacgg tcaccgtctc ctca           354

<210>  70
<211>  107
<212>  PRT
<213>  Mus musculus

<400>  70
Asp Ile Lys Met Thr Gln Ser Pro Ser Ser Met Tyr Ala Ser Leu Gly
1               5                   10                  15
Glu Arg Val Thr Ile Gly Cys Lys Ala Ser Gln Asp Ile Asn Ser Tyr
            20                  25                  30
Leu Ser Trp Phe Gln Gln Lys Pro Gly Lys Ser Pro Lys Thr Leu Ile
        35                  40                  45

Tyr Arg Thr Asn Arg Leu Val Asp Gly Val Pro Ser Arg Phe Ser Gly
    50                  55                  60
Ser Gly Ser Gly Gln Asp Tyr Ser Leu Thr Ile Ser Ser Leu Glu Tyr
65                  70                  75                  80
Glu Asp Val Gly Ile Tyr Tyr Cys Leu Gln Tyr Asp Glu Phe Pro Leu
                85                  90                  95
Thr Phe Gly Ala Gly Thr Lys Leu Glu Leu Lys
            100                 105

<210>  71
<211>  11
<212>  PRT
<213>  Mus musculus

<400>  71
Lys Ala Ser Gln Asp Ile Asn Ser Tyr Leu Ser
1               5                   10

<210>  72
<211>  7
<212>  PRT
<213>  Mus musculus

<400>  72
Arg Thr Asn Arg Leu Val Asp
1               5

<210>  73
<211>  9
<212>  PRT
<213>  Mus musculus

<400>  73
Leu Gln Tyr Asp Glu Phe Pro Leu Thr
1               5

<210>  74
<211>  321
<212>  DNA
<213>  Mus musculus

<400>  74
gacatcaaga tgacccagtc tccatcttcc atgtatgcat ctctaggaga gagagtcact        60
atcggttgca aggcgagtca ggacattaat agctatttaa gctggttcca acagaaacca       120
gggaaatctc ctaagaccct gatctatcgt acaaacagat tggtagatgg ggtcccatca       180
aggttcagtg gcagtggatc tgggcaagat tattctctca ccatcagcag cctggaatat       240
gaagatgtgg gaatttatta ttgtctacag tatgatgagt ttccgctcac gttcggtgct       300
gggaccaagc tggagctgaa a                                                 321

<210>  75
<211>  112
<212>  PRT
<213>  Mus musculus

<400>  75
Gln Val Gln Leu Gln Gln Ser Gly Pro Glu Leu Val Lys Pro Gly Ala
1               5                   10                  15
Ser Val Lys Ile Ser Cys Lys Ala Ser Gly Tyr Ile Phe Thr Asn Tyr
            20                  25                  30
Tyr Ile His Trp Val Lys Gln Arg Pro Gly Gln Gly Leu Glu Trp Ile
        35                  40                  45
Gly Trp Ile Phe Pro Gly Ser Gly Asn Thr Lys Tyr Ser Glu Lys Phe
    50                  55                  60

Lys Asp Lys Ala Thr Leu Thr Ala Asp Thr Ser Ser Ser Thr Ala Tyr
65                  70                  75                  80
Met Gln Leu Ser Ser Leu Thr Ser Glu Asp Ser Ala Val Tyr Phe Cys
                85                  90                  95
Ala Gly Leu Ser Asp Trp Gly Gln Gly Thr Thr Leu Thr Val Ser Ser
            100                 105                 110

<210>  76
<211>  10
<212>  PRT
<213>  Mus musculus

<400>  76
Gly Tyr Ile Phe Thr Asn Tyr Tyr Ile His
1               5                   10

<210>  77
<211>  17
<212>  PRT
<213>  Mus musculus

<400>  77
Trp Ile Phe Pro Gly Ser Gly Asn Thr Lys Tyr Ser Glu Lys Phe Lys
1               5                   10                  15
Asp

<210>  78
<211>  5
<212>  PRT
<213>  Mus musculus

<400>  78
Ala Gly Leu Ser Asp
1               5

<210>  79
<211>  336
<212>  DNA
<213>  Mus musculus

<400>  79
caggtccagc tgcagcagtc tggacctgag ctggtgaagc ctggggcttc agtgaagata      60
tcctgcaagg cttctggcta catcttcaca aactactata tacactgggt gaagcagagg     120
cctggacagg gacttgagtg gattggatgg atttttcctg gaagtggtaa tactaagtac     180
agtgagaagt tcaaggacaa ggccacactg acggcagaca catcctccag cacagcctac     240
atgcagctca gcagcctgac atctgaggac tctgcagtct atttctgtgc tggcctgtcc     300
gactggggcc aaggcaccac tctcacagtc tcctca                               336

<210>  80
<211>  107
<212>  PRT
<213>  Mus musculus

<400>  80
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Asp Arg
            20                  25                  30
Leu Ile Trp Leu Gln Gln Glu Pro Ala Gly Thr Phe Lys Arg Leu Ile
        35                  40                  45
Tyr Ala Thr Ser Ser Leu Asp Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Ser Ser Pro Trp
                85                  90                  95
Thr Phe Gly Gly Gly Thr Lys Leu Glu Ile Lys
            100                 105

<210>  81
<211>  11
<212>  PRT
<213>  Mus musculus

<400>  81
Arg Ala Ser Gln Asp Ile Gly Asp Arg Leu Ile
1               5                   10

<210>  82
<211>  7
<212>  PRT
<213>  Mus musculus

<400>  82
Ala Thr Ser Ser Leu Asp Ser
1               5

<210>  83
<211>  9
<212>  PRT
<213>  Mus musculus

<400>  83
Leu Gln Tyr Ala Ser Ser Pro Trp Thr
1               5

<210>  84
<211>  321
<212>  DNA
<213>  Mus musculus

<400>  84
gacatccaga tgacccagtc tccttcctcc ttatctgcct ctctgggaga aagagtcagt      60
ctcacttgtc gggcaagtca ggacattggt gatagattaa tctggcttca gcaggaacca     120
gctggaactt ttaaacgcct gatctacgcc acatccagtt tagattctgg tgtccccaaa     180
aggttcagtg gcagtaggtc tgggtcagat tattctctca ccatcagcag ccttgagtct     240
gaagactttg tagactatta ctgtctacag tatgctagtt ctccgtggac gttcggtgga     300
ggcaccaagc tggaaatcaa a                                               321

<210>  85
<211>  112
<212>  PRT
<213>  Mus musculus

<400>  85
Gln Ile Gln Leu Gln Gln Ser Gly Ala Glu Leu Ala Arg Pro Gly Ala
1               5                   10                  15
Ser Val Asn Leu Ser Cys Lys Ala Ser Gly Tyr Ser Phe Thr Ser Tyr
            20                  25                  30
Trp Met Gln Trp Leu Lys Gln Arg Pro Gly Gln Gly Leu Glu Trp Ile
        35                  40                  45
Gly Ala Ile Tyr Pro Gly Asp Gly Asp Thr Arg Tyr Thr Gln Lys Phe
    50                  55                  60
Lys Gly Lys Ala Thr Leu Thr Ala Asp Lys Ser Ser Ser Thr Ala Tyr
65                  70                  75                  80

Met Gln Leu Ser Ser Leu Ala Ser Glu Asp Ser Ala Val Tyr Tyr Cys
                85                  90                  95
Ala Leu Phe Ser Tyr Trp Gly Gln Gly Thr Leu Val Thr Val Ser Ala
            100                 105                 110

<210>  86
<211>  10
<212>  PRT
<213>  Mus musculus

<400>  86
Gly Tyr Ser Phe Thr Ser Tyr Trp Met Gln
1               5                   10

<210>  87
<211>  17
<212>  PRT
<213>  Mus musculus

<400>  87
Ala Ile Tyr Pro Gly Asp Gly Asp Thr Arg Tyr Thr Gln Lys Phe Lys
1               5                   10                  15
Gly

<210>  88
<211>  5
<212>  PRT
<213>  Mus musculus

<400>  88
Ala Leu Phe Ser Tyr
1               5

<210>  89
<211>  336
<212>  DNA
<213>  Mus musculus

<400>  89
cagattcagc tccagcagtc tggggctgaa ctggcaagac ctggggcttc agtgaatttg      60
tcctgcaagg cttctggcta cagctttact agctactgga tgcagtggct aaaacagagg     120
cctggacagg gtctggaatg gattggggct atttatcctg gagatggtga tactaggtac     180
actcaaaagt tcaagggcaa ggccacattg actgcagata aatcctccag cacagcctac     240
atgcaactca gcagcttggc atctgaggac tctgcggtct attactgtgc actcttctct     300
tactggggcc aagggactct ggtcactgtc tctgca                               336

<210>  90
<211>  107
<212>  PRT
<213>  Mus musculus

<400>  90
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Ser Ser
            20                  25                  30
Leu Asn Trp Leu Gln Gln Glu Pro Asp Gly Thr Phe Lys Arg Leu Ile
        35                  40                  45
Tyr Ala Thr Ser Thr Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80

Glu Asp Phe Val Asp Tyr Tyr Cys Leu Gln Tyr Ala Ser Phe Pro Tyr
                85                  90                  95
Thr Phe Gly Gly Gly Thr Lys Leu Glu Ile Lys
            100                 105

<210>  91
<211>  11
<212>  PRT
<213>  Mus musculus

<400>  91
Arg Ala Ser Gln Asp Ile Gly Ser Ser Leu Asn
1               5                   10

<210>  92
<211>  7
<212>  PRT
<213>  Mus musculus

<400>  92
Ala Thr Ser Thr Leu Asp Ser
1               5

<210>  93
<211>  9
<212>  PRT
<213>  Mus musculus

<400>  93
Leu Gln Tyr Ala Ser Phe Pro Tyr Thr
1               5

<210>  94
<211>  321
<212>  DNA
<213>  Mus musculus

<400>  94
gacatccaga tgacccagtc tccatcctcc ttatctgcct ctctgggaga aagagtcagt      60
ctcacttgtc gggcaagtca ggacattggt agtagtttaa actggcttca gcaggaacca     120
gatggaactt ttaaacgcct gatctacgcc acatccactt tagattctgg tgtccccaaa     180
aggttcagtg gcagtaggtc tgggtcagat tattctctca ccatcagcag ccttgagtct     240
gaagattttg tagactatta ctgtctacaa tatgctagtt ttccgtacac gttcggaggg     300
gggaccaagc tggaaatcaa a                                               321

<210>  95
<211>  119
<212>  PRT
<213>  Mus musculus

<400>  95
Gln Val Gln Phe Gln Gln Ser Gly Ala Glu Leu Ala Arg Pro Gly Ala
1               5                   10                  15
Ser Val Lys Leu Ser Cys Lys Ala Ser Gly Tyr Thr Phe Thr Gly Tyr
            20                  25                  30
Trp Ile Gln Trp Val Lys Gln Arg Pro Gly Gln Gly Leu Glu Trp Ile
        35                  40                  45
Gly Ala Ile Tyr Pro Gly Asp Asp Asp Thr Arg Tyr Thr Gln Lys Phe
    50                  55                  60
Lys Gly Arg Ala Thr Leu Thr Ala Asp Thr Ser Ser Ser Thr Ala Tyr
65                  70                  75                  80
Met Gln Leu Ser Ser Leu Ala Ser Glu Asp Ser Ala Val Tyr Tyr Cys
                85                  90                  95

Ala Arg Gly Asn Trp Asp Asp Trp Asp Phe Asp Val Trp Gly Ala Gly
            100                 105                 110
Thr Thr Val Thr Val Ser Ser
        115

<210>  96
<211>  10
<212>  PRT
<213>  Mus musculus

<400>  96
Gly Tyr Thr Phe Thr Gly Tyr Trp Ile Gln
1               5                   10

<210>  97
<211>  17
<212>  PRT
<213>  Mus musculus

<400>  97
Ala Ile Tyr Pro Gly Asp Asp Asp Thr Arg Tyr Thr Gln Lys Phe Lys
1               5                   10                  15
Gly

<210>  98
<211>  10
<212>  PRT
<213>  Mus musculus

<400>  98
Gly Asn Trp Asp Asp Trp Asp Phe Asp Val
1               5                   10

<210>  99
<211>  357
<212>  DNA
<213>  Mus musculus

<400>  99
caggttcagt tccagcagtc tggggctgag ctggcaagac ctggggcttc agtgaagttg      60
tcctgcaagg cttctggcta cacctttact ggctactgga tacagtgggt aaaacagagg     120
cctggacagg gtctggaatg gattggggct atttatcctg gagatgatga tactaggtac     180
actcagaagt ttaagggcag ggccacattg actgcagata catcctccag cacagcctac     240
atgcaactca gcagcttggc atctgaggac tctgcggtct attactgtgc aagagggaac     300
tgggacgact gggacttcga tgtctggggc gcagggacca cggtcaccgt ctcctca        357

<210>  100
<211>  107
<212>  PRT
<213>  Mus musculus

<400>  100
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Asn Tyr
            20                  25                  30
Leu Asn Trp Tyr Gln Gln Lys Pro Asp Gly Thr Val Lys Leu Leu Ile
        35                  40                  45
His Tyr Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asp Thr Leu Pro Trp
                85                  90                  95
Thr Phe Gly Gly Gly Thr Lys Leu Glu Ile Lys
            100                 105

<210>  101
<211>  11
<212>  PRT
<213>  Mus musculus

<400>  101
Arg Ala Ser Gln Asp Ile Ser Asn Tyr Leu Asn
1               5                   10

<210>  102
<211>  7
<212>  PRT
<213>  Mus musculus

<400>  102
Tyr Thr Ser Arg Leu His Ser
1               5

<210>  103
<211>  9
<212>  PRT
<213>  Mus musculus

<400>  103
Gln Gln Gly Asp Thr Leu Pro Trp Thr
1               5

<210>  104
<211>  321
<212>  DNA
<213>  Mus musculus

<400>  104
gatatccaga tgacacagac tacatcctcc ctgtctgcct ctctgggaga cagagtcacc        60
atcagttgca gggcaagtca ggacattagc aattatttaa actggtatca gcagaaacca       120
gatggaactg ttaaactcct gatccattac acatcaagat tacactcagg agtcccatca       180
aggttcagtg gcagtgggtc tggaacagac tattctctca ccattagcaa cctggaacaa       240
gaagatattg ccacttactt ttgccaacag ggtgatacgc ttccgtggac gttcggtgga       300
ggcaccaagc tggaaatcaa a                                                 321

<210>  105
<211>  119
<212>  PRT
<213>  Mus musculus

<400>  105
Gln Val His Leu Gln Gln Ser Gly Ala Glu Leu Ala Arg Pro Gly Ala
1               5                   10                  15
Ser Val Lys Leu Ser Cys Lys Ala Ser Gly Tyr Ser Phe Ile Ser Tyr
            20                  25                  30
Trp Met Glu Trp Val Lys Gln Arg Pro Gly Gln Gly Leu Glu Trp Ile
        35                  40                  45
Gly Ala Ile Tyr Pro Gly Asp Gly Asp Thr Arg Tyr Thr Gln Glu Phe
    50                  55                  60
Lys Gly Lys Ala Thr Leu Thr Ala Asp Glu Ser Ser Ser Thr Ala Tyr
65                  70                  75                  80
Met Gln Leu Ser Ser Leu Ala Ser Glu Asp Ser Ala Val Tyr Tyr Cys
                85                  90                  95

Ala Arg Gly Asn Trp Asp Asp Trp Asp Phe Asp Val Trp Gly Ala Gly
            100                 105                 110
Thr Thr Val Thr Val Ser Ser
        115

<210>  106
<211>  10
<212>  PRT
<213>  Mus musculus

<400>  106
Gly Tyr Ser Phe Ile Ser Tyr Trp Met Glu
1               5                   10

<210>  107
<211>  17
<212>  PRT
<213>  Mus musculus

<400>  107
Ala Ile Tyr Pro Gly Asp Gly Asp Thr Arg Tyr Thr Gln Glu Phe Lys
1               5                   10                  15
Gly

<210>  108
<211>  10
<212>  PRT
<213>  Mus musculus

<400>  108
Gly Asn Trp Asp Asp Trp Asp Phe Asp Val
1               5                   10

<210>  109
<211>  357
<212>  DNA
<213>  Mus musculus

<400>  109
caggttcacc tccagcagtc tggggctgag ctggcaagac ctggggcttc agtgaagttg      60
tcctgcaagg cttctggcta ctcctttatt agttactgga tggagtgggt aaaacagagg     120
cctggacagg gtctggaatg gattggggct atttatcctg gagatggtga tactaggtac     180
actcaggagt tcaagggcaa ggccacattg actgcagatg aatcctccag cacagcctac     240
atgcaactca gcagcttggc atctgaggac tctgcggtct attactgtgc aagagggaac     300
tgggacgact gggacttcga tgtctggggc gcagggacca cggtcaccgt ctcctca        357

<210>  110
<211>  107
<212>  PRT
<213>  Mus musculus

<400>  110
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Asn Tyr
            20                  25                  30
Leu Asn Trp Tyr Gln Gln Lys Pro Asp Gly Thr Val Lys Leu Leu Ile
        35                  40                  45
His Tyr Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Trp
                85                  90                  95
Thr Phe Gly Gly Gly Thr Lys Leu Glu Ile Lys
            100                 105

<210>  111
<211>  11
<212>  PRT
<213>  Mus musculus

<400>  111
Arg Ala Ser Gln Asp Ile Ser Asn Tyr Leu Asn
1               5                   10

<210>  112
<211>  7
<212>  PRT
<213>  Mus musculus

<400>  112
Tyr Thr Ser Arg Leu His Ser
1               5

<210>  113
<211>  9
<212>  PRT
<213>  Mus musculus

<400>  113
Gln Gln Gly Asn Thr Leu Pro Trp Thr
1               5

<210>  114
<211>  321
<212>  DNA
<213>  Mus musculus

<400>  114
gatatccaga tgacacagac tacatcctcc ctgtctgcct ctctgggaga cagagtcacc        60
atcagttgca gggcaagtca ggacattagc aattatttaa actggtatca gcagaaacca       120
gatggaactg ttaaactcct gatccattac acatcaagat tacactcagg agtcccatca       180
aggttcagtg gcagtgggtc tggaacagac tattctctca ccattagcaa cctggaacaa       240
gaagatattg ccacttactt ttgccaacag ggtgatacgc ttccgtggac gttcggtgga       300
ggcaccaagc tggaaatcaa a                                                 321

<210>  115
<211>  117
<212>  PRT
<213>  Mus musculus

<400>  115
Gln Val Gln Leu Gln Gln Pro Gly Ala Glu Val Val Lys Pro Gly Ala
1               5                   10                  15
Ser Val Arg Leu Ser Cys Lys Pro Ser Gly Tyr Phe Phe Thr Ser Tyr
            20                  25                  30
Trp Met His Trp Val Lys Gln Arg Pro Gly Gln Gly Leu Glu Trp Ile
        35                  40                  45
Gly Glu Ile Asn Pro Ser Asn Gly Arg Thr Asn Tyr Asn Glu Asn Phe
    50                  55                  60
Lys Ser Lys Ala Thr Leu Thr Val Asp Thr Ser Ser Ser Thr Ala Tyr
65                  70                  75                  80
Leu Gln Leu Ser Ser Leu Thr Ser Glu Asp Ser Ala Val Tyr Tyr Cys
                85                  90                  95

Ala Arg Arg Asn Tyr Gly Tyr Pro Asp Tyr Trp Gly Gln Gly Thr Thr
            100                 105                 110
Leu Thr Val Ser Ser
        115

<210>  116
<211>  10
<212>  PRT
<213>  Mus musculus

<400>  116
Gly Tyr Phe Phe Thr Ser Tyr Trp Met His
1               5                   10

<210>  117
<211>  17
<212>  PRT
<213>  Mus musculus

<400>  117
Glu Ile Asn Pro Ser Asn Gly Arg Thr Asn Tyr Asn Glu Asn Phe Lys
1               5                   10                  15
Ser

<210>  118
<211>  8
<212>  PRT
<213>  Mus musculus

<400>  118
Arg Asn Tyr Gly Tyr Pro Asp Tyr
1               5

<210>  119
<211>  351
<212>  DNA
<213>  Mus musculus

<400>  119
caggtccaac tgcagcagcc tggggctgaa gtggtgaagc ctggggcttc agtgagactg      60
tcctgtaagc cttctggcta cttcttcacc agctactgga tgcactgggt gaaacagagg     120
cctggacaag gccttgagtg gattggagag attaatccta gtaacggtcg tactaactac     180
aatgagaact tcaagagcaa ggccacactg actgtagaca catcttccag cacagcctac     240
ctgcaactca gcagcctgac atctgaggac tctgcggtct attactgtgc aagaagaaac     300
tacggctacc ctgactactg gggccaaggc accactctca cagtctcctc a              351

<210>  120
<211>  107
<212>  PRT
<213>  Mus musculus

<400>  120
Asp Ile Lys Met Thr Gln Ser Pro Ser Ser Met Tyr Ala Ser Leu Gly
1               5                   10                  15
Glu Arg Val Thr Phe Thr Cys Lys Ala Ser Gln Asp Ile Asn Thr Tyr
            20                  25                  30
Leu Asn Trp Phe Gln Gln Lys Pro Gly Lys Ser Pro Lys Thr Leu Ile
        35                  40                  45
Tyr Arg Ala Asn Arg Leu Leu Asp Gly Val Pro Ser Arg Phe Ser Gly
    50                  55                  60
Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Gln Tyr Asp Glu Ile Pro Tyr
                85                  90                  95
Thr Phe Gly Gly Gly Thr Lys Leu Glu Ile Lys
            100                 105

<210>  121
<211>  11
<212>  PRT
<213>  Mus musculus

<400>  121
Lys Ala Ser Gln Asp Ile Asn Thr Tyr Leu Asn
1               5                   10

<210>  122
<211>  7
<212>  PRT
<213>  Mus musculus

<400>  122
Arg Ala Asn Arg Leu Leu Asp
1               5

<210>  123
<211>  9
<212>  PRT
<213>  Mus musculus

<400>  123
Leu Gln Tyr Asp Glu Ile Pro Tyr Thr
1               5

<210>  124
<211>  321
<212>  DNA
<213>  Mus musculus

<400>  124
gacatcaaga tgacccagtc tccatcttcc atgtatgcat ctctaggaga gagagtcact      60
ttcacttgca aggcgagtca ggacattaat acctatttaa actggttcca gcagaaacca     120
gggaaatctc ctaagaccct gatataccgt gcaaacagat tgttagatgg ggtcccatca     180
aggttcagtg gcagtggatc tgggcaagat tattctctca ccatcagcag cctggagtat     240
gaagacatgg gaatttatta ttgtctacag tatgatgaga ttccgtacac gttcggaggg     300
gggaccaagc tggaaatcaa a                                               321

<210>  125
<211>  119
<212>  PRT
<213>  Homo sapiens

<400>  125
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Trp Ile Gln Trp Val Arg Gln Ala Pro Gly Gln Gly Leu Glu Trp Met
        35                  40                  45
Gly Ala Ile Tyr Pro Gly Asp Ser Asp Thr Arg Tyr Thr Gln Lys Phe
    50                  55                  60
Gln Gly Arg Val Thr Met Thr Ala Asp Thr Ser Ile Ser Thr Ala Tyr
65                  70                  75                  80
Met Glu Leu Ser Arg Leu Arg Ser Asp Asp Thr Ala Val Tyr Tyr Cys
                85                  90                  95

Ala Arg Gly Asn Trp Asp Asp Trp Asp Phe Asp Val Trp Gly Gln Gly
            100                 105                 110
Thr Thr Val Thr Val Ser Ser
        115

<210>  126
<211>  10
<212>  PRT
<213>  Homo sapiens

<400>  126
Gly Tyr Thr Phe Thr Gly Tyr Trp Ile Gln
1               5                   10

<210>  127
<211>  17
<212>  PRT
<213>  Homo sapiens

<400>  127
Ala Ile Tyr Pro Gly Asp Ser Asp Thr Arg Tyr Thr Gln Lys Phe Gln
1               5                   10                  15
Gly

<210>  128
<211>  8
<212>  PRT
<213>  Homo sapiens

<400>  128
Gly Asn Trp Asp Asp Trp Asp Phe
1               5

<210>  129
<211>  357
<212>  DNA
<213>  Homo sapiens

<400>  129
caggttcagc tggttcagtc tggcgccgaa gtgaagaaac ctggcgcctc tgtgaaggtg      60
tcctgcaagg ctagcggcta cacattcacc ggctattgga tccagtgggt ccgacaggct     120
cctggacagg gacttgaatg gatgggcgct atctaccctg gcgactccga caccagatac     180
acccagaaat tccagggcag agtgaccatg accgccgaca ccagcatcag caccgcctac     240
atggaactga gcagactgag aagcgacgac acagccgtgt actactgcgc cagaggcaac     300
tgggacgact gggactttga tgtgtggggc cagggcacca ccgtgacagt ttcttct        357

<210>  130
<211>  119
<212>  PRT
<213>  Homo sapiens

<400>  130
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Trp Ile Gln Trp Val Arg Gln Ala Pro Gly Gln Gly Leu Glu Trp Met
        35                  40                  45
Gly Ala Ile Tyr Pro Gly Asp Glu Asp Thr Arg Tyr Thr Gln Lys Phe
    50                  55                  60
Gln Gly Arg Val Thr Met Thr Ala Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95
Ala Arg Gly Asn Trp Asp Asp Trp Asp Phe Asp Val Trp Gly Gln Gly
            100                 105                 110
Thr Thr Val Thr Val Ser Ser
        115

<210>  131
<211>  10
<212>  PRT
<213>  Homo sapiens

<400>  131
Gly Tyr Thr Phe Thr Gly Tyr Trp Ile Gln
1               5                   10

<210>  132
<211>  17
<212>  PRT
<213>  Homo sapiens

<400>  132
Ala Ile Tyr Pro Gly Asp Glu Asp Thr Arg Tyr Thr Gln Lys Phe Gln
1               5                   10                  15
Gly

<210>  133
<211>  8
<212>  PRT
<213>  Homo sapiens

<400>  133
Gly Asn Trp Asp Asp Trp Asp Phe
1               5

<210>  134
<211>  357
<212>  DNA
<213>  Homo sapiens

<400>  134
caggttcagc tggttcagtc tggcgccgaa gtgaagaaac ctggcgcctc tgtgaaggtg      60
tcctgcaagg ctagcggcta cacattcacc ggctattgga tccagtgggt ccgacaggct     120
cctggacagg gacttgaatg gatgggcgct atctaccctg gcgacgaaga caccagatac     180
acccagaaat tccagggcag agtgaccatg accgccgaca ccagcatcag caccgcctac     240
atggaactga gcagactgag aagcgacgac acagccgtgt actactgcgc cagaggcaac     300
tgggacgact gggactttga tgtgtggggc cagggcacca ccgtgacagt ttcttct        357

<210>  135
<211>  119
<212>  PRT
<213>  Homo sapiens

<400>  135
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Gly Trp Ile Arg Gln Pro Pro Gly Lys Ala Leu Glu
        35                  40                  45
Trp Leu Ala His Ile Trp Trp Asp Asp Asp Lys Tyr Tyr Asn Pro Ser
    50                  55                  60

Leu Lys Ser Arg Leu Thr Ile Ser Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Arg Trp Ala Trp Phe Ala Tyr Trp Gly Gln Gly
            100                 105                 110
Thr Leu Val Thr Val Ser Ser
        115

<210>  136
<211>  12
<212>  PRT
<213>  Homo sapiens

<400>  136
Gly Phe Ser Leu Ser Thr Ser Gly Met Gly Val Gly
1               5                   10

<210>  137
<211>  16
<212>  PRT
<213>  Homo sapiens

<400>  137
His Ile Trp Trp Asp Asp Asp Lys Tyr Tyr Asn Pro Ser Leu Lys Ser
1               5                   10                  15

<210>  138
<211>  9
<212>  PRT
<213>  Homo sapiens

<400>  138
Arg Glu Arg Trp Ala Trp Phe Ala Tyr
1               5

<210>  139
<211>  357
<212>  DNA
<213>  Homo sapiens

<400>  139
caagtgaccc tgagagagtc tggacccgct ctggtcaagc ccacacagac cctgacactg        60
acctgtacct tcagcggctt cagcctgagc acatctggca tgggagtcgg ctggatcaga       120
cagcctcctg gcaaagctct ggaatggctg gcccacattt ggtgggacga cgacaagtac       180
tacaacccca gcctgaagtc cagactgacc atcagcaagg acaccagcaa gaaccaggtg       240
gtgctgacca tgaccaacat ggaccctgtg gacaccgcca cctactactg cgctagaaga       300
gaaagatggg cttggtttgc ctactggggc cagggaacac tggtcaccgt tagctct          357

<210>  140
<211>  119
<212>  PRT
<213>  Homo sapiens

<400>  140
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Gly Trp Ile Arg Gln Pro Pro Gly Lys Ala Leu Glu
        35                  40                  45
Trp Leu Ala His Ile Trp Trp Asp Asp Asp Lys Tyr Tyr Asn Pro Ser
    50                  55                  60

Leu Lys Thr Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Arg Trp Ala Trp Phe Ala Tyr Trp Gly Gln Gly
            100                 105                 110
Thr Leu Val Thr Val Ser Ser
        115

<210>  141
<211>  12
<212>  PRT
<213>  Homo sapiens

<400>  141
Gly Phe Ser Leu Ser Thr Ser Gly Met Gly Val Gly
1               5                   10

<210>  142
<211>  16
<212>  PRT
<213>  Homo sapiens

<400>  142
His Ile Trp Trp Asp Asp Asp Lys Tyr Tyr Asn Pro Ser Leu Lys Thr
1               5                   10                  15

<210>  143
<211>  9
<212>  PRT
<213>  Homo sapiens

<400>  143
Arg Glu Arg Trp Ala Trp Phe Ala Tyr
1               5

<210>  144
<211>  357
<212>  DNA
<213>  Homo sapiens

<400>  144
caagtgaccc tgagagagtc tggacccgct ctggtcaagc ccacacagac cctgacactg        60
acctgtacct tcagcggctt cagcctgagc acatctggca tgggagtcgg ctggatcaga       120
cagcctcctg gcaaagctct ggaatggctg gcccacattt ggtgggacga cgacaagtac       180
tacaacccca gcctgaagac cagactgacc atcagcaagg acaccagcag aaaccaggtg       240
gtgctgacca tgaccaacat ggaccctgtg gacaccgcca cctactactg cgctagaaga       300
gaaagatggg cttggtttgc ctactggggc cagggaacac tggtcaccgt tagctct         357

<210>  145
<211>  119
<212>  PRT
<213>  Homo sapiens

<400>  145
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Gly Trp Ile Arg Gln Pro Pro Gly Lys Ala Leu Glu
        35                  40                  45
Trp Leu Ala His Ile Trp Trp Asp Glu Asp Lys Tyr Tyr Asn Pro Ser
    50                  55                  60

Leu Lys Ser Arg Leu Thr Ile Ser Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Arg Trp Ala Trp Phe Ala Tyr Trp Gly Gln Gly
            100                 105                 110
Thr Leu Val Thr Val Ser Ser
        115

<210>  146
<211>  12
<212>  PRT
<213>  Homo sapiens

<400>  146
Gly Phe Ser Leu Ser Thr Ser Gly Met Gly Val Gly
1               5                   10

<210>  147
<211>  16
<212>  PRT
<213>  Homo sapiens

<400>  147
His Ile Trp Trp Asp Glu Asp Lys Tyr Tyr Asn Pro Ser Leu Lys Ser
1               5                   10                  15

<210>  148
<211>  9
<212>  PRT
<213>  Homo sapiens

<400>  148
Arg Glu Arg Trp Ala Trp Phe Ala Tyr
1               5

<210>  149
<211>  357
<212>  DNA
<213>  Homo sapiens

<400>  149
caagtgaccc tgagagagtc tggacccgct ctggtcaagc ccacacagac cctgacactg        60
acctgtacct tcagcggctt cagcctgagc acatctggca tgggagtcgg ctggatcaga       120
cagcctcctg gcaaagctct ggaatggctg gcccacattt ggtgggacga ggacaagtac       180
tacaacccca gcctgaagtc cagactgacc atcagcaagg acaccagcaa gaaccaggtg       240
gtgctgacca tgaccaacat ggaccctgtg gacaccgcca cctactactg cgctagaaga       300
gaaagatggg cttggtttgc ctactggggc cagggaacac tggtcaccgt tagctct          357

<210>  150
<211>  107
<212>  PRT
<213>  Homo sapiens

<400>  150
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
His Tyr Thr Ser Arg Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Gly Asp Thr Leu Pro Trp
                85                  90                  95
Thr Phe Gly Gln Gly Thr Lys Val Glu Ile Lys
            100                 105

<210>  151
<211>  11
<212>  PRT
<213>  Homo sapiens

<400>  151
Arg Ala Ser Gln Asp Ile Ser Asn Tyr Leu Asn
1               5                   10

<210>  152
<211>  7
<212>  PRT
<213>  Homo sapiens

<400>  152
Tyr Thr Ser Arg Leu His Ser
1               5

<210>  153
<211>  9
<212>  PRT
<213>  Homo sapiens

<400>  153
Gln Gln Gly Asp Thr Leu Pro Trp Thr
1               5

<210>  154
<211>  321
<212>  DNA
<213>  Homo sapiens

<400>  154
gacatccaga tgacacagag ccctagcagc ctgtctgcca gcgtgggaga cagagtgacc      60
atcacctgta gagccagcca ggacatcagc aactacctga actggtatca gcagaagccc     120
ggcaaggccc ctaagctgct gatccactac accagcagac tgcacagcgg cgtgccaagc     180
agattttctg gcagcggctc tggcaccgac tacaccctga caatctctag cctgcagcct     240
gaggacttcg ccacctacta ttgtcagcag ggcgacaccc tgccttggac atttggccag     300
ggcaccaagg tggaaatcaa g                                               321

<210>  155
<211>  107
<212>  PRT
<213>  Homo sapiens

<400>  155
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
His Tyr Thr Ser Arg Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asp Thr Leu Pro Trp
                85                  90                  95
Thr Phe Gly Gln Gly Thr Lys Val Glu Ile Lys
            100                 105

<210>  156
<211>  11
<212>  PRT
<213>  Homo sapiens

<400>  156
Arg Ala Ser Gln Asp Ile Ser Asn Tyr Leu Asn
1               5                   10

<210>  157
<211>  7
<212>  PRT
<213>  Homo sapiens

<400>  157
Tyr Thr Ser Arg Leu His Ser
1               5

<210>  158
<211>  9
<212>  PRT
<213>  Homo sapiens

<400>  158
Gln Gln Gly Asp Thr Leu Pro Trp Thr
1               5

<210>  159
<211>  321
<212>  DNA
<213>  Homo sapiens

<400>  159
gacatccaga tgacacagag ccctagcagc ctgtctgcca gcgtgggaga cagagtgacc      60
atcacctgta gagccagcca ggacatcagc aactacctga actggtatca gcagaagccc     120
ggcaaggccc ctaagctgct gatccactac accagcagac tgcacagcgg cgtgccaagc     180
agattttctg gcagcggctc tggcaccgac ttcaccctga caatctctag cctgcagcct     240
gaggacttcg ccacctacta ttgtcagcag ggcgacaccc tgccttggac atttggccag     300
ggcaccaagg tggaaatcaa g                                               321

<210>  160
<211>  106
<212>  PRT
<213>  Homo sapiens

<400>  160
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asn Val Phe Thr Asn
            20                  25                  30
Val Ala Trp Tyr Gln Gln Lys Pro Gly Lys Ala Pro Lys Val Leu Ile
        35                  40                  45
Tyr Ser Ala Ser Tyr Arg Tyr Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Phe Cys Gln Gln Tyr Asn Ser Tyr Pro Thr
                85                  90                  95

Phe Gly Gln Gly Thr Lys Leu Glu Ile Lys
            100                 105

<210>  161
<211>  11
<212>  PRT
<213>  Homo sapiens

<400>  161
Arg Ala Ser Gln Asn Val Phe Thr Asn Val Ala
1               5                   10

<210>  162
<211>  7
<212>  PRT
<213>  Homo sapiens

<400>  162
Ser Ala Ser Tyr Arg Tyr Ser
1               5

<210>  163
<211>  8
<212>  PRT
<213>  Homo sapiens

<400>  163
Gln Gln Tyr Asn Ser Tyr Pro Thr
1               5

<210>  164
<211>  318
<212>  DNA
<213>  Homo sapiens

<400>  164
gacatccaga tgacacagag ccctagcagc ctgtctgcca gcgtgggaga cagagtgacc        60
atcacctgta gagccagcca gaacgtgttc accaacgtgg cctggtatca gcagaagcct       120
ggcaaggctc ccaaggtgct gatctacagc gccagctaca gatacagcgg cgtgccctct       180
agattcagcg gctctggctc tggcaccgac ttcaccctga caatctctag cctgcagcct       240
gaggacttcg ctacctactt ctgccagcag tacaacagct accccacctt cggccagggc       300
accaagctgg aaatcaag                                                     318

<210>  165
<211>  106
<212>  PRT
<213>  Homo sapiens

<400>  165
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Val Phe Thr Asn
            20                  25                  30
Val Ala Trp Tyr Gln Gln Lys Pro Gly Lys Ala Pro Lys Val Leu Ile
        35                  40                  45
Tyr Ser Ala Ser Tyr Arg Tyr Ser Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Tyr Asn Ser Tyr Pro Thr
                85                  90                  95
Phe Gly Gln Gly Thr Lys Leu Glu Ile Lys
            100                 105

<210>  166
<211>  11
<212>  PRT
<213>  Homo sapiens

<400>  166
Gln Ala Ser Gln Asn Val Phe Thr Asn Val Ala
1               5                   10

<210>  167
<211>  7
<212>  PRT
<213>  Homo sapiens

<400>  167
Ser Ala Ser Tyr Arg Tyr Ser
1               5

<210>  168
<211>  8
<212>  PRT
<213>  Homo sapiens

<400>  168
Gln Gln Tyr Asn Ser Tyr Pro Thr
1               5

<210>  169
<211>  318
<212>  DNA
<213>  Homo sapiens

<400>  169
gacatccaga tgacacagag ccctagcagc ctgtctgcca gcgtgggaga cagagtgacc        60
atcacctgtc aggccagcca gaacgtgttc accaacgtgg cctggtatca gcagaagcct       120
ggcaaggctc ccaaggtgct gatctacagc gccagctaca gatacagcgg cgtgccctct       180
agattcagcg gctctggctc tggcaccgac ttcaccttca caatctctag cctgcagcct       240
gaggacatcg ctacctacta ctgccagcag tacaacagct accccacctt cggccagggc       300
accaagctgg aaatcaag                                                     318

<210>  170
<211>  106
<212>  PRT
<213>  Homo sapiens

<400>  170
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Asn Val Phe Thr Asn
            20                  25                  30
Val Ala Trp Tyr Gln Gln Lys Pro Gly Gln Ala Pro Arg Val Leu Ile
        35                  40                  45
Tyr Ser Ala Ser Tyr Arg Tyr Ser Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Phe Cys Gln Gln Tyr Asn Ser Tyr Pro Thr
                85                  90                  95
Phe Gly Gln Gly Thr Lys Leu Glu Ile Lys
            100                 105

<210>  171

<211>  11
<212>  PRT
<213>  Homo sapiens

<400>  171
Arg Ala Ser Gln Asn Val Phe Thr Asn Val Ala
1               5                   10

<210>  172
<211>  7
<212>  PRT
<213>  Homo sapiens

<400>  172
Ser Ala Ser Tyr Arg Tyr Ser
1               5

<210>  173
<211>  8
<212>  PRT
<213>  Homo sapiens

<400>  173
Gln Gln Tyr Asn Ser Tyr Pro Thr
1               5

<210>  174
<211>  318
<212>  DNA
<213>  Homo sapiens

<400>  174
gagatcgtga tgacacagag ccccgccaca ctgagtgtgt ctccaggcga aagagctacc        60
ctgagctgta gagccagcca gaacgtgttc accaacgtgg cctggtatca gcagaagcct       120
ggacaggctc ccagagtgct gatctacagc gccagctaca gatacagcgg catccccgct       180
agattcagcg gctctggatc tggcaccgag ttcaccctga caatcagcag cctgcagagc       240
gaggacttcg ccgtgtactt ctgccagcag tacaacagct accccacctt cggccagggc       300
accaagctgg aaatcaag                                                     318